(19) Europäisches Patentamt
European Patent Office
Office européen des brevets

(11) Publication number: **0 157 609 B1**

# (12) EUROPEAN PATENT SPECIFICATION

(45) Date of publication of patent specification: **14.10.92**

(51) Int. Cl.⁵: **C07C 271/08**, C07C 275/46, C07D 295/125, C07D 295/205, C07D 207/09, C07D 209/49, C07D 211/56, C07D 213/40, A61K 31/27, C07D 213/75

(21) Application number: **85302202.8**

(22) Date of filing: **29.03.85**

## (54) Lipid derivatives their production and use.

(30) Priority: **03.04.84 PCT/JP84/00163**
**11.10.84 PCT/JP84/00476**
**15.02.85 PCT/JP85/00062**

(43) Date of publication of application:
**09.10.85 Bulletin 85/41**

(45) Publication of the grant of the patent:
**14.10.92 Bulletin 92/42**

(84) Designated Contracting States:
**AT BE CH DE FR GB IT LI LU NL SE**

(56) References cited:
**EP-A- 0 094 586**

(73) Proprietor: **Takeda Chemical Industries, Ltd.**
**1-1, Doshomachi 4-chome**
**Chuo-ku, OSAKA(JP)**

(72) Inventor: **Nomura, Hiroaki**
**9-15, Higashikanmaki 3-chome**
**Takatsuki-shi Osaka 569(JP)**
Inventor: **Nishikawa, Kohei**
**5-19, Oharano-kamisatotorimicho Nishikyo-ku**
**Kyoto-shi Kyoto 610-11(JP)**
Inventor: **Tsushima, Susumu**
**1-705, 3 Momoyamadai 4-chome**
**Suita-shi Osaka 565(JP)**

(74) Representative: **Laredo, Jack Joseph et al**
**Elkington and Fife Prospect House 8 Pembroke Road**
**Sevenoaks, Kent TN13 1XR(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to novel lipid derivatives which are of value as pharmaceuticals.

PAF "Platelet Activating Factor" possesses the structure of a phospholiphid and is a chemical transmitter which exists in living organisms. It has been clarified that PAF, as its in-vivo function, is closely involved in allergy, anaphylaxis and inflammation as well as platelet aggregation, and furthermore, PAF is known to exhibit potent hypotensive activity.

When PAF is given to animals, on the other hand, complication of these activities causes the animals to produce symptoms of shock, occasionally leading to death. The shock produced by PAF resembles symptoms of shock due to endotoxin, and PAF is suspected to be involved in the endotoxin shock.

With reference to the metastasis of cancer, it is thought that platelet aggregation is implicated in the stage of implantation of cancer cells, and lipid derivatives having the ether and carbamoyl bonds, because enzymes capable of breaking such bonds are deficient particuarly in cancer cells, further tend to accumulate in cancer cells and exhibit the action to bring about change in the lipid metabolism within the cancer cell, eventually resulting in death of cancer cells. EPA 94586 describes glycerol derivatives of the general formula:

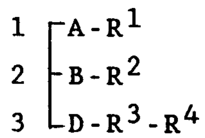

I

[wherein A represents an oxygen atom or a sulfur atom, B represents an oxygen atom or a sulfur atom, D represents an oxygen atom or a carbonyloxy group (i.e.

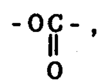

in which the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group, $R^2$ represents, when B is an oxygen atom, a hydrogen atom, or a group of the general formula:

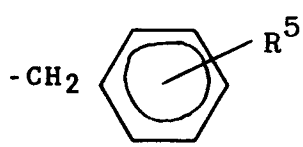

$-COR^6$ or $-CONHR^7$, in which $R^5$ represents a hydrogen atom, a halogen atom, an alkyl group of 1 to 4 carbon atoms, $R^6$ represents an alkyl group of 1 to 6 carbon atoms or a phenyl group and $R^7$ represents an alkyl group of 1 to 6 carbon atoms or a phenyl group, and $R^2$ represents, when B is a sulfur atom, a hydrogen atom, an alkyl group of 1 to 6 carbon atoms, or a group of the general formula:

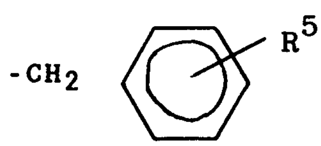

or $-CS-OR^8$, in which $R^8$ represents an alkyl group of 1 to 6 carbon atoms or a phenyl group and $R^5$ is as hereinbefore defined, $R^3$ represents an alkylene group of 1 to 12 carbon atoms and $R^4$ represents an amino group, or a group of the general formula:

$-NHCOR^9$, $-NHR^{10}$, $-N(R^{10})_2$, $-\overset{\oplus}{N}(R^{10})_3 \cdot \overset{\ominus}{Y}$, in which $R^9$ represents an alkyl group of 1 to 6 carbon atoms or a phenyl group, $R^{10}$ represents an alkyl group of 1 to 8 carbon atoms, with the proviso that, when $R^4$ represents a group of the formula $-N(R^{10}2$ or $-\overset{\oplus}{N}(R^{10})_3 \cdot \overset{\ominus}{Y}$, two or more of $R^{10}$ may be same or different to

each other, and $\overset{\ominus}{Y}$ represents an anion of an acid], or a non-toxic acid addition salt thereof. These glycerol derivatives are stated to possess an antagonistic effect on PAF, an hypertensive effect like PAF, an inhibitory effect on phospholipase and an inhibitory effect on the growth of tumour cells or induction of differentiation.

EP-A-147768 describes glycerine derivatives of the formula

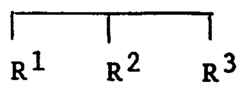

I

wherein one of the residues $R^1$, $R^2$ and $R^3$ is a group U of the formula $OY^1$ or
$X^1$-C(O)-$(A^1)_n$-$Z^1$
another of the residues is a group V of the formula
$OY^2$ or $X^2$-C(O)-$(A^2)_p$-$Z^2$
and the remaining residue is a group W of the formula
$X^3$T-($C_{2-6}$-alkylene)-N(Het)$Q^-$
whereby one of $X^1$, $X^2$ and $X^3$ is oxygen or NH and the other two are oxygen.
$Y^1$ is $C_{10-26}$-alkyl or $C_{10-26}$-alkenyl,
$Y^2$ is $C_{1-6}$-alkyl or $C_{2-6}$-alkenyl,
$C_{3-6}$-cycloalkyl, $C_{5-6}$-cycloalkenyl, phenyl, benzyl or 2-tetrahydropyranyl,
$A^1$ and $A^2$ are oxygen or NH,
n and p are the number 1 or 0,
$Z^1$ is $C_{9-25}$-alkyl or $C_{9-25}$ alkenyl,
$Z^2$ is $C_{1-5}$-alkyl, $C_{2-5}$-alkenyl, phenyl or,
where $(A^2)_p$ is not oxygen. $Z^2$ is also hydrogen,
T is carbonyl, C(O)O or C(O)NH or, where $X^3$ is oxygen,
T is also methylene,
-$\overset{+}{N}$(Het) is a 5- to 7-membered aromatic heterocyclic residue, optionally with an additional O-, S- or N-atom, optionally with a fused benzene ring and optionally monosubstituted by hydroxy, $C_{1-4}$-alkyl, $C_{1-4}$-alkoxy, 2-(hydroxy or amino)-ethyl, carbamoyl or ureido, and
Q is the anion of a strong inorganic or organic acid and their hydrates.

These compounds and their hydrates are stated to inhibit the blood platelet activating factor (PAF) and to be useful in the control of prevention of illnesses such as thrombosis, apoplexy, heart infarct, angina pectoris and bronchial asthma caused by allergies, and also as antiinflammatories and antirheumatics.

The present inventors, after intensive search into lipid derivatives having the PAF inhibitory activity which are valuable as a preventive and therapeutic agent for a variety of circulatory disturbances and diseases and allergic disorders and also as an antineoplastic agent, succeeded in the production of lipid derivatives exhibiting excellent activities and have come to complete the present invention.

The present invention relates to lipid derivatives of the formula:

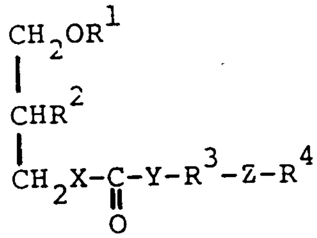

(I)

[wherein $R^1$ is $C_{10-30}$ alkyl or a group of the formula:

$R^5$NHCO-

(in which $R^5$ is $C_{10-30}$ alkyl); $R^2$ is hydrogen, hydroxy, $C_{1-5}$ alkoxy, phenyl-$C_{1-5}$ alkoxy, $C_{1-5}$ alkanoyloxy, benzyloxy, phenoxycarbonyloxy, $C_{1-5}$ alkoxycarbonyloxy, a group of the formula:

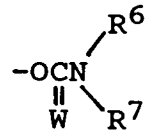

(in which W is oxygen or sulfur, and $R^6$ and $R^7$ are independently hydrogen or $C_{1-5}$ alkyl or both, taken together with the adjacent nitrogen atom, form a 3- to 7-membered hetero ring), amino, $C_{1-5}$ alkanoylamino, benzoylamino, 3- to 7-membered monocyclic amino or $C_{8-9}$ fused cyclic amino; said 3- to 7-membered monocyclic animo or $C_{8-9}$ fused cyclic amino group being unsubstituted or substituted by one or two oxo groups; $R^3$ is a chemical binding or $C_{1-8}$ alkylene unsubstituted or substituted by $C_{1-4}$ alkoxycarbonyl or carboxylato; $R^4$ is hydrogen, $C_{1-6}$ alkyl, $C_{2-6}$ alkenyl or phenyl-$C_{1-6}$ alkyl; X is O or a group of the formula:

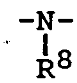

(in which $R^8$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl); Y is O or a group of the formula:

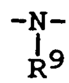

(in which $R^9$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl), and $R^8$ and $R^9$, or $R^4$ and $R^9$ may form $C_{1-4}$ alkenylene or alkylene, each of said groups being unsubstituted or substituted by one or two oxo groups; Z is a nitrogen-containing heterocyclic ring which may be substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl, $R^3$ being bound to a position other than the nitrogen atom in said nitrogen-containing heterocyclic ring; provided that X is O, then Y is a group of the formula:

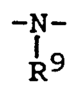

in which $R^9$ is as defined above] or a pharmaceutically acceptable salt thereof.

With reference to the above formula (I), the $C_{10-30}$ alkyl group represented by $R^1$ may be straight-chain or branched-chain ones, such as decyl, undecyl, tridecyl, tetradecyl, pentadecyl, hexadecyl, heptadecyl, octadecyl, nonadecyl, eicosanyl, heneicosanyl, docosanyl, tricosanyl, tetracosanyl, pentacosanyl, hexacosanyl, heptacosanyl, octacosanyl, nonacosanyl, triacontanyl, farnesyl and dihydrophytyl, among others, alkyl groups of 14 to 20 carbon atoms are preferable, with alkyl groups of 14 to 18 carbon atoms being more preferable, with alkyl groups of 15 to 18 carbon atoms being still more preferable, with alkyl groups of 16 to 18 carbon atoms being most preferable. In cases in with $R^1$ is an alkylcarbamoyl group, which can be expressed as the formula:

$R^5$NHCO- (II)

wherein $R^5$ includes alkyl groups having 10 to 30 carbon atoms as is the case with the alkyl groups represented by $R^1$ as mentioned above; among others, preferred are those having an alkyl group having 14 to 20 carbon atoms as the alkyl group in the alkylcarbamoyl group, and those having an alkyl group of 14 to 18 carbon atoms are more preferred, and those having an alkyl group of 15 to 18 carbon atoms are still more preferred and those having an alkyl group of 16 to 18 carbon atoms are most preferred.

As the $C_{1-5}$ alkoxy group represented by $R^2$, there may be mentioned alkoxy groups such as methoxy,

ethoxy, propoxy, isopropoxy, butoxy, isobutoxy and pentoxy.

As the phenyl-$C_{1-5}$ alkoxy group represented by $R^2$, there may be mentioned alkoxy groups such as benzyloxy, phenethyloxy, α-methylbenzyloxy, α-methylphenethyloxy and β-methylphenethyloxy.

As the $C_{1-5}$ alkanoyloxy group represented by $R^2$, there may be mentioned alkanoyloxy groups such as formyloxy, acetyloxy, propionyloxy, butyryloxy, isobutyryloxy, valeryloxy and isovaleryloxy, and acyloxy groups, such as benzoyloxy, phenoxycarbonyloxy and $C_{1-5}$ alkoxycarbonyloxy (e.g., methoxycarbonyloxy, ethoxycarbonyloxy, propoxycarbonyloxy, butoxycarbonyloxy, etc.).

Referring to the formula

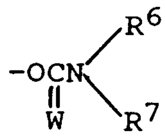

the $C_{1-5}$ alkyl group represented by $R^6$ or $R^7$ includes alkyl groups such as methyl, ethyl, propyl, isopropyl, butyl, isobutyl and pentyl. The 3- to 7-membered hetero ring which $R^6$ and $R^7$, taken together with the adjacent nitrogen atom, form includes 3- to 7-membered heterocyclic rings which may have hetero atoms, such as nitrogen, oxygen and sulfur atoms, in addition to the said nitrogen atom, such as 1-aziridinyl, 1-azetidinyl, 1-pyrrolidinyl, piperidino, 1-perhydroazepinyl, 1-piperazinyl, morpholino, thiomorpholino, 1-perhydrodiazepinyl, 4-perhydrooxazepinyl and 4-perhydrothiazepinyl,

As the $C_{1-5}$ alkanoylamino group represented by $R^2$, there may be mentioned alkanoylamino groups such as formamido, acetamido, propionamido, butanamido, isobutanamido, valeramido and isovaleramido.

As the 3- to 7-membered mono-cyclic amino represented by $R^2$, there by be mentioned 1-aziridinyl, 1-azetidinyl, 1-pyrrolidinyl, piperidino, 1-perhydroazepinyl, 1-piperazinyl, morpholino, thiomorpholino, 1-perhydrodiazepinyl, 4-perhydrooxazepinyl and 4-perhydrothiazepinyl, and as the $C_{8-9}$ fused cyclic amino, 2-isoindolinyl. The said heterocyclic and fused rings may be substituted by one or two oxo groups, at their positions susceptible to substitution, and the substituted mono-cyclic and fused rings include, for example, 2,5-dioxopyrrolidinyl and 1,3-dioxoisoindolinyl.

The compounds having alkoxy as $R^2$ are more preferable.

The $C_{1-8}$ alkylene chain represented by $R^3$ includes straight-chain or branched-chain alkylene chains and there may be mentioned, for example, methylene, ethylene, trimethylene, tetramethylene, pentamethylene, hexamethylene, heptamethylene, and octamethylene. The said alkylene chains may be substituted by, for example, $C_{1-4}$ alkoxycarbonyl [e.g. methoxycarbonyl, ethoxycarbonyl] or carboxylato, and the case that said substituent is bound to the position adjacent to the carbon atom of the group $R^3$ being bound to the group Z is preferable. As $R^3$, among others methylene, ethylene and trimethylene are preferable, and methylene or ethylene is more preferable.

The $C_{1-6}$ alkyl group represented by $R^4$ includes groups such as methyl, ethyl, propyl, isopropyl, butyl, isobutyl, sec-butyl, tert-butyl, pentyl and hexyl. $C_{2-6}$ alkenyl groups include groups such as vinyl, allyl, 2-butenyl and 3-butenyl.

The phenyl-$C_{1-6}$ alkyl group represented by $R^4$ includes groups such as benzyl, phenethyl, phenylpropyl, phenylbutyl, α-methylphenethyl and β-methylphenethyl.

In cases in which X is a group represented by the formula:

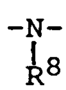

(IV)

the $C_{1-5}$ alkyl group represented by $R^8$ includes alkyl groups such as methyl, ethyl, propyl, butyl and pentyl, wherein the said alkyl groups may be substituted, by carboxyl or $C_{1-5}$ alkoxycarbonyl (e.g., methoxycarbonyl, ethoxycarbonyl, propoxycarbonyl, butoxycarbonyl, pentoxycarbonyl, etc.)

The $C_{1-5}$ alkanoyl group represented by $R^8$ includes, for example, formyl, acetyl, propionyl, butyryl, isobutyryl, valeryl, isovarelyl. The $C_{1-5}$ alkoxycarbonyl includes methoxycarbonyl, ethoxycarbonyl, propoxycarbonyl, butoxycarbonyl and pentoxycarbonyl.

The mono $C_{1-5}$ alkylcarbamoyl group represented by $R^8$ includes, for example ethylcarbamoyl, propylcarbamoyl, butylcarbamoyl. The di-$C_{1-5}$ alkylcarbamoyl includes, for example, dimethylcarbamoyl, methylethylcarbamoyl, diethylcarbamoyl, methylpropylcarbamoyl. The 3- to 7-membered cyclic amino

carbonyl includes, for example, (aziridin-1-yl)carbonyl, (azetidin-1-yl)carbonyl, (pyrrolidin-1-yl)carbonyl, piperidinocarbonyl, (perhydroazepin-1-yl)carbonyl, (piperazin-1-yl)carbonyl, morpholinocarbonyl, thiomorpholinocarbonyl.

In cases in which Y is represented by the formula:

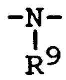 (V)

The group represented by $R^9$ includes groups like those exemplified for $R^8$.

In cases in which X and Y both are an imino group, $R^8$ and $R^9$ may be the same or different, and $R^8$ and $R^9$ may combine with each other to form $C_{1-4}$ alkenylene or alkylene.

The $C_{1-4}$ alkenylene and alkylene bridges which $R^8$ and $R^9$ combine with each other to form include alkenylene and alkylene bridges such as methylene, ethylene, trimethylene, tetramethylene, vinylene and propenylene, and may be substituted by one or two oxo groups at their positions susceptible to substitution. The substituted alkylene and alkenylene include, for example, 1-oxoethylene, 3-oxopropenylene and 1,2-dioxoethylene. Specifically, the group represented by the formula:

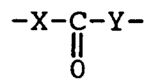

includes;

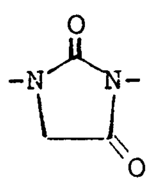, 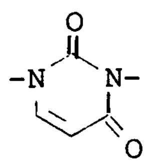, 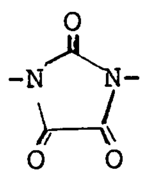 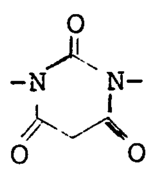

In cases in which Y is an imino group, $R^9$ and $R^4$ may combine with each other to form alkenylene and alkylene, whereby the alkenylene and alkylene bridges which $R^9$ and $R^4$ combine with each other to form include lower alkenylene and alkylene bridges having 1 to 4 carbon atoms, such as methylene, ethylene, trimethylene, tetramethylene, vinylene and propenylene, and these groups may be substituted by one or two oxo groups at their positions susceptible to substitution. The substituted alkylene and alkenylene groups include, for example, 1-oxoethylene, 3-oxopropenylene and 1,2-dioxoethylene.

As X, is preferable O.

Y includes preferably an imino group, which may be substitited more preferably a substituted imino group still more preferably an imino group substituted by lower alkanoyl.

The nitrogen-containing heterocyclic ring represented by Z includes heterocyclic rings containing at least one nitrogen atom, such as monocyclic or bicyclic heterocyclic rings which may contain, as ring forming atom, a nitrogen, oxygen or sulfur atom in addition to the said nitrogen atom. The said heterocyclic rings may be any of saturated one, partially saturated one or such minimum hydrogenated one as heteroaromatic rings, and their examples include azetidinyl, pyrrolidinyl, piperidinyl, perhydroazepinyl, pyrrolinyl, pyrazolinyl, pyrrolyl, pyridyl, oxazolyl, thiazolyl, pyridazinyl, pyrimidyl, pyrazinyl, imidazolyl, morpholinyl, thiomorpholinyl, piperazinyl, pyrazolidinyl, indolyl, isoindolyl, 1H-indazolyl, purinyl, quinolinyl, isoquinolinyl, 1,2,3,4-tetrahydroquinolinyl, perhydroindolyl and perhydroisoquinolinyl; in the case of monocyclic heterocyclic rings, among others, the 4- to 7-membered rings are preferable, and 5- to 6-membered rings are more preferable and thiazolyl or pyridyl is most preferable. These groups may be substituted at their positions susceptible to substitution by $C_{1-4}$ alkyl (e.g., methyl, ethyl, propyl, butyl, etc.), hydroxyl, amino(imino), mono- or di $C_{1-4}$ alkylamino (e.g., methylamino, dimethylamino, etc.), carbamoyl, ureido, carboxyl, carboxylato and $C_{1-4}$ alkoxycarbonyl (e.g. methoxycarbonyl), and their examples include N-methylmorpholinyl, N-methylpiperidinyl, N-methylpiperazinyl, N-methylpyrrolidinyl, N-ethylpyrrolidinyl.

The nitrogen atom in the said nitrogen-containing heterocyclic ring may be converted into a quaternary salt with $R^4$, and there may be mentioned, for example, groups, such as N, N-dimethylpyrrolidinio, N-methylpyridinio, N-ethylpyridinio, N-propylpyridinio, N-butylpyridinio, N-methyl-N-ethylpyrrolidinio, 3-methylthiazolio, 3-ethyl-thiazolio, 3-propylthiazolio, 3-butylthiazolio, N-allyl-pyridinio, N-ethoxycarbonylmethylpyridinio.

In case that $R^4$ is $C_{1-6}$ alkyl, $C_{1-4}$ alkoxycarbonyl-$C_{1-6}$ alkyl, carboxylato-$C_{1-6}$-alkyl or aralkyl, the position in the nitrogen-containing heterocyclic ring represented by Z to which $R^4$ is bound may be any positions if they are the positions susceptible to such binding, but the case that the position in said heterocyclic ring to which $R^4$ is bound is the nitrogen atom is preferable.

The group represented by Z-$R^4$ includes, for example, optionally $C_{1-6}$ alkyl, $C_{1-4}$-alkoxycarbonyl-$C_{1-6}$ alkyl, carboxylato-$C_{1-6}$ alkyl or phenyl-$C_{1-6}$ allyl substituted azetidinyl, pyrrolidinyl, piperidinyl, perhydroazepinyl, pyrrolinyl, pyrazolinyl, pyrrolyl, pyridyl, oxazolyl, thiazolyl, pyridazinyl, pyrimidyl, pyrazinyl, imidazolyl, morpholinyl, thiomorpholinyl, piperazinyl, pyrazolidinyl, indolyl, isoindolyl, 1H-indazolyl, purinyl, quinolinyl, isoquinolinyl, perhydroindolyl, perhydroisoquinolinyl. These groups may be further substituted by, for example, optionally hydroxy or amino substituted $C_{1-4}$ alkyl, hydroxy, amino(imino), mono- or di-$C_{1-4}$-alkylamino, carbamoyl, ureido, carboxy, carboxylato or $C_{1-4}$ alkoxycarbonyl.

With reference to the example of $R^4$ combining with the imino group represented by Y, specifically, the group as represented by the formula:

$-Y-R^3-Z-R^4$

includes;

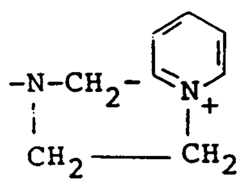
,
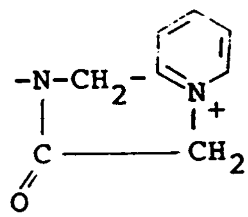

and the like.

As the nitrogen-containing heterocyclic ring represented by Z, the heterocyclic rings containing the quaternary nitrogen atom are more preferable.

The position in the heterocyclic ring to which $R^3$ is bound may be any position, other than the nitrogen atom [e.g. 2-pyridyl, thiazol-2-yl, thiazol-4-yl, N-methylpyridinio-2-yl, N-methylpyridinio-3-yl, N-ethylpyridinio-2-yl, N-butylpyridinio-2-yl, N-methoxycarbonylmethylpyridinio-2-yl, N-ethylpiperidin-2-yl, N-methyl-N-ethylpyperidinio-2-yl, N-ethylpiperidin-3-yl, N-methyl-N-ethylpiperidinio-3-yl, 3-methylthiazolio-2-yl, 3-ethylthiazolio-2-yl, 3-propylthiazolio-2-yl, 3-butyl-thiazolio-2-yl, 3-methylthiazolio-4-yl, 3-ethylthiazolio-4-yl, 3,4-dimethylthiazolio-5-yl, N-allylpyridinio-2-yl], but the positions adjacent to the nitrogen atom are more preferable.

The salt of the compound (I) includes pharmaceutically acceptable salts, such as hydrochlorides, hydrobromides, hydroiodides, sulfates, nitrates and phosphates, and among others, the acid additions salts are preferable. In cases in which Z has the quaternary nitrogen atom, the compound (I) may form salts with pharmaceutically acceptable anions such as acid anions (e.g. chlorine ion, bromine ion, iodine ion, sulfate ion, nitrate ion, phosphate ion and acetate ion) and hydroxyl ion, and may also form intramolecular salts.

The said salts with pharmaceutically acceptable anions and intramolecular salts fall within the scope of the pharmaceutically acceptable salt.

The compound (I), in some instances, may have an asymmetric carbon in the molecule, depending upon the type of the substituent represented by $R^2$, and when the compound exists in two kinds of the stereoisomers with the R- and S-configurations, their individual isomers and mixture thereof both fall within the scope of the present invention.

The compound (I) and its salt exhibits excellent PAF inhibitory activity, and are useful as a prophylactic and therapeutic agent against circulatory disturbances and diseases caused by PAF, such as thrombosis, cerebral apoplexy (e.g., cerebral hemorrhage, cerebral thrombosis, etc.), myocardial infarction, angina pectoris, thrombophlebitis, glomerulonephritis and shock (e.g., endotoxin shock, endotoxin-associated intravascular hemagglutination syndrome, anaphylactic shock, hemorrhagic shock, etc.) and disorders (e.g., bronchial asthma, etc.) associated with allergy as well as an antineoplastic agent.

The compound (I) and its salt are so hydrophilic and lipophilic and so low in toxicity, and can therefore be safely administered orally or parenterally, as it is in a form of powder or as a pharmaceutical

composition in the suitable dosage form, to mammals. The dosage varies depending upon the subject to be administered, disease to be treated, conditions thereof and route of administration, and when the compound (I) or its salt is to be administered through intravenous injection for the prophylaxis or treatment of shock in a human adult, for example, it can be advantageously administered usually in a single dose in the range of about 0.01 to 20 mg/kg body weight, preferably in the range of about 0.1 to 10 mg/kg body weight, more preferably in the range of 0.1 to 2 mg/kg body weight, about once to 5 times a day, preferably about once to 3 times a day. In addition, the compound (I) or its salt can be administered through drip infusion in a single dose in the range of about 0.01 to 1.0 mg/kg body weight/min. over a period of about 1 hour, about once to 5 times a day, preferably about once to 3 times a day. The dosages for other non-oral routes as well as the oral dosage may be selected referring to the above-mentioned dose-levels. When shock symptoms are very serious, dosage increases may be made according to the severity of the symptoms.

When the compound (I) or its salt is administered orally for the prophylaxis and treatment of thrombosis in an human adult, for example, it can be advantageously administered usually in a single dose in the range of about 0.1 to 20 mg/kg body weight, about once to 5 times a day, preferably about once to 3 times. More particularly, it is preferable to administer it in a single dose in the range of about 0.5 to 4 mg/kg body weight for the purpose of prophylaxis of thrombosis and in a single dose in the range of about 4 to 10 mg/kg body weight for the purpose of treatment of thrombosis, respectively, about once to 3 times a day. The dosages for other non-oral routes may be selected referring to the above-mentioned dose-levels.

The pharmaceutical composition to be used for the above administration comprises an effective amount of the compound (I) or its salt and a pharmaceutically acceptable carrier or excipient, and the said composition is provided in a dosage form suitable for oral or non-oral administration.

The composition for oral administration includes, for example, solid or liquid dosage forms, and as their specific examples, there may be mentioned tablets (inclusive of sugar-coated tablets and film-coated tablets), pills, granules, powders, capsules (inclusive of soft capsules), syrups, emulsions, suspensions, etc. Such compositions can be manufactured by the *per se* known procedures and comprise carriers and excipients commonly used in the pharmaceutical industry. Examples of the carriers and excipients for the preparation of tablets include lactose, starch, sugar and magnesium stearate, etc.

The compositions for non-oral administration include for example, injections and suppositories, and as examples of the injection, there may be mentioned dosage forms, such as injectable solutions for intravenous injection, for subcutaneous injection, for intracutaneous injection, for intramuscular injection and for drip injection. Such injectable solutions are prepared by the *per se* known procedure, for example, by dissolving, suspending or emulsifying the compound (I) or its salt in a sterile aqueous or oily solution usually used for injectable solutions. The aqueous solution for injection includes, for example, physiological saline solution, isotonic solution containing glucose and other adjuvants, and may be employed in combination with a suitable solubilizer, such as alcohols (e.g., ethanol, etc.), polyalcohols (e.g., propylene glycol, polyethylene glycol, etc.), and nonionic surface active agents [e.g., polysorbate 80, HCO-50 (polyoxyethylene (50 mol) adduct of hydrogenated castor oil), etc.]. The oily solution for injection includes, for example, sesame oil and soybean oil, and may be used in combination with such a solubilizer as benzyl benzoate and benzyl alcohol. The injectable solution prepared is usually filled into suitable ampoules to be supplied as an injection. The suppositories for rectal administration are produced by the *per se* known procedure, for example, by incorporating the compound (I) or its salt into a usual suppository base, followed by compression into a shape.

Each of the above-mentioned compositions may contain other active ingredients, unless they bring about unfavorable interactions with the compound (I) or its salt.

The compound (I) or its salt can be produced, for example, by the following methods.

A) A compound of the formula:

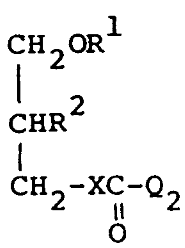

(IX)

[wherein $Q_2$ is a carbonyl-activating group (e.g., halogen (e.g., chlorine, etc.), phenoxy, etc): other symbols are as defined hereinbefore] is reacted with a compound of the formula:

HY-$R^3$-Z-$R^4$ (X)

[wherein each of the symbols is as defined hereinbefore] to give the compound (I). The reaction between (IX) and (X) can be carried out in the presence or absence of a solvent at -10 to +150 °C. As the solvent, there can be used, for example, toluene, benzene, ether, dioxane, tetrahydrofuran and chloroform, and in order to accelerate the reaction, a base such as triethylamine and pyridine may be added. Also, (X) may be reacted with sodium hydride, n-butyllithium, etc. in the above mentioned solvent to convert into a metal salt, and be reacted with the compound (IX).

B) A compound of the formula:

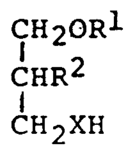 (XI)

[wherein $R^1$, $R^2$ and X are as defined hereinbefore] is reacted with a compound of the formula:

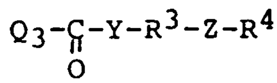 (XII)

[wherein $Q_3$ is a carbonyl-activating group (e.g., halogen (e.g., chlorine, etc.), phenoxy, etc.); other symbols are as defined hereinbefore] to give the compound (I). The reaction between the compounds (XI) and (XII) can be conducted in a manner similar to that of the reaction between the compounds (IX) and (X) in the above B.

C) A compound of the formula:

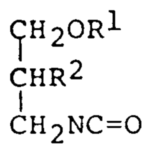 (XIII)

[wherein $R^1$ and $R^2$ are as defined hereinbefore] is reacted with the compound represented by the formula (X) to give (I) (X = NH). The reaction can be carried out in a manner similar to that of the reaction between (IX) and (X).

D) A compound of the formula:

O = C = N-$R^3$-Z-$R^4$ (XIV)

[wherein the symbols are as defined hereinbefore] is reacted with a compound represented by the formula (XI) to give (I) (Y = NH). The reaction can be conducted in a manner similar to that of the reaction between (XI) and (XII)

The compound (XIV) can be readily synthesized, for example, by reacting a compound represented by the formula $H_2N$-$R^3$-Z-$R^4$ (XV) [wherein each of the symbols is as defined hereinbefore] with diphosgene at -20 to +120 °C in the presence or absence of an inert solvent, such as methylene chloride, chloroform, benzene, tetrahydrofuran and toluene or by reacting a compound represented by the formula HOOC-$R^3$-Z-$R^4$ (XVI) [wherein each of the symbols is as defined hereinbefore] with DPPA at 0 to +150 °C in a solvent such as chloroform, toluene, benzene, dichloromethane and tetrahydrofuran in the presence of a tertiary amine, such as triethylamine and tributylamine, and further reacting those at 0 to 150 °C in the presence of a tertiary amine, such as pyridine.

The compound of the formula (I) where X and/or Y is an unsubstituted imino group can be reacted, for example, with an acid anhydride, acid halide, alkyl halide or alkyl isocyanate corresponding to $R^8$ or $R^9$ to give the compound of the formula (I) wherein X and/or Y is a substituted imino group. The reaction is generally allowed to proceed by maintaining the reaction temperature within the range of -10 °C to +150 °C in a solvent (e.g., benzene, toluene, chloroform, dichloromethane, ether, tetrahydrofuran, dimethylsulfoxide,

dimethylformamide, etc.). In such a case, a base (e.g., triethylamine, pyridine, dimethylaminopyridine, sodium hydroxide, sodium hydride, etc.) can be allowed to coexist in the reaction for the purpose of accelerating the reaction rate.

The compound of the formula (I) wherein the nitrogen atom contained in a group represent by Z is a primary, secondary or tertiary amino can also be reacted, for example, with an alkyl halide to give the compound of the formula (I) wherein the nitrogen atom contained in a group represented by Z is a secondary, tertiary or quaternary amino. This reaction is allowed to proceed by maintaining the reaction mixture at a temperature of 0° to +150°C in a solvent, such as ether, chloroform, tetrahydrofuran, benzene and toluene, in the presence of an equal amount or large excess of an alkyl halide or aralkyl halide.

A compound of the formula (I) wherein $R^1$ is hydrogen is reacted with an alkyl isocyanate to give a compound of the formula (I) wherein $R^1$ is alkylcarbamoyl. This reaction can be conducted in a manner similar to that of the reaction between (IX) and (X).

When a group readily susceptible to elimination is contained in the formula (I), such a group can be allowed to undergo elimination, followed by a subsequent reaction to introduce other substituents. When $R^2$ in the formula (I) is a benzyloxy group, for example, catalytic reduction is conducted to convert $R^2$ into a hydroxy group, and then acylation or carbamoylation can be carried out.

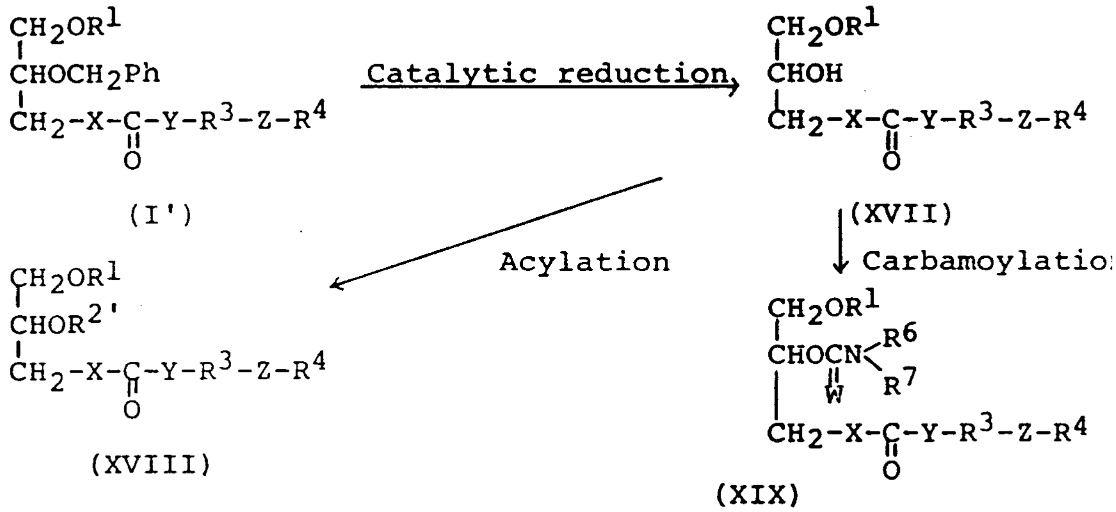


$R^{2'}$ is an acyl group.

In this reaction, when X and/or Y is an unsubstituted imino group, X and/or Y can also be acylated or carbamoylated simultaneosuly with the above acylation or carbamoylation. The catalytic reduction reaction in this reaction can be carried out by maintaining the reaction mixture at a temperature of room temperature to +100°C in a solvent, such as alcohol, tetrahydrofuran, water and acetic acid, with use of a catalyst such as platinum oxide, palladium-carbon and Raney nickel. The acylation reaction of (XVII) can be carried out by maintaining a mixture of (XVII) and an active derivative of carboxylic acid (e.g., acid anhydrides, acid halides, etc.) at a temperature of -10° to +150°C in an inert solvent (e.g., ether, chloroform, benzene, toluene, dichloromethane, tetrahydrofuran, dimethylformamide, etc.). In such a case, a tertiary amine (e.g., triethylamine, pyridine, dimethylaminopyridine, etc.) and the like may be added for the purpose of accelerating the reaction. The reaction of carbamoylating (XVII) to convert into (XIX) can be conducted by a procedure similar to that of the carbamoylation reaction [the method of producing the compound (XLIV)] of the starting compound to be described hereinafter.

(IX) and (XII) can be synthesized, for example, by reacting (XI) and (X) respectively with phenyl chlorocarbonate, phosgene or diphosgene.

The compound (XI) can be synthesized for example by the following method.

(Descriptions will be given below with regard to the individual cases in which X is O and NH, respectively.)

The compound of the formula:

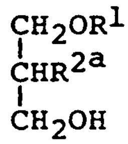

(XXXV)

[wherein $R^{2a}$ is an acyloxy group; $R^1$ is as defined hereinbefore] can be produced, for example, by the following reaction schema:

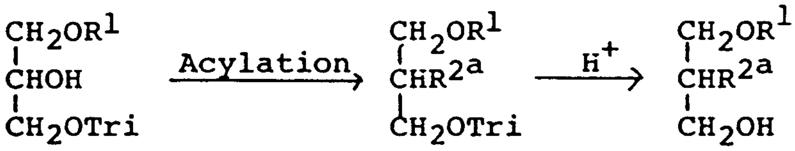


(XXXVI) (XXXVII) (XXXV)

The compound of the formula:

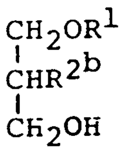

(XXXVIII)

[wherein $R^{2b}$ is an acylamino group; $R^1$ is as defined hereinbefore] can be produced, for example, by the following reaction schema:

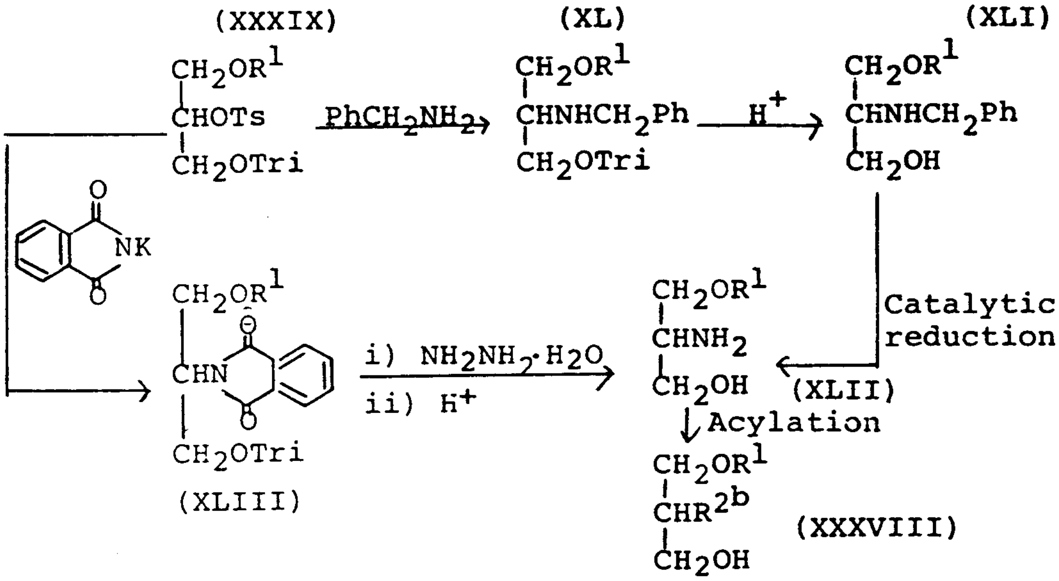


(XXXIX) (XL) (XLI) (XLIII) (XLII) (XXXVIII)

The compound of the formula:

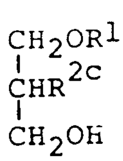

(XLIV)

[wherein $R^{2c}$ is a group represented by the formula (III); $R^1$ is as defined hereinbefore] can be produced, for example, by the following reaction schema.

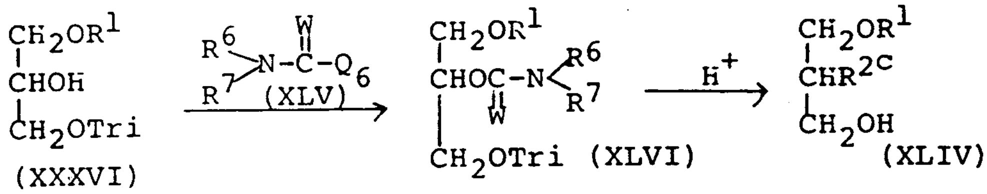


[wherein $Q_6$ is a halogen atom; other symbols are as defined hereinbefore]

The reaction (XXXVI)→(XLVI) can be preferably conducted without a solvent or in an inert solvent (e.g. toluene, benzene, chloroform, dichloromethane, tetrahydrofuran) at 0 to 150 ° C, preferably in the presence of a tertiary amine, such as pyridine, triethylamine, dimethyllaminopyridine. The reaction (XLVI)→(XLIV) can be allowed to proceed generally in water or an alcohol at +10 to +110 ° C in the presence of an acid (e.g. hydrochloric acid, acetic acid).

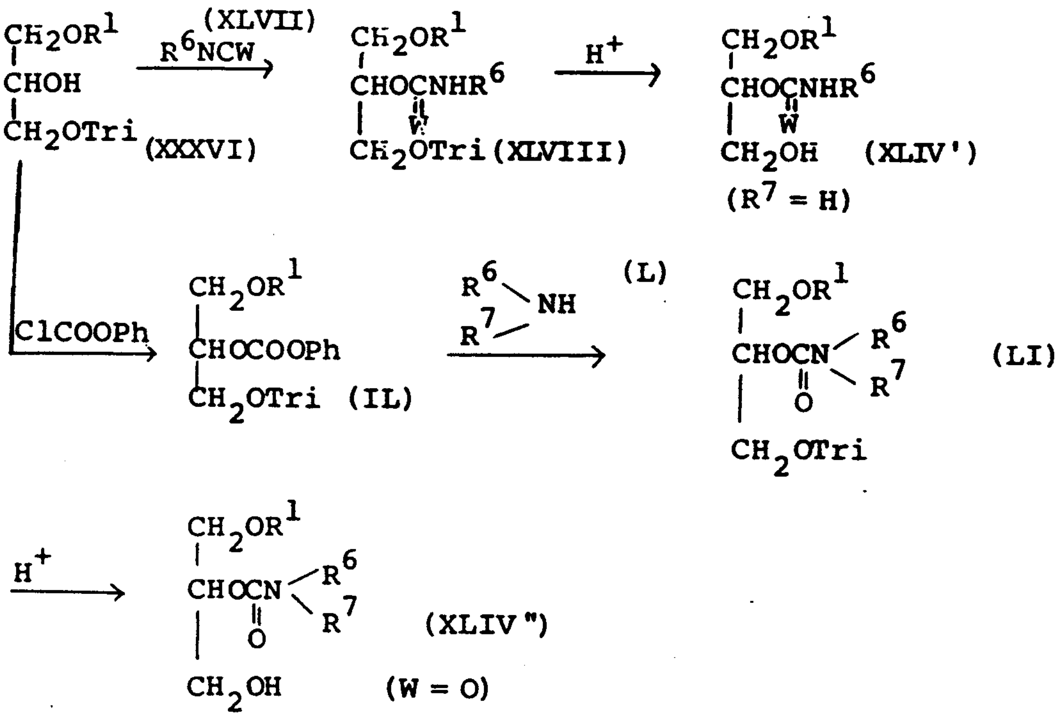


The reaction (XXXVI) → (XLVII) can be preferably conducted without a solvent or in an inert solvent, such as benzene, toluene, chloroform, dichloromethane and tetrahydrofuran at 0 to +150 ° C, preferably in the presence of a tertiary amine such as pyridine, and the reaction (XXXVI) → (LI) can be preferably conducted by reacting, without a solvent or in an inert solvent such as benzene, toluene, chloroform, dichloromethane and tetrahydrofuran at 0 to +150 ° C, the compound (L) with the compound (IL) as provided by reacting (XXXVI) and phenyl chlorocarbonate in an inert solvent (e.g., chloroform, dichloromethane, benzene, toluene, diethyl ether) at 0 to +100 ° C.

The compound of the formula:

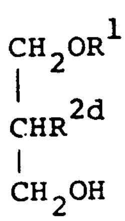

(LII)

[wherein $R^{2d}$ is alkoxy or aralkyloxy; $R^1$ is as defined hereinbefore] can be produced, for example, by the following reaction schema.

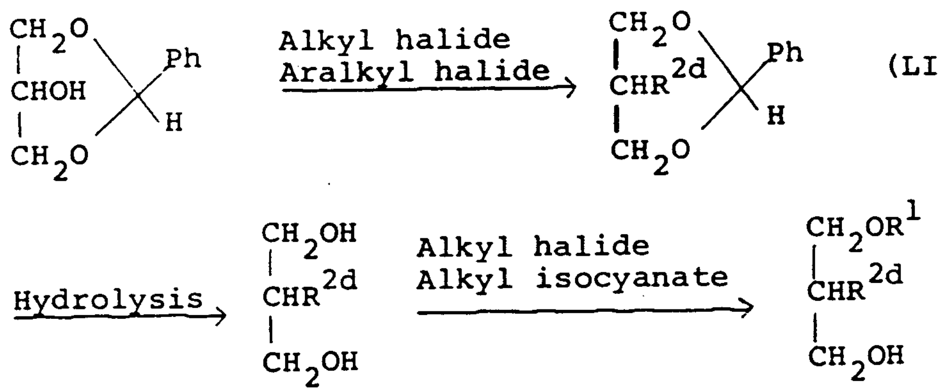


(LIII)

(LIV)

(LII)

The compound of the formula:

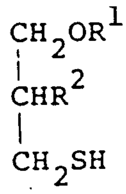

(LV)

[wherein $R^1$ and $R^2$ are as defined hereinbefore] can be produced, for example, by the following reaction schema.

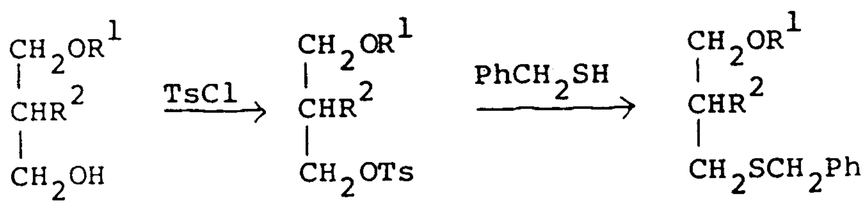


(XXV) (LVI) (LVII)

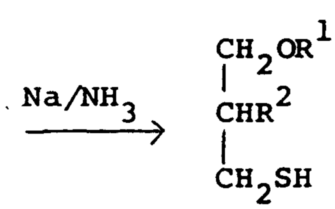


(LV)

The compound of the formula:

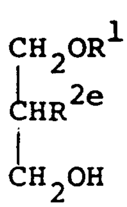

(LVIII)

[wherein $R^1$ is as defined hereinbefore, $R^{2e}$ is cyclic amino] can be produced, for example, by the following reaction schema.

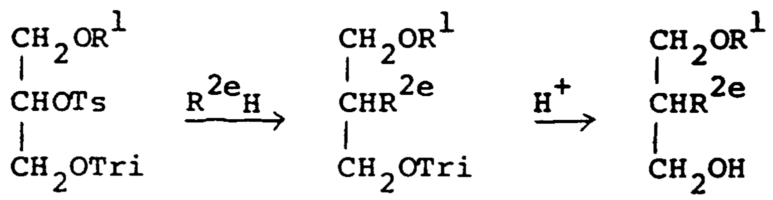


(XXXIX) (LIX) (LVIII)

In the above reaction schema, $R^{2e}$ is cyclic amino, and the symbols Ts and Tri are tosyl and trityl, respectively.

The compound of the formula:

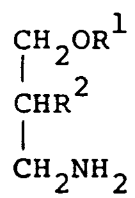

(LX)

[wherein $R^1$ and $R^2$ are as defined hereinbefore] can be produced, for example, by the following reaction schema.

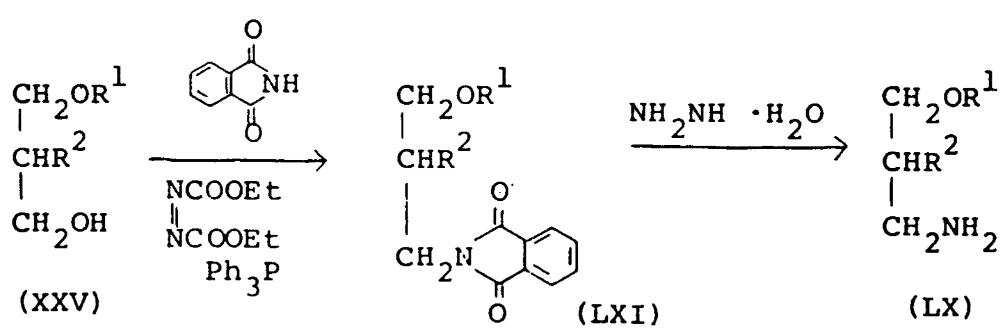


(XXV) (LXI) (LX)

In each of the above reaction schema, the symbols Ph, Ts, Tri and DPPA are phenyl, tosyl, trityl and diphenylphosphorylazide, respectively.

With reference to the compounds to be used in each of the reactions, any compound having a group liable to affect adversely the reaction can be subjected to the reaction after having the said group protected with the per se known protective group (e.g., benzyl, tosyl, trityl, phthalimide, succinimide, benzyloxycarbonyl, tert-butoxycarbonyl, etc.), and thereafter subjected to the per se known deprotection reaction to give the objective compound.

The salt of the compound (I), in some instances, can be obtained for example by the above-mentioned processes for producing the compound (I)themselves, but can also be produced by adding an acid or base to the compound (I), if desired, and the form of a salt can be converted into another one with ion-exchange resin.

The invention will be further illustrated in more detail by the following Examples, Experiment Examples and a Preparation Example, which, however, are by no means limitative of the present invention.

Example 1

3-O-[N-Acetyl-N-(1'-ethylpyrrolidin-2'-yl)methyl]carbamoyl-2-O-methyl-1-O-octadecylcarbamoylgycerol-hydrochloride.

i) 3-O-[N-(1'-Ethylpyrrolidin-2'-yl)methyl]carbamoyl-2-O-methyl-1-O-octadecylcarbamoylglycerol

A 347 µl (2.4 mmole) portion of 2-(aminomethyl)-1-ethylpyrrolidine was added to the crude carbonate as synthesized from 803 mg (2 mmole) of 2-O-methyl-1-O-octadecylcarbamoylglycerol, 345 mg (2.2 mmole) of phenyl chloroformate, 317 mg (4 mmole) of pyridine and 5 ml of methylene chloride, followed by heating under a nitrogen atmosphere at 80 ° C for 16 hours. After cooling, the crude product was purified by column chromatography [silica gel: 50 g; an eluent: n-hexane-ethyl acetate (1:4) and chloroform-methanol (10:1)] to give 1.066 g (95.9 %) of the objective compound (colorless solid).

TLC [silica gel; chloroform-methanol (10:1)] Rf = 0.22

NMR [90 MHz, $CDCl_3$] δ : 0.88(3H,t), 1.10(3H,t), 1.28(32H,s), 1.75(4H,m), 2.10-2.98(5H,m), 3.19(4H,m), 3.46(3H,s), 3.60(1H,quint.), 4.18(4H,d), 4.98(1H,br.t), 5.42(1H,br.)

IR [kBr] $cm^{-1}$: 3330, 2925, 2855, 1695, 1540, 1472, 1260.

ii) 3-[N-Acetyl-N-(1'-ethylpyrrolidin -2'-yl)methyl]carbamoyl-2-O-methyl-1-O-octadecylcarbamoylglycerol-hydrochloride.

In 20 ml of chloroform was dissolved 921 mg (1.657 mmole) of the compound as synthesized in i), and 4 ml of acetic anhydride and 20 ml of triethylamine were added to the solution, followed by heating under reflux at 80 to 100 ° C for 24 hours. After cooling, the reaction solution was concentrated under reduced pressure, and 1 % aqueous $NaHCO_3$ solution was added to the residue, followed by extraction with chloroform. The organic layer was dried over magnesium sulfate, and the solvent was distilled off under reduced pressure. The resulting crude product was purified by column chromatography [silica gel: 50 g; an elument: ethyl acetate-acetone (1:1)] to give 948 mg (95.7 %) of the objective compound (Free Base) (colorless oil). 50 mg of this Free Base was dissolved in ethyl ether, and the solution was treated with hydrogen chloride gas under ice-cooling to give 53 mg of the hydrochloride.

"Free Base"

TLC [silica gel; chloroform-methanol (5:1)]: Rf = 0.56

NMR [90 MHz, $CDCl_3$] δ : 0.88(3H,t), 1.10(3H,t) , 1.26(32H,s), 1.72(4H,m), 2.16-2.98(5H,m), 2.50(3H,s), 3.15(2H,q), 3.45(3H,s), 3.64(1H,m), 3.80(2H,d), 4.08-4.40(4H,m), 4.92(1H,br.).

IR [film] $cm^{-1}$: 3330, 2930, 2850, 1740, 1710, 1535, 1470, 1375, 1250, 1220, 1175, 1140.

Example 2

3-O-[N-Acetyl-N-(1'-ethyl-1'-methylpyrrolidinio-2'-yl)methyl]carbamoyl-2-O-methyl-1-O-octadecylcarbamoylglycerol-iodide.

In 12 ml of ethyl ether was dissolved 607 mg (1.015 mmole) of the compound (Free Base) as synthesized in Example 1-ii), and after 432 mg (3.046 mmole) of methyl iodide was added to the solution, the mixture was allowed to stand at room temperature for 5 days under shielding from light.Petroleum ether was added to reaction solution, and the precipitate, which separated out, was collected by filtration to give 622 mg (82.8 %) of the objective compound (colorless powder).

TLC [silica gel: chloroform-methanol (3:1)] Rf = 0.47

NMR [90 MHz, $CDCl_3$] δ : 0.88(3H,t), 1.26(32H,s), 1.48(3H,t), 1.90-2.40(4H,m), 2.53(3H,s), 3.12(5H,m), 3.47(3H,s), 3.60-4.56(12H,m), 5.15(1H,br.).

IR [KBr] $cm^{-1}$: 3450, 2920, 2850, 1755-1705, 1470, 1260, 1220, 1200.

Example 3

2-O-Methyl-3-O-[( 1'-methylpyridinio-2'-yl)methyl]carbamoyl-1-O-octadecylcarbamoyglycerol-iodide.

i) 2-O-Methyl-3-O-[N-(2'-pyridylmethyl)]carbamoyl-1-O-octadecylcarbamoylglycerol

To 567 mg of the crude carbonate synthesized from 402 mg (1 mmole) of 2-O-methyl-1-O-octadecylcarbamoylglycerol, 172 mg (1.1 mmole) of phenyl chloroformate, 158 mg (2 mmole) of pyridine and 3 ml of methylene chloride were added 122 µl (1.2 mmole) of 2-(aminomethyl)pyridine and 1 ml of chloroform, followed by heating under reflux for 12 hours. The reaction solution was concentrated under reduced

pressure, and the resulting crude product was purified by column chromatography [silica gel; 20 g: an eluent: n-hexane-ethyl acetate (1:3)] to give 454 mg (84.7 %) of the objective compound (colorless solid).

TLC [silica gel; n-hexane-ethyl acetate (1:3)] Rf = 0.21

NMR [90 MHz, $CDCl_3$] δ : 0.87(3H,t), 1.25(32H,s), 3.15(2H,q), 3.43(3H,s), 3.59(1H,quint.), 4.18(4H,m), 4.50(2H,d), 4.90(1H,br,), 6.00(1H,br.), 7.22(2H,m), 7.65(1H,m), 8.52(1H,m).

IR [KBr] $cm^{-1}$: 3320, 2925, 2850, 1695, 1535, 1470, 1260, 1250.

ii) 2-O-Methyl-3-O-[(1'-methylpyridinio-2'-yl)methyl]carbamoyl-1-O-octadecylcarbamoylglycerol-iodide.

In 0.5 ml of chloroform and 6 ml of ethyl ether was dissolved 170 mg (0.317 mmole) of the compound as synthesized in (i), and after 135 mg (0.952 mmole) of methyl iodide was added to the solution, the mixture was allowed to stand at room temperature for 14 days under shielding from light. The precipitate, which separated out, was collected by filtration to give 123 mg (57.1 %) of the objective compound (pale yellow powder).

TLC [silica gel: chloroform-methanol(3:1)] Rf = 0.20

NMR [90 MHz, $CDCl_3$] δ : 0.87(3H,t), 1.24(32H,s) , 3.12(2H,q), 3.42(3H,s), 3.59(1H,quint.), 4.15(4H,m), 4.55(3H,s), 4.89(2H,d), 5.20(1H,br.), 7.04(1H,br.), 7.85-8.23(2H,m), 8.46(1H,t), 9.19(1H,d).

IR [KBr] $cm^{-1}$: 3340, 2920, 2850, 1698, 1635,1530, 1470, 1260

Example 4

2-Methoxy-3-octadecylcarbamoyloxypropylamine

A 3.48 g (20 mmole) portion of diethyl diazocarboxylate was added dropwise to a solution of 4 g (10 mmole) of 2-methoxy-3-octadecylcarbamoyloxy-1-propanol, 2.94 g (20 mmole) of phthalimide and 5.24 g (20 mmole) of triphenylphosphine in 100 ml of anhydrous tetrahydrofuran, followed by further stirring at room temperature for 40 hours. The reaction solution was concentrated to dryness under reduced pressure, and the residue was subjected to separation and purification by chromatography on a column of 100 g of silica gel. From the eluate of n-hexane-ethyl acetate (4:1), there was obtained 5.7 g of the crude phthalimide derivative. 100 ml of methanol and 0.5 ml of hydrazine hydrate were added to the compound, followed by heating under reflux for 2 hours. The reaction solution was concentrated to dryness under reduced pressure, and chloroform was added to the residue. The insoluble matter was filtered off, and the filtrate was concentrated to dryness under reduced pressure. The residue was subjected to separation and purification by chromatography on a column of 80 g of silica gel.
From the eluate of chloroform-methanol-triethylamine (95:5:0.125), there was obtained the objective compound in the form of a colorless powder. Yield of 2.7 g (68 %).

IR (KBr) $cm^{-1}$: 3350($NH_2$), 2915(CH), 2845(CH), 1690(C=O), 1520(CONH), 1469($CH_2$), 1273.

NMR δ ($CDCl_3$): 0.87(3H), 1.23(32H), 2.7-2.95(2H,$C\underline{H_2}NE_2$), 3.03-3.4(3H,CH+CONHC$\underline{H_2}$), 3.43-(3H,s,$OCH_3$), 4.14(2H,d,J=4.8Hz, $CH_2O$), 4.7-4.95(CONH).

Example 5

3-[N-Acetyl-N-(2-pyridyl)methyl]carbamoyl-2-methyl-1-octadecylcarbamoylglycerol

In 7 ml of chloroform was dissolved 252 mg of 3-O-[N-(2-pyridyl)methyl]carbamoyl-2-O-methyl-1-O-octadecylcarbamoylglycerol as obtained in Example 3-i), and 5.5 ml of triethylamine and 1 ml of acetic anhydride were added to the solution, followed by heating under reflux for 3 days. After cooling, 3 % sodium hydrogencarbonate was added to the reaction solution, and the mixture was extracted with chloroform. The organic layer was dried over magnesium sulfate and the solvent was distilled off under reduced pressure. The resulting residue was purified by column chromatography [silica gel, 15g; eluent, n-hexane-ethyl acetate;1:2] to give 89 mg of the objective compound.

TLC: Rf = 0.33 [silica gel, n-hexane-ethyl acetate, 1:3]

IR (KBr) $cm^{-1}$: 3370, 2923, 2850, 1740, 1700, 1600, 1535, 1475, 1370, 1232, 1090, 1065, 985, 775

NMR (90 MHz, $CDCl_3$) δ : 0.88(3H,t,Me), 1.28(32H,s,$CH_2$), 2.60 (3H,s,NAc), 3.12(2H,q,CONHC$\underline{H_2}$), 3.29-(3H,s,OMe), 3.46(1H,quint,C$\underline{H}$-O), 3.98(2H,d,$CH_2$O CO), 4.21(2H,d,$CH_2$OCO), 4.92(1H,broad t N$\underline{H}$), 5.09-(2H,s,C$\underline{H_2}$-pyridyl), 7.11, 7.60, 8.48(4H,pyridine).

Example 6

3-O-[N-Acetyl-N-(N-methylpyridinio-2-yl)methyl]carbamoyl-2-O-methyl-1-O-octadecylcarbamoylglycerol chloride

To 85 mg of the compound as obtained in Example 5 was added 0.5 ml of methyl iodide, and the mixture was refluxed for 2 days, under shielding from light. After cooling, the reaction solution was concentrated under reduced pressure, and the residue was treated with IRA-410 [$Cl^-$] (5 ml, eluent: MeOH-$H_2O$, 7:3). The resulting crude chloride derivative was purified by column chromatography [silica gel, 5g; eluent, chloroform-methanol, 6:1 to 4:1] to give 52 mg of the objective compound.

TLC: Rf = 0.17 (silica gel, $CHCl_3$:MeOH, 4:1)

IR (film) $cm^{-1}$: 3340, 2920, 2850, 1740, 1700, 1630, 1465, 1370, 1210.

NMR (90 MHz, $CDCl_3$) $\delta$ : 0.88(3H,t,Me), 1.24(32H,s,$CH_2$), 2.61(3H,s,NAc), 3.10(2H,q,CONH $CH_2$), 3.38-(3H,s,OMe), 3.70(1H,m,CHO), 3.99(4H,m,$CH_2$OCOx2), 4.35(1H,m,NH), 4.70(3H,s,NMe), 5.43(2H,br,s,$CH_2$-pyridinio), 7.71, 8.02, 8.43, 9.65(4H,m,pyridinio).

Example 7

1-Methyl-2-[N-acetyl-N'-(3-octadecylcarbamoyloxy-2-methoxypropyl)ureido]methylpyridinium chloride

i) 2-[N'-(3-octadecylcarbamoyloxy-2-methoxypropyl)ureido]methylpyridine

In 5 ml of toluene was dissolved 200 mg (0.5 mmole) of the amino derivative as obtained in Example 4, and 181 µl (1.5 mmole) of diphosgene was added to the solution, followed by stirring at room temperature for 10 minutes under a nitrogen atmosphere and further at 79°C for 5 hours. After cooling, the reaction solution was concentrated under reduced pressure to give a crude isocyanate derivative.

In 2 ml of chloroform was dissolved the isocyanate derivative, and 61 µl (0.6 mmole) of 2-(aminomethyl)pyridine was added to the solution under ice-cooling. The reaction solution was stirred at room temperature for 17.5 hours and concentrated under reduced pressure. The resulting crude product was purified by column chromatography [silica gel: 20 g; eluent: chloroform-methanol = 20:1] to give 206 mg (77.0%) of the objective compound as a colorless solid.

TLC[silica gel: $CHCl_3$/MeOH(20/1)]: Rf = 0.27

NMR[90MHz, $CDCl_3$] $\delta$: 0.86(3H, t), 1.25(32H, s), 3.12(2H, q), 3.37(3H, s), 3.30-3.55(3H, m), 4.12(2H, m), 4.47(2H, d), 5.25(1H, br), 5.58(1H, br, t), 6.04(1H, br, t), 7.18(2H, m), 7.62(1H, d, t), 8.47(1H, br, d)

IR[KBr]$cm^{-1}$: 3330, 2920, 2855, 1695, 1635, 1590, 1539, 1478, 1442, 1290, 1280, 1262, 1251, 1235, 1139, 1105, 1055.

ii) 2-[N-Acetyl-N'-(3-octadecylcarbamoyloxy-2-methoxypropyl)ureido]methylpyridine

In 5 ml of chloroform was dissolved 200 mg (0.374 mmole) of the compound as obtained in i), and 3.8 ml of triethylamine and 0.7 ml of acetic anhydride were added to the solution, followed by refluxing for 2 days under a nitrogen atmosphere. After cooling, the reaction solution was treated with 3% aqueous $NaHCO_3$ solution and extracted with chloroform.

Example 8

1-Ethyl-2-[N-acetyl-N-(3-octadecylcarbamoyloxy-2-methoxypropyloxy)carbonyl]aminomethylpyridinium chloride

To 140 mg (0.243 mmole) of the compound as produced in Example 5 was added 3 ml of iodoethane, and the reaction solution was refluxed for 3 days under a nitrogen atmosphere. After cooling, the reaction solution was concentrated to dryness and the residue was treated with IRA-410 ($Cl^-$ type) [15 ml; eluent: 70% methanol/water] to give 192 mg of a crude chloride derivative. This crude chloride derivative was dissolved in acetone and cooled with ice, and the precipitate which was separated out was collected by filtration to give 120 mg (76.9%) of the objective compound as a colorless powder.

TLC[silica gel; $CHCl_3$/MeOH(3/1)] Rf = 0.32

NMR[90MHz, $CDCl_3$] $\delta$: 0.88(3H, t), 1.25(32H, s), 1.71(3H, t), 2.65(3H, s), 3.12(2H, q), 3.38(3H, s), 3.66-(1H, quint), 4.02(2H, br, d), 4.37(2H, m), 5.20(2H, q), 5.31(1H, br), 5.48(2H, br, s), 7.75(1H, br, d), 8.06(1H, br, t), 8.47(1H, br, t), 10.00(1H, br, d)

IR[KBr]$cm^{-1}$: 3405, 2930, 2850, 1754, 1700, 1638, 1224.

Example 9

1-Ethoxycarbonylmethyl-2-[N-acetyl-N-(3-octadecylcarbamoyloxy-2-methoxypropyloxy)carbonyl]-aminomethylpyridinium chloride

To 173 mg (0.3 mmole) of the compound as produced in Example 5 was added 1 ml of ethyl chloroacetate, and the reaction solution was heated at 50 °C for 2 hours and further at 90 °C for 24 hours. After cooling, the reaction solution was concentrated under reduced pressure, and the resulting crude product was purified by column chromatography [silica gel: 10 g; eluent: chloroform/methanol = 4/1] to give 62 mg (29.5%) of the objective compound as a pale brown powder.

TLC[silica gel; $CHCl_3$/MeOH(3/1)] Rf = 0.30

NMR[90MHz, $CDCl_3$] δ: 0.86(3H, t), 1.24(32H, s), 1.37(3H, m), 2.59(3H, s), 3.13(2H, q), 3.37(3H, s), 3.70-(1H, m), 3.89-4.56(6H, m), 5.31(3H, br), 6.32(2H, br), 7.86, 8.10, 8.55, 9.90(each 1H, m)

IR[KBr]$cm^{-1}$: 3380, 2925, 2850, 1750, 1700, 1627, 1525, 1465, 1420, 1375, 1348, 1220.

Example 10

2-[N-(3-Octadecylcarbamoyloxy-2-ethoxypropyloxycarbonyl)amino]methyl-N-methylpyridinium chloride

i) 2-O-Ethyl-3-O-[N-(pyridin-2-yl)methyl]carbamoyl-1-O-octadecylcarbamoylglycerol

To the carbonate derivative synthesized from 830 mg (2 mmole) of 2-O-ethyl-1-O-octadecylcarbamoyl-glycerol (m.p. 55-56 °C), 344 mg(2.2 mmole) of phenyl chloroformate, 320 mg of pyridine and 10 ml of methylene chloride were added 260 mg of 2-(aminomethyl)pyridine and 5 ml of chloroform, and the reaction solution was refluxed for 12 hours and concentrated to dryness. The resulting product was purified by column chromatography [silica gel: 40 g; eluent: n-hexane-ethyl acetate (1:3)] to give 727 mg (66%) of the objective compound.

IR[KBr]$cm^{-1}$: 3325, 2925, 2850, 1697, 1540, 1470, 1270

NMR[60MHz, $CDCl_3$] δ: 0.87(3H), 1.08(3H, t), 1.27(32H, s), 3.16(2H, q), 3.3-4.0(3H, m), 4.18(4H, m), 4.50(2H, d), 4.80 (1H, br), 5.90(1H, br), 7.20(2H, m), 7.65(1H, m), 8.50(1H, m).

ii) 2-O-Ethyl-3-O-[N-acetyl-N-(pyridin-2-yl)methyl]carbamoyl-1-O-octadecylcarbamoylglycerol

In 5 ml of pyridine was dissolved 285 mg of the carbamoyl derivative as obtained in i), and 2 ml of acetic anhydride was added to the solution, followed by heating at 100 °C for 23 hours. The reaction solution was concentrated to dryness and the residue was purified by silica gel chromatography (eluent: n-hexane-ethyl acetate (1:1)] to give 228 mg (75%) of the objective compound.

IR[KBr]$cm^{-1}$: 3350, 2930, 2855, 1742, 1705, 1698, 1598, 1532, 1370, 1115, 1080, 980, 778, 760

NMR[90MHz, $CDCl_3$] δ: 0.87(3H, t), 1.07(3H, t), 1.23(32H, s), 2.60(3H, s, Ac), 3.11(2H, q), 3.3-3.7(3H, m), 3.97(2H, d), 4.21(2H, m), 4.83(1H, br, NH), 5.08(2H, s, $C\underline{H}_2$Py), 7.10(2H), 7.60(1H), 8.48(1H).

iii) 2-[N(3-Octadecylcarbamoyloxy-2-ethoxypropyloxycarbonyl)amino]methyl-N-methylpyridinium chloride

To 222 mg of the compound as obtained in ii) was added 1.5 ml of methyl iodide, and the solution was refluxed for 2 days. After cooling, the reaction solution was concentrated under reduced pressure and the residue was treated with IRA-410 (Cl type) [15 ml; eluent: MeOH-$H_2O$ (7:3)]. The resulting chloride derivative was purified by column chromatography (silica gel: 15 g; eluent: chloroform-methanol (4:1)] to give 125 mg of the objective compound.

NMR[60MHz, $CDCl_3$] δ: 0.87(3H, t), 1.12(3H), 1.25(32H, s), 2.64(3H, s, Ac), 3.14(2H), 3.60(2H, q), 3.6-4.6(6H), 4.72 (3H, s, $\overset{+}{N}$Me), 5.45(2H, br-s, $C\underline{H}_2$-Py), 7.6-8.6(3H, m), 9.60(1H).

Example 11

2-[N-(3-Octadecylcarbamoyloxy-2-ethoxypropyloxycarbonyl)amino]methyl-N-ethylpyridinium iodide

In 0.5 ml of ethyl iodide was dissolved 130 mg of the compound as obtained in Example 10-iii), and the solution was refluxed for 27 hours, and purified by silica gel chromatography [eluent: chloroform to chloroform-methanol (20:1)] to give 103 mg of the objective compound.

IR(KBr)$cm^{-1}$: 3420, 2925, 2850, 1738, 1700, 1628, 1530, 1465, 1370, 1220, 1160, 985, 778

NMR[90MHz, $CDCl_3$) δ: 0.87(3H, t), 1.15(3H, t), 1.25(32H, s), 1.73(3H, t), 2.66(3H, s, N-Ac), 3.11(2H, q), 3.61(2H, q), 3.85(1H, m), 4.02(2H), 4.39(2H), 4.93(1H, br, NH), 5.08(2H, q, $\overset{+}{N}$-$CH_2$), 5.47(2H, s, $\underline{CH_2}$-Py), 7.84(1H), 8.05(1H), 8.41(1H), 9.64(1H).

Example 12

3-Octadecylcarbamoyl-2-methyl-1-(pyridin-3-yl)carbamoylglycerol

In 10 ml of toluene were dissolved 0.615 g (5 mmole) of nicotinic acid, 1.515 g (5.05 mmole) of diphenylphosphorylazide and 0.6 g of triethylamine, and the reaction solution was stirred at room temperature for 2 hours and refluxed for 1 hour. The reaction solution was concentrated to about a half volume, and 1.9 g (4.74 mmole) of 3-octadecylcarbamoyl-2-methylglycerol was added to the resulting solution. The mixture was heated at 100°C overnight and concentrated to dryness under reduced pressure. The residue was purified by silica gel (25 g) with chloroform as an eluent to give 1.74 g (yield 70.4%) of colorless crystals.

TLC[silica gel, $CHCl_3$-MeOH(19:1)] Rf = 0.27 single spot

NMR[60MC, $CDCl_3$] δ: 0.97(3H), 1.25(32H), 3.17(2H), 3.47(3H), 3.67(1H), 4.23(2H), 4.33(2H), 7.23(1H), 7.95(1H).

Example 13

3-[(3-Octadecylcarbamoyloxy-2-methoxypropoxy)carbonylamino]-1-methylpyridinium iodide

In 2 ml of methyl iodide and 2 ml of dichloromethane was dissolved 300 mg (0.57 mmole) of the pyridine derivative as obtained in Example 12, and the solution was allowed to stand at room temperature for 2 days.

The reaction solution was concentrated to dryness under reduced pressure, and the residue was washed with n-hexane to give 375 mg (yield: 100%) of a colorless powder.

IR(KBr)$cm^{-1}$: 3310, 2920, 2850, 1730, 1695, 1550, 1510, 1460, 1270, 1240, 1160, 1060

NMR(60MC, $CDCl_3$) δ: 0.87(3H), 1.25(32H), 3.17(2H), 3.45(3H), 3.70(1H), 4.20(4H), 4.50(3H), 5.13(1H), 7.90(1H), 8.70(1H), 8.87(1H), 9.38(1H), 9.93(1H).

Example 14

3-Octadecylcarbamoyl-2-methyl-1-(N-acetyl-N-pyridin-3-yl)carbamoylglycerol

In a mixture of 3 ml of pyridine and 1.5 ml of acetic anhydride was dissolved 320 mg (0.613 mmole) of the pyridine derivative as obtained in Example 12, and the solution was refluxed for 3 hours. The reaction solution was concentrated to dryness under reduced pressure, and the residue was purified by silica gel (5 g) with chloroform as an eluent to give 203 mg (yield 58.7%) of a colorless solid.

TLC[silica gel; $CHCl_3$-MeOH(19:1)] Rf = 0.52 single spot

NMR(60MC, $CDCl_3$) δ: 0.92(3H), 1.23(32H), 2.65(3H), 3.07(2H), 3.25(3H), 3.40(1H), 3.90(2H), 4.20(2H), 4.90(1H), 7.43(2H), 8.40(1H), 8.57(1H)

Example 15

3-[N-(3-Octadecylcarbamoyloxy-2-methoxypropoxycarbonyl)-N-acetyl]amino-1-methylpyridinium iodide

In a mixture of 1 ml of dichloromethane and 2 ml of methyl iodide was dissolved 200 mg (0.35 mmole) of the acetate as obtained in Example 14, and the solution was allowed to stand at room temperature for 2 days, followed by concentration to dryness under reduced pressure. The residue was washed with n-hexane to give 246 mg (yield 100%) of a colorless powder.

TLC[silica gel; $CHCl_3$, MeOH, $H_2O$(65.25:4)] Rf = 0.50 single spot

IR(KBr)$cm^{-1}$: 3400, 2920, 2850, 1760, 1720, 1500, 1470, 1370, 1250, 1100, 1045, 915

NMR(60MC, $CDCl_3$) δ: 0.92(3H), 1.25(32H), 2.72(3H), 3.05(2H), 3.37(3H), 3.60(1H), 3.89(2H), 4.28(2H), 4.63(3H), 5.00(1H), 8.0-8.5(2H), 9.17-9.47(2H).

Example 16

3-Octadecylcarbamoyl-2-methyl-1-(pyridin-2-yl)carbamoylglycerol

α-Picolinic acid (1.23 g: 10 mmole), diphenylphosphorylazide (3.03 g: 11 mmole), triethylamine (1.2 g) and toluene (20 ml) were treated in the same manner as described in Example 12 to give 2.7 g (yield 83%) of colorless crystals.

TLC[silica gel; $CHCl_3$, MeOH(19:1)] Rf = 0.51 single spot

NMR(60MC, $CDCl_3$) δ: 0.92(3H), 1.25(32H), 3.13(2H), 3.47(3H), 3.70(1H), 4.20(2H), 4.30(2H), 4.73(1H), 6.90(1H), 7.65(1H), 7.90(1H), 8.33(1H), 8.83(1H)

Example 17

1-(3-Trityloxy-2-methoxypropyl)uracil

In 20 ml of dimethylformamide was suspended 2.24 g (20 mmole) of uracil and 5.03 g (10 mmole) of 3-trityl-2-methylglycerol in the presence of 4.24 g of sodium carbonate, and the suspension was stirred at 105 ° C for 16 hours. The reaction solution was concentrated to dryness under reduced pressure, and 100 ml of water, and 100 ml of chloroform were added to the residue. After the aqueous layer was adjusted to pH 7.0 with acetic acid and shaken vigorously, the organic layer was separated,dried over sodium sulfate and concentrated to dryness under reduced pressure. The residue was purified by silica gel (30 g) [eluent: chloroform-ethyl acetate-n-hexane (4:1:3)] to give 2.67 g (yield 60.5%) of a colorless powder.

TLC[silica gel; $CHCl_3$, MeOH(19:1)] Rf = 0.44

Example 18

1-(3-Trityloxy-2-methoxypropyl)-3-(pyridin-2-yl)methyluracil

In 6 ml of dimethylsulfoxide were dissolved 1.2 g (2.72 mmole) of the uracil derivative as obtained in Example 17 and 1.04 g (8.15 mmole) of 2-chloromethylpyridine hydrochloride in the presence of 1.12 g of powdered potassium hydroxide, and the reaction solution was stirred at 50 ° C for 1 hour and poured into 60 ml of water. The mixture was adjusted to pH 7.0 and extracted with 60 ml of ether. The ether layer was dried over sodium sulfate and concentrated to dryness under reduced pressure. The residue was purified by silica gel (10 g) [eluent: ethyl acetate-n-hexane (2:1)] to give 1.4 g (yield 96.5%) of a colorless solid.

TLC[silica gel; n-hexane-EtOAc(1:2)] Rf = 0.14

NMR(60MC, $CDCl_3$) δ: 3.22(1H), 3.28(3H), 3.60(2H), 4.05(2H), 5.23(2H), 7.0-8.0(18H), 8.27(1H)

Example 19

1-(3-Hydroxy-2-methoxypropyl)-3-(pyridin-2-yl)methyluracil

In a mixture of 40 ml of methanol, 5 ml of water and 3 ml of conc. hydrochloric acid was dissolved 1.4 g (2.62 mmole) of the trityl derivative as obtained in Example 18, and the solution was stirred at room temperature for 1 hour. After the solution was cooled to 4 ° C and the crystals separated were removed by filtration, the filtrate was neutralized with 1 N sodium hydroxide and concentrated to dryness under reduced pressure. The residue was dissolved in a mixture of 19 ml of chloroform and 1 ml of methanol. The insoluble material was removed and the solution was concentrated to dryness under reduced pressure. The residue was purified by silica gel (10 g) [eluent: chloroform, methanol (19:1)] to give 729 mg (yield 100%) of a colorless solid material.

TLC[silica gel; $CHCl_3$,MeOH (19:1)] Rf = 0.25

NMR(60MC, $CDCl_3$) δ: 2.85(1H), 3.88(3H), 3.60(3H), 3.93(2H), 5.27(2H), 5.73(1H), 7.23(3H), 7.60(1H), 8.30(1H)

Example 20

1-(3-Octadecylcarbamoyloxy-2-methoxy)propyl-3-(pyridin-2-yl)methyluracil

In 1 ml of pyridine were dissolved 729 mg (2.65 mmole) of the hydroxy derivative as obtained in

Example 19 and 0.78 g (2.65 mmole) of octadecyl isocyanate, the reaction solution was heated at 105°C for 16 hours, followed by concentration to dryness under reduced pressure. The residue was purified by silica gel (15 g) [eluent: chloroform, methanol (49:1)] to give 1.2 g (yield 77.2%) of a colorless powder.

TLC[silica gel; $CHCl_3$, MeOH(19:1)] Rf = 0.42

NMR(60MC, $CDCl_3$) δ: 0.87(3H), 1.23(32H), 3.38(3H), 3.63(1H), 4.11(2H), 4.15(2H), 5.08(1H), 5.27(2H), 5.72(1H), 7.17(2H), 7.23(1H), 7.58(1H), 8.45(1H)

Example 21

2-[1-(3-Octadecylcarbamoyloxy-2-methoxypropyl)uracil-3-yl]methyl-1-methylpyridinium iodide

In a mixture of 0.5 ml of dichloromethane and 338.6 mg (2.386 mmole) of methyl iodide was dissolved 700 mg (1.19 mmole) of the pyridine derivative as obtained in Example 20, and the reaction solution was allowed to stand at room temperature overnight, followed by concentration to dryness under reduced pressure. The residue was recrystallized from a mixture of 1.5 ml of dichloromethane and 15 ml of n-hexane to give 880 mg (yield 100%) of a colorless powder.

TLC[silica gel; $CHCl_3$ MeOH, $H_2O$ (65:25:4)] Rf = 0.33

IR(film)$cm^{-1}$: 3300, 2940, 2850, 1710, 1660, 1515, 1455, 1400, 1370, 1250

NMR(60MC, $CDCl_3$) δ: 0.90(3H), 1.25(32H), 6.48(2H), 3.40(3H), 3.70(1H) , 4.15(4H), 4.72(3H), 5.15(1H), 5.25(2H), 5.80(1H), 7.50(1H), 7.97(2H), 8.43(1H), 9.35(1H)

| Elemental Analysis, for $C_{34}H_{57}N_4O_5I \cdot 2H_2O$ (764.79) | | | |
|---|---|---|---|
| Calcd.: | C, 53.40; | H, 8.04; | N, 7.33 |
| Found : | C, 53.40; | H, 7.89; | N, 7.43 |

Example 22

2-(3-Octadecylcarbamoyloxy-2-methoxypropoxycarbonyl)aminomethylthiazole

In 2 ml of toluene were dissolved 2.33 g (4.47 mmole) of 3-octadecylcarbamoyl-2-methyl-1-phenoxycarbonylglycerol synthesized as indicated below, 0.77 g (6.13 mmole) of 2-aminomethylthiazole and 2 ml of triethylamine, and the reaction solution was allowed to stand at room temperature for 1 day and concentrated to dryness under reduced pressure. The residue was purified by silica gel (25 g) [eluent: n-hexane, ethyl acetate (1:1)] to give 1.35 g (yield 55.7%) of a pale yellow powder.

TLC[silica gel; n-hexane, EtOAc(1:1)] Rf = 0.37 single spot

NMR(60MC, $CDCl_3$) δ: 0.90(3H) 1.25(32H), 3.13(2H), 3.47(3H), 3.62(1H), 4.17(2H), 4.67(2H), 4.80(1H), 5.63(1H), 7.27(1H), 7.67(1H)

3-O-(2'-Dimethylaminoethyl)carbamoyl-2-O-methyl-1-O-octadecylcarbamoylglycerol

A 445 mg (4.8 mmole) portion of asym.-dimethylethylenediamine was added to the carbonate obtained from 1.607 g (4 mmole) of 2-O-methyl-1-O-octadecylcarbamoylglycerol, 632 mg (8 mmole) of pyridine, 689 mg (4.4 mmole) of phenyl chlorocarbonate and dichloromethane (10 ml) by the same procedure as described in Examples 1 and 5, and the mixture was heated at 70°C for 5 hours. After cooling, the crude product was purified by silica gel column chromatography with chloroform-methanol (10:1) used as an eluent to give 1.895 g (yield of 91.9 %) of the objective compound (colorless solid).

TLC [silica gel, chloroform-methanol (5:1)] Rf = 0.31

NMR [90 MHz, $CDCl_3$] δ : 0.88(3H), 1.27(32H), 2.20(6H), 2.39(2H), 3.02-3.34(4H), 3.43(3H), 3.58(1H), 4.16(4H), 5.00(1H), 5.45(1H).

IR [KBr] $cm^{-1}$: 3300, 2925, 2850, 1695, 1535, 1470, 1280, 1260, 1115, 1075

Example 23

2-[N-Acetyl-N-(3-octadecylcarbamoyloxy-2-methoxypropoxycarbonyl)aminomethyl]thiazole

In 0.5 ml of toluene were dissolved 108 mg (0.2 mmole) of the thiazole derivative as obtained in

Example 22, 122 mg (1.0 mmole) of dimethylaminopyridine and 102 mg (1.0 mmole) of acetic anhydride, and the reaction solution was heated at 80 °C for 4 hours and concentrated to dryness under reduced pressure. The residue was purified by silica gel (3 g) [eluent: chloroform, methanol (39:1)] and further silica gel (3 g) [eluent: n-hexane, ethyl acetate (1:1)] to give 110 mg (yield 78.1%) of a colorless solid.

TLC[silica gel; n-hexane, EtOAc(1:2)] Rf = 0.43 single spot

IR(film)$cm^{-1}$: 3350, 2930, 2850, 1745, 1715, 1525, 1470, 1430, 1375, 1345, 1210, 1145, 1080, 920

NMR(60MC, $CDCl_3$) δ: 0.92(3H), 1.23(32H), 2.6(3H), 3.08(2H), 3.37(3H), 3.57(1H), 4.10(2H), 4.27(2H), 4.90(1H), 5.27(2H), 7.23(1H), 7.67(1H)

## Example 24

2-[N-(3-Octadecylcarbamoyloxy-2-methoxypropoxycarbonyl)amino]methyl-3-methylthiazolium iodide

In 1.5 ml of methyl iodide was dissolved 100 mg (0.185 mmole) of the thiazole derivative as obtained in Example 22 and the reaction solution was heated at 70 °C for 8 hours and concentrated to dryness under reduced pressure to give 124 mg (yield 100%) of a pale yellow solid.

TLC[silica gel; $CHCl_3$, MeOH, $H_2O$(65:25:4)] Rf = 0.35 single spot

IR(film)$cm^{-1}$: 3340, 2930, 2850, 1700, 1520, 1470, 1250, 1140, 1050

NMR(60MC, $CDCl_3$) δ: 0.88(3H), 1.25(32H), 3.15(2H), 3.45(3H), 3.65(1H), 4.13(2H), 4.23(2H), 5.08(3H), 8.27(1H), 8.45(1H)

## Example 25

2-[N-(3-Octadecylcarbamoyloxy-2-methoxypropoxycarbonyl)amino]methyl-3-ethylthiazolium iodide

In 1.0 ml of ethyl iodide was dissolved 100 mg (0.185 mmole) of the thiazole derivative as obtained in Example 22, and the reaction solution was heated at 80 °C overnight and concentrated to dryness under reduced pressure. The residue was purified by silica gel (2 g) [eluent: $CHCl_3$ → $CHCl_3$, MeOH (19:1)] to give 91 mg (yield 70.5%) of a pale yellow solid.

TLC[silica gel; $CHCl_3$, MeOH, $H_2O$(65:25:4)] Rf = 0.66 single spot

IR(film)$cm^{-1}$: 3320, 2920, 2850, 1720, 1520, 1465, 1240, 1140, 1050, 910

NMR(60MC, $CDCl_3$) δ: 0.88(3H), 1.27(32H), 1.67(3H), 3.13(2H), 3.45(3H), 3.63(1H), 4.13(2H), 4.25(2H), 4.73(2H), 4.98(1H), 5.10(2H), 7.33(1H), 8.25(1H), 8.45(1H)

## Example 26

2-[N-Acetyl-N-(3-octadecylcarbamoyloxy-2-methoxypropoxycarbonyl)amino]methyl-3-methylthiazolium iodide

In 1.5 ml of methyl iodide was dissolved 100 mg (0.185 mmole) of the acetate derivative as obtained in Example 23, and the reaction solution was heated at 50 °C for 16 hours and concentrated to dryness under reduced pressure. The residue was purified by silica gel (2 g) [eluent: $CHCl_3$, MeOH, $H_2O$ (65:25:4)] to give 116 mg (yield 100%) of a pale yellow powder.

TLC[silica gel: $CHCl_3$, MeOH, $H_2O$(65:25:4)] Rf = 0.25 single spot

IR(film)$cm^{-1}$: 3400, 2930, 2850, 1700, 1560, 1530, 1470, 1375, 1330, 1250, 1200, 1140, 1075

NMR(60MC, $CDCl_3$) δ: 0.92(3H), 1.25(32H), 2.62(3H), 3.08(2H), 3.48(3H), 3.80(1H), 4.17(2H), 4.45(2H), 4.52(3H), 5.33(1H), 5.59(2H), 8.43(1H), 8.58(1H)

## Example 27

2-[N-Acetyl-N-(3-octadecylcarbamoyloxy-2-methoxypropoxycarbonyl)amino]methyl-3-ethylthiazolium iodide

In 1.0 ml of ethyl iodide was dissolved 117 mg (0.2 mmole) of the acetate derivative as obtained in Example 23, and the reaction solution was heated at 90 °C for 5 hours and concentrated to dryness under reduced pressure. The residue was purified by silica gel (2 g) [eluent: $CHCl_3$ → $CHCl_3$, MeOH (19:1)] to give 116 mg (yield 50.7%) of a pale yellow powder.

TLC[silica gel; $CHCl_3$, MeOH, $H_2O$(65:25:4)] Rf = 0.71 single spot

IR(film)$cm^{-1}$: 3300, 2920, 2850, 1750, 1700, 1525, 1465, 1370, 1340, 1240, 1205, 1140, 1105, 1070, 990

NMR(60MC, $CDCl_3$) δ: 0.83(3H), 1.23(32H), 1.69(3H), 2.62(3H), 3.13(2H), 3.47(3H), 3.80(1H), 4.17(2H), 4.46(2H), 4.92(2H), 5.10(1H), 5.63(2H), 8.43(1H), 8.67(1H)

Example 28

2-[N-Acetyl-N-(3-octadecylcarbamoyloxy-2-methoxypropoxycarbonyl)amino]methyl-3-propylthiazolium iodide

In 1.0 ml of propyl iodide was dissolved 117 mg (0.2 mmole) of the acetate derivative as obtained in Example 23, and the reaction solution was heated at 90°C for 5 hours and concentrated to dryness under reduced pressure. The residue was purified by silica gel (2 g) [eluent: $CHCl_3$ → $CHCl_3$, MeOH(19:1)] to give 116 mg (yield 50.7%) of a pale yellow powder.

TLC[silica gel; $CHCl_3$, MeOH, $H_2O$(65:25:4)] Rf = 0.73 single spot

IR(film)$cm^{-1}$: 3350, 2920, 2850, 1750, 1700, 1535, 1470, 1375, 1340, 1240, 1205, 1150, 1100, 1070, 990

NMR(60MC, $CDCl_3$) δ: 0.88(3H), 1.08(3H), 1.23(32H), 2.16(2H), 2.62(3H), 3.15(2H), 3.45(3H), 3.83(1H), 5.62(2H), 8.43(1H), 8.67(1H)

Example 29

2-[N-Acetyl-N-(3-octadecylcarbamoyloxy-2-methoxypropoxycarbonyl)amino]methyl-3-butylthiazolium iodide

In 1.0 ml of butyl iodide was dissolved 117 mg (0.2 mmole) of the acetate derivative as obtained in Example 23, and the reaction solution was heated at 90°C for 5 hours and concentrated to dryness under reduced pressure. The residue was purified by silica gel (2 g) [eluent: $CHCl_3$ → $CHCl_3$, MeOH(19:1)] and further silica gel (2 g) [eluent: acetone, ethyl acetate (1:1)] to give 47 mg (yield 30.5%) of a pale brown solid.

TLC[silica gel; $CHCl_3$, MeOH, $H_2O$(65:25:4)] Rf = 0.74 single spot

IR(film)$cm^{-1}$: 3340, 2925, 2855, 1755, 1700, 1535, 1470, 1430, 1375, 1340, 1240, 1205, 1150, 1110, 1070, 1040, 985

NMR(60MC, $CDCl_3$) δ: 0.88(3H), 1.00(3H), 1.25(32H), 2.00(4H), 2.65(3H), 3.10(2H), 3.45(3H), 3.78(1H), 5.12(1H), 5.58(2H), 8.42(1H), 8.62(1H)

Example 30

4-(3-Octadecylcarbamoyloxy-2-methoxypropoxycarbonyl)aminomethylthiazole

In 5 ml of dichloromethane were dissolved 2.60 g (5.0 mmole) of 3-octadecylcarbamoyl-2-methyl-1-phenoxycarbonylglycerol synthesized in the same manner as described in Example 22, 0.57 g (5.00 mmole) of 4-aminomethylthiazole and 1 ml of triethylamine, and the reaction solution was heated at 50°C overnight and concentrated to dryness under reduced pressure. The residue was purified by silica gel (25 g) [eluent: n-hexane, ethyl acetate (1:1)] to give 440 mg (yield 16.0%) of a pale yellow powder.

TLC[silica gel; n-hexane, EtOAc(1:1)] Rf = 0.51 single spot

Example 31

4-[N-Acetyl-N-(3-octadecylcarbamoyloxy-2-methoxypropoxycarbonyl)amino]methylthiazole

In 1.0 ml of toluene were dissolved 162 mg (0.3 mmole) of the thiazole derivative as obtained in Example 30, 244 mg (2.0 mmole) of dimethylaminopyridine and 204 mg (2.0 mmole) of acetic anhydride, and the reaction solution was heated at 110°C overnight and concentrated to dryness under reduced pressure. The residue was purified by silica gel (2 g) [eluent: chloroform] and further silica gel (2 g) [eluent: n-hexane, ethyl acetate (1:1)] to give 84 mg (yield 48.0%) of a colorless solid.

TLC[silica gel; n-hexane, EtOAc(1:2)] Rf = 0.63 single spot

IR(film)$cm^{-1}$: 3350, 2920, 2850, 1740, 1705, 1535, 1465, 1435, 1370, 1350, 1200, 1140, 1080, 980, 950

NMR(60MC, $CDCl_3$) δ: 0.88(3H), 1.25(32H), 2.57(3H), 3.18(2H), 3.38(3H), 3.67(1H), 4.12(2H), 4.24(2H), 4.92(1H), 5.14(2H), 7.17(1H), 8.73(1H)

Example 32

4-[N-Acetyl-N-(3-octadecylcarbamoyloxy-2-methoxypropoxycarbonyl)amino]methyl-3-methylthiazolium iodide

In 0.5 ml of methyl iodide was dissolved 4.5 mg of the acetate as obtained in Example 31, and the reaction solution was heated at 60° C for 16 hours and concentrated to dryness under reduced pressure. The residue was purified by silica gel (0.5 g) [eluent: $CHCl_3$, MeOH (65:25)] to give 1.85 mg of a pale yellow powder.

TLC[silica gel; $CHCl_3$, MeOH, $H_2O$(65:25:4)] Rf = 0.69 single spot

Example 33

4-[N-Acetyl-N-(3-octadecylcarbamoyloxy-2-methoxypropoxycarbonyl)amino]methyl-3-ethylthiazolium iodide

In 1.0 ml of ethyl iodide was dissolved 79.5 mg (0.136 mmole) of the acetate derivative as obtained in Example 31, and the reaction solution was heated at 90° C overnight and concentrated under reduced pressure. The residue was purified by silica gel (1.7 g) [eluent: $CHCl_3$ → $CHCl_3$, MeOH (19:1)] to give 44 mg (yield 43.7%) of a pale yellow solid.

TLC[silica gel; $CHCl_3$, MeOH, $H_2O$(65:25:4)] Rf = 0.72 single spot

IR(film)$cm^{-1}$: 3350, 2925, 2855, 1750, 1705, 1525, 1465, 1370, 1335, 1240, 1205, 1140, 1105, 1070, 1040, 995, 940

NMR(60MC, $CDCl_3$) δ: 0.87(3H), 1.23(32H), 1.72(3H), 2.60(3H), 3.08(2H), 3.45(3H), 3.79(1H), 4.14(2H), 4.42(2H), 4.87(2H), 5.07(1H), 5.20(2H), 8.25(1H), 10.80(1H)

Example 34

4-Methyl-5-(3-octadecylcarbamoyloxy-2-methoxypropoxycarbonylaminoethylthiazole

In 1 ml of toluene were dissolved 439 mg (1.26 mmole) of 3-octadecylcarbamoyl-2-methyl-1-phenoxycarbonylglycerol synthesized in the same manner as described in Example 22, 179.5 mg (1.26 mmole) of 4-methyl-5-aminoethylthiazole and 1 ml of triethylamine, and the reaction solution was heated at 50° C for 4 hours and concentrated to dryness under reduced pressure. The residue was purified by silica gel (5 g) [eluent: n-hexane, ethyl acetate (1:1)] to give 345 mg (yield 73.6%) of a pale yellow powder.

TLC[silica gel; n-hexane, EtOAc(1:1)] Rf = 0.21 single spot

NMR(60MC, $CDCl_3$) δ: 0.80(3H), 1.25(32H), 2.46(3H), 2.52(3H), 3.07(2H), 3.13(2H), 3.47(3H), 3.63(1H), 4.16(2H), 4.23(1H), 4.97(1H), 8.50(1H)

Example 35

3,4-Dimethyl-5-[2-(3-octadecylcarbamoyloxy-2-methoxypropoxycarbonyl)amino]ethylthiazolium iodide

In 1.5 ml of methyl iodide was dissolved 100 mg (0.18 mmole) of the thiazole derivative as obtained in Example 34, and the reaction solution was heated at 70° C for 8 hours and concentrated to dryness under reduced pressure to give 128 mg (yield 100%) of a pale yellow solid.

TLC[silica gel; $CHCl_3$, MeOH, $H_2O$(65:25:4)] Rf = 0.39 single spot

NMR(60MC, $CDCl_3$) δ: 0.87(3H), 1.27(32H), 2.52(3H), 3.18(4H), 3.45(3H), 3.58(3H), 4.17(4H), 4.27(3H), 5.06(1H), 6.27(1H), 10.45(1H)

Example 36

4-Methyl-5-[N-acetyl-N-(3-octadecylcarbamoyloxy-2-methoxypropoxycarbonyl)amino]ethylthiazole

In 1 ml of toluene were dissolved 190 mg (0.341 mmole) of the thiazole derivative as obtained in Example 34, 244 mg (2.0 mmole) of dimethylaminopyridine and 204 mg (2.0 mmole) of acetic anhydride, and the reaction solution was heated at 100° C for 16 hours and concentrated to dryness under reduced pressure. The residue was purified by silica gel (2 g) [eluent: chloroform, methanol (39:1)] and further silica gel (2 g) [eluent: n-hexane, ethyl acetate (1:1)] to give 20 mg (yield 9.8%) of a colorless oil.

TLC[silica gel; n-hexane, EtOAc(1:1)] Rf = 0.21 single spot

NMR(60MC, $CDCl_3$) δ: 0.80(3H), 1.25(32H), 2.46(3H), 2.52(3H), 3.07(2H), 3.13(2H), 3.47(3H), 3.63(1H), 4.16(2H), 4.23(1H), 4.97(1H), 8.50(1H)

Example 37

3,4-Dimethyl-5-[N-acetyl-N-(3-octadecylcarbamoyloxy-2-methoxypropoxycarbonyl)amino]ethylthiazolium iodide

In 1 ml of methyl iodide was dissolved 20 mg (0.033 mmole) of the acetate derivative as obtained in Example 36, and the reaction solution was allowed to stand at room temperature for 16 hours and concentrated to dryness under reduced pressure to give 25 mg (yield 100%) of the objective compound.

TLC[silica gel; $CHCl_3$, MeOH, $H_2O$(65:25:4)] Rf = 0.42 single spot

IR(KBr)$cm^{-1}$: 3330, 2920, 2850, 1730, 1700, 1520, 1470, 1370, 1325, 1270, 1250, 1200, 1150, 1100, 1060, 1040, 975, 920

NMR(60MC, $CDCl_3$) δ: 0.87(3H), 1.23(32H), 2.52(3H), 2.59(3H), 3.72(1H), 3.97(2H), 4.22(4H), 4.40(3H), 5.10(1H), 10.88(1H)

Example 38

1-Octadecylcarbamoyl-2-methyl-3-(1-ethylpiperidin-3-yl)carbamoylglycerol

3-Amino-1-ethylpiperidine (1 ml) was added to 1.3 g of 1-octadecylcarbamoyl-2-methyl-3-phenoxycarbonylglycerol, and the reaction solution was heated at 60 °C for 1 hour and purified by silica gel chromatography [ethyl acetatemethanol (10:1)] to give 1.13 g of the objective compound as a milk-white solid.

NMR(90MHz, $CDCl_3$) δ: 0.87(3H, t), 1.02(3H, t), 1.25(32H, m), 1.57(4H, m), 2.34(6H, m), 3.14(2H, m), 3.43(3H, s), 3.57(1H, m), 3.77(1H, m), 4.16(4H, m), 4.73(1H, br), 5.25(1H, br)

TLC:Rf = 0.16 [AcOEt-MeOH(10:1)]

Example 39

3-(3-Octadecylcarbamoyloxy-2-methoxypropoxycarbonyl)amino-1-ethyl-1-methylpiperidinium iodide

In 10 ml of ether was dissolved 200 mg of the N-ethyl derivative as obtained in Example 38, and 0.5 ml of methyl iodide was added to the solution. The reaction solution was stirred at room temperature for 2 days and concentrated to dryness to give 250 mg of the objective compound as a pale brown powder.

IR(KBr, $cm^{-1}$) 3460, 3330, 2925, 2852, 1702, 1530, 1472, 1260, 1140, 1060, 782, 730

NMR(90MHz, $CDCl_3$) δ: 0.87(3H, t), 1.25(32H, m), 1.45(3H, t), 2.10(4H, m), 3.13(2H, m), 4.18, 4.38(3H, s), 3.43(3H, s), 3.5-4.1(8H, m), 4.14(4H, m), 4.91(1H, br), 5.8(1H, br)

TLC:Rf = 0.25 [$CHCl_3$-MeOH = 5:1]

Example 40

1-Octadecylcarbamoyl-2-methyl-3-[N-acetyl-N-(1-ethylpiperidin-3-yl)]carbamoylglycerol

In a mixture of 5 ml of pyridine and 2 ml of acetic anhydride was dissolved 600 mg of the compound as obtained in Example 38, and the reaction solution was refluxed for 18 hours and concentrated to dryness. The residue was purified by silica gel chromatography to give 125 mg of the objective compound as a pale brown solid.

IR(KBr, $cm^{-1}$) 3350, 2930, 2855, 1740, 1710, 1530, 1460, 1378, 1230, 1220, 1145, 780

NMR(90MHz, $CDCl_3$) δ: 0.87(3H, t), 1.03(3H, t), 1.25(32H, m), 1.5-2.1(4H, m), 2.25-3.0(6H, m), 2.40(3H, s), 3.14(2H, m), 3.45(3H, s), 3.64(1H, m), 4.25(4H, m), 4.60(1H, m), 5.10(1H, br)

TLC:Rf = 0.19 [AcOEt-MeOH (10:1)]

Example 41

3-[N-Acetyl-N-(3-octadecylcarbamoyloxy-2-methoxypropoxycarbonyl]amino-1-ethyl-1-methylpiperidinium io-

dide

In 5 ml of ether was dissolved 118 mg of the compound as obtained in Example 40, and 0.2 ml of methyl iodide was added to the solution. The reaction solution was stirred at room temperature for 3 days and concentrated to dryness to give 146 mg of the objective compound as a pale brown powder.

IR(KBr, $cm^{-1}$) 3420, 2925, 2852, 1738, 1700, 1535, 1470, 1372, 1325, 1240, 1180

Example 42

3-[N-Acetyl-N-(3-octadecylcarbamoyloxy-2-methoxypropoxycarbonyl)]amino-1-ethyl-1-methylpiperidinium chloride

The iodide derivative (145 mg) as obtained in Example 41 was subjected to exchange of an ion by Amberlite IRA-410 ion exchange resin ($Cl^-$ type) to give 120 mg of the chloride derivative as a pale brown solid.

IR(KBr, $cm^{-1}$) 3400, 2925, 2850, 1735, 1698, 1538, 1470, 1372, 1325, 1240, 1178, 780, 725

NMR(90MHz, $CDCl_3$) δ: 0.87(3H, t), 1.25(32H, m), 1.70(3H, t), 2.02(4H, m), 2.44(3H, s), 3.12(2H, m), 3.3-3.65(4H, m), 3.45(3H, s), 3.65-4.1(4H, m), 4.19(2H, d), 4.40(2H, m), 4.9(1H, br), 5.15(1H, br)

TLC Rf = 0.2 [$CHCl_3$-MeOH (5:1)]

Example 43

3-[N-Acetyl-N-(N'-ethylpyridinio-2-yl)methyl]carbamoyl-1-hexadecylcarbamoyl-2-methylglycerol chloride

1) 2-Methyl-3-(2'-pyridylmethyl)carbamoyl-1-hexadecylcarbamoylglycerol

In 4 ml of methylene chloride were dissolved 567 mg (1.52 mmole) of 1-hexadecylcarbamoyl-2-methylglycerol and 240 mg (3.04 mmole) of pyridine, and 261 mg (1.67 mmole) of phenyl chloroformate was added dropwise to the solution under ice-cooling, followed by stirring at room temperature for 1 hour. Aqueous sodium hydrogencarbonate (5%) solution was added to the reaction solution, followed by extraction with chloroform. The organic layer was dried over sodium sulfate and the solvent was distilled off under reduced pressure.

To the obtained crude carbonate was added 0.185 ml (1.82 mmole) of 2-(aminomethyl)pyridine, and the mixture was heated at 90 °C for 3 hours. After cooling, the crude product was purified by column chromatography [silica gel: 20 g; eluent: n-hexane/ethyl acetate = 1/4] to give 633 mg (82.1%) of the objective compound as a colorless solid.

TLC(silica gel: n-hexane/AcOEt = 1/4): Rf = 0.25

NMR(90MHz, $CDCl_3$) δ: 0.87(3H, t), 1.24(28H, s), 3.15(2H, q), 3.44(3H, s), 3.60(1H, quint), 4.18(4H, m), 4.50(2H, d), 4.85(1H, br), 5.95(1H, br), 7.23(2H, m), 7.67(1H, d, t), 8.53(1H, d, d)

IR(KBr)$cm^{-1}$: 3325, 2922, 2850, 1692, 1596, 1539, 1466, 1265, 1250

2) 3-[N-Acetyl-N-(2'-pyridylmethyl)]carbamoyl-2-methyl-1-hexadecylcarbamoylglycerol

In 12.5 ml of pyridine was dissolved 633 mg (1.25 mmole) of the compound as synthesized in 1), and 2.35 ml (24.94 mmole) of acetic anhydride was added to the solution, followed by heating at 110 °C for 60 hours under a nitrogen atmosphere. The reaction solution was concentrated to dryness and 5% aqueous sodium hydrogencarbonate solution was added to the residue. Extraction with chloroform was conducted and the organic layer was dried over sodium sulfate. The solvent was distilled off under reduced pressure, and the obtained crude product was purified by column chromatography (silica gel: 27 g; eluent : n-hexane/ethyl acetate = 1/2) to give 415 mg (60.5%) of the objective compound as a pale yellow solid.

TLC(silica gel; n-hexane/AcOEt): Rf = 0.47

NMR(90MHz, $CDCl_3$) δ: 0.87(3H, t), 1.25(28H, s), 2.62(3H, s), 3.14(2H, q), 3.30(3H, s), 3.47(1H, quint), 4.00(2H, d), 4.24(2H, d.d.), 4.83(1H, br), 5.09(2H, s), 7.17(2H, m), 7.62(1H, d, t), 8.49(1H, d,d)

IR(KBr)$cm^{-1}$: 3352, 2920, 2848, 1738, 1694, 1590, 1532, 1365, 1226

3) 3-[N-Acetyl-N-(N'-ethylpyridinio-2-yl)methyl]carbamoyl-1-hexadecylcarbamoyl-2-methylglycerol chloride

To 385 mg (0.70 mmole) of the compound as synthesized in 2) was added 3 ml of ethyl iodide, and the

reaction solution was refluxed for 2 days under a nitrogen atmosphere under shielding from light. After cooling, the reaction solution was concentrated to dryness and the obtained iodide was treated with IRA-410($Cl^-$) [30 ml; eluent: 70% methanol/water] to give 430 mg (100%) of the objective compound as a pale yellow powder.

TLC(silica gel; $CHCl_3$/MeOH = 3/1): Rf = 0.12

NMR(90MHz, $CDCl_3$) $\delta$: 0.87(3H, t), 1.24(28H, s), 1.71(3H, t), 2.64(3H, s), 3.10(2H, q), 3.37(3H, s), 3.66-(1H, quint), 4.00(2H, d), 4.37(2H, m), 5.22(2H, q and 1H, br), 5.47(2H, s), 7.75(1H, br, d), 8.05(1H, br, t), 8.49(1H, br, t), 10.07(1H, br, d)

IR(KBr)$cm^{-1}$: 3425, 2925, 2851, 1750, 1700, 1629, 1532, 1466, 1371, 1220

## Example 44

3-[N-Acetyl-N-(N'-ethylpyridinio-2-yl)methyl]carbamoyl-2-methyl-1-tetradecylcarbamoylglycerol chloride

1) 2-Methyl-3-(2'-pyridylmethyl)carbamoyl-1-tetradecylcarbamoylglycerol

In 4 ml of methylene chloride were dissolved 518 mg (1.50 mmole) of 1-tetradecylcarbamoyl-2-methylglycerol and 237 mg (3.00 mmole) of pyridine, and 258 mg (1.65 mmole) of phenyl chloroformate was added dropwise to the solution under ice-cooling, followed by stirring at room temperature for 30 minutes. Aqueous sodium hydrogencarbonate (5%) solution was added to the reaction solution, followed by extraction with chloroform. The organic layer was dried over sodium sulfate and the solvent was distilled off under reduced pressure.

To the obtained crude carbonate was added 0.183 ml (1.80 mmole) of 2-(aminomethyl)pyridine, and the mixture was heated at 90°C for 3 hours. After cooling, the crude product was purified by column chromatography (silica gel: 20 g; eluent: n-hexane/ethyl acetate = 1/4) to give 591 mg (82.1%) of the objective compound as a colorless solid.

TLC(silica gel; n-hexane/AcOEt = 1/4): Rf = 0.20

NMR(90MHz, $CDCl_3$) $\delta$: 0.87(3H, t), 1.24(24H, s), 3.15(2H, q), 3.44(3H, s), 3.59(1H, quint), 4.18(4H, m), 4.50(2H, d), 4.82(1H, br), 5.92(1H, br), 7.22(2H, m), 7.66(1H, d, t), 8.53(1H, d, d)

IR(KBr)$cm^{-1}$: 3320, 2924, 2850, 1690, 1592, 1542, 1465, 1270

2) 3-[N-Acetyl-N-(2'-pyridylmethyl)]carbamoyl-2-methyl-1-tetradecylcarbamoylglycerol

In 11.7 ml of pyridine was dissolved 562 mg (1.17 mmole) of the compound as synthesized in 1), and 2.21 ml (23.43 mmole) of acetic anhydride was added to the solution, followed by heating at 110°C for 60 hours under a nitrogen atmosphere. The reaction solution was concentrated to dryness and 5% aqueous sodium hydrogencarbonate solution was added to the residue, followed by extraction with chloroform. The organic layer was dried over sodium sulfate and the solvent was distilled off under reduced pressure. The obtained crude product was purified by column chromatography (silica gel: 25 g; eluent: n-hexane/ethyl acetate = 1/2) to give 376 mg (61.5%) of the objective compound as a pale yellow solid.

TLC(silica gel; n-hexane/AcOEt = 1/3) Rf = 0.46

NMR(90MHz, $CDCl_3$) $\delta$: 0.87(3H, t), 1.24(24H, s), 2.60(3H, s), 3.13(2H, q), 3.28(3H, s), 3.47(1H, quint), 4.00(2H, d), 4.23(2H, d, d), 4.88(1H, br), 5.08(2H, s), 7.17(2H, m), 7.61(1H, d, t), 8.48(1H, d, d)

IR(KBr)$cm^{-1}$: 3352, 2920, 2850, 1738, 1694, 1590, 1532, 1365, 1225

3) 3-[N-Acetyl-N-(N'-ethylpyridinio-2-yl)methyl]carbamoyl-2-methyl-1-tetradecylcarbamoylglycerol chloride

To 365 mg (0.70 mmole) of the compound as synthesized in 2) was added 3 ml of ethyl iodide, and the reaction solution was heated under reflux for 2 days under a nitrogen atmosphere under shielding from light. After cooling, the reaction solution was concentrated to dryness and the obtained iodide was treated with IRA-410($Cl^-$) [30 ml; eluent: 70% methanol/water] to give 402 mg (98.0%) of the objective compound as a pale yellow powder.

TLC(silica gel; $CHCl_3$/MeOH = 3/1): Rf = 0.13

NMR(90MHz, $CDCl_3$) $\delta$: 0.87(3H, t), 1.25(24H, s), 1.71(3H, t), 2.65(3H, s), 3.11(2H, q), 3.37(3H, s), 3.66-(1H, quint), 4.01(2H, d), 4.38(2H, m), 5.20(2H, q and 1H, br), 5.48(2H, s), 7.77(1H, br, d), 8.06(1H, br, t), 8.49 (1H, br, t), 10.00(1H, b,r, d)

IR(KBr)$cm^{-1}$: 3390, 2920, 2850, 1750, 1700, 1625, 1524, 1450, 1370, 1215

Example 45

4-[N-Acetyl-N-(3-octadecylcarbamoyloxy-2-methoxypropoxycarbonyl)amino]methyl-3-ethylthiazolium chloride

i) 4-(3-Octadecylcarbamoyloxy-2-methoxypropoxycarbonylaminomethylthiazole

A reaction mixture of 10.96 g (21 mmole) of 3-octadecylcarbamoyl-2-methyl-1-phenoxycarbonylglycerol as synthesized in the same manner as described in Example 22 and 2.1 g (21 mmole) of 4-aminomethylthiazole was heated at 50 °C overnight, and purified by silica gel (100 g) [eluent: n-hexane, ethyl acetate (1:1)] to give 7.5 g (yield 65.9%) of a pale yellow powder.

The IR spectrum of this product was the same as that of the product as obtained in Example 30.

ii) 4-[N-Acetyl-N-(3-octadecylcarbamoyloxy-2-methoxypropoxycarbonyl)aminomethyl]thiazole

In 18 ml of toluene were dissolved 6.3 g (11.3 mmole) of the thiazole derivative as obtained in i), 5.6 g (46.5 mmole) of dimethylaminopyridine and 5.0 g (49 mmole) of acetic anhydride, and the reaction solution was heated at 70 °C for 4 days and concentrated to dryness under reduced pressure. The residue was purified by silica gel (60 g) [eluent: chloroform] and further by silica gel (60 g) [eluent: n-hexane-ethyl acetate (1:1)] to give 3.0 g (yield 45.5%) of a colorless solid.

The IR spectrum of this product was the same as that of the product as obtained in Example 31.

iii) 4-[N-Acetyl-N-(3-octadecylcarbamoyloxy-2-methoxypropoxycarbonyl)amino]methyl-3-ethylthiazolium iodide

In 20 ml of ethyl iodide was dissolved 3.0 g (5.1 mmole) of the acetate as obtained in ii), and the reaction solution was heated at 120 °C overnight in a sealed vessel and concentrated to dryness under reduced pressure to give 3.78 g (yield 100%) of a pale yellow solid.

The IR spectrum of this product was the same as that of the product as obtained in Example 32.

iv) 4-[N-Acetyl-N-(3-octadecylcarbamoyloxy-2-methoxycarbonyl)amino]methyl-3-ethylthiazolium chloride

In 150 ml of 75% methanol was dissolved 3.3 g (4.46 mmole) of the iodide as obtained in iii), and the solution was passed through a column of IRA-410 ($Cl^-$) [150 ml] and washed with a small amount of 75% methanol. The effluent and washing were combined and concentrated to dryness under reduced pressure. The residue was recrystallized from 4 ml of acetone, 4 ml of ether and 40 ml of n-hexane to give 2.8 g (96.9%) of colorless crystals.

TLC [silica gel; $CHCl_3$, MeOH, $H_2O$ (65:25:4)] Rf = 0.72 single spot

IR [film]$cm^{-1}$: 3350, 2925, 2855, 1750, 1705, 1525, 1465, 1370, 1335, 1240, 1205, 1140, 1105, 1070, 1040, 995, 940.

NMR [60 MC, $CDCl_3$] δ: 0.87(3H), 1.23(32H), 1.72(3H), 2.60(3H), 3.08(2H), 3.45(3H), 3.79(1H), 4.14(2H), 4.42(2H), 4.87(2H), 5.07(1H), 5.20(2H), 8.25(1H), 10.80(1H)

| Elemental Analysis for $C_{32}H_{58}N_3O_6SCl \cdot H_2O$ | | | | |
|---|---|---|---|---|
| Calcd.: | C,57.68; | H,9.08; | N,6.31; | S,4.81 |
| Found : | C,57.89; | H,9.07; | N,6.41; | S,4.90 |

Example 46

4-[N-Acetyl-N-(3-octadecylcarbamoyloxy-2-methoxypropoxycarbonyl)amino]methyl-3-ethylthiazolium acetate

In 15 ml of 75% methanol was dissolved 330 mg (0.446 mmole) of the iodide as obtained in Example 45, and the solution was passed through a column of IRA-410 ($AcO^-$) [15 ml], and washed with a small amount of 75% methanol. The effluent and washing were combined and concentrated to dryness under reduced pressure. The residue was recrystallized from 1 ml of ether and 15 ml of n-hexane to give 290 mg (97.0%) of colorless crystals.

TLC [silica gel; $CHCl_3$, MeOH, $H_2O$ (65:25:4)] Rf = 0.72 single spot

IR [film]$cm^{-1}$: 3350, 2925, 2855, 1750, 1705, 1600, 1525, 1465, 1370, 1335, 1240, 1205, 1140, 1105, 1070, 1040, 995, 940

NMR [60MC, $CDCl_3$] δ: 0.87(3H), 1.23(32H), 1.72(3H), 2.02(3H), 2.60(3H), 3.08(2H), 3.45(3H), 3.79(1H), 4.15(2H), 4.42(2H), 4.88(2H), 5.07(1H), 5.21(2H), 8.25(1H), 10.80(1H)

Example 47

3-[N-Acetyl-N-(N'-propylpyridinio-2-yl)methyl]carbamoyl-1-octadecylcarbamoyl-2-methylglycerol chloride

To 173 mg (0.30 mmole) of the compound as synthesized in Example 5 was added 1 ml of n-propyl iodide, and the reaction solution was refluxed for 36 hours under a nitrogen atmosphere under shielding from light. After cooling, the reaction solution was concentrated to dryness and the resulting iodide was purified by column chromatography [silica gel: 10 g; eluent: chloroform/methanol = 8/1] and treated with IRA-410 ($Cl^-$) [5 ml; eluent: 70% methanol/water] to give 118 mg (59.9%) of the objective compound as a pale yellow powder.

TLC [silica gel; $CHCl_3$/MeOH = 3/1]: Rf = 0.22

NMR [90MHz, $CDCl_3$] δ: 0.86(3H,t), 1.10(3H,t), 1.23(32H,s), 2.05(2H,m), 2.65(3H,s), 3.10(2H,q), 3.37-(3H,s), 3.65(1H,quint), 4.01(2H,d), 4.37(2H,br,t), 5.08(2H,t), 5.33(1H,br), 5.43(2H,s), 7.76(1H,br,d), 8.05-(1H,br,t), 8.47(1H,br,t), 10.00(1H,br,d)

IR(KBr)$cm^{-1}$: 3425, 2924, 2852, 1750, 1700, 1628, 1532, 1468, 1370, 1220

Example 48

3-[N-Acetyl-N-(N'-allylpyridinio-2-yl)methyl]carbamoyl-1-octadecylcarbamoyl-2-methylglycerol chloride

To 173 mg (0.30 mmole) of the compound as synthesized in Example 5 was added 1 ml of allyl iodide, and the reaction solution was refluxed for 16 hours under a nitrogen atmosphere under shielding from light. After cooling, the reaction solution was concentrated to dryness, and the residue was treated with IRA-410 ($Cl^-$) [15 ml; eluent: 70% methanol/water]. The resulting chloride was purified by column chromatography [silica gel: 10 g; eluent: chloroform/methanol = 8/1 to 3/1) to give 43 mg (21.9%) of the objective compound as a pale yellow powder.

TLC [silica gel; $CHCl_3$/MeOH = 3/1]: Rf = 0.25

NMR (90MHz, $CDCl_3$) δ: 0.88(3H,t), 1.25(32H,s), 2.64(3H,s), 3.13(2H,q), 3.39(3H,s), 3.68(1H,quint), 4.03-(2H,br,d), 4.37(2H,m), 5.14-5.63(5H,m), 5.74-6.39(3H,m), 7.77(1H,br,d), 8.06(1H,br,t), 8.50(1H,br,t), 9.96-(1H,br,d)

IR(KBr)$cm^{-1}$: 3400(br), 2920, 2850, 1750, 1700, 1624, 1530, 1465, 1220

Example 49

2-(3-Octadecylcarbamoyloxypropyloxycarbonyl)aminomethylpyridine

To 20 ml of dichloromethane were added 1.50 g of 3-(octadecylcarbamoyloxy)propanol (mp 80 ° C) and 0.76 g of phenyl chlorocarbonate, and 0.4 g of pyridine was added to the solution with stirring under ice-cooling. The reaction solution was stirred under ice-cooling for 1 hour and further stirred at room temperature overnight. As aqueous sodium hydrogencarbonate solution was added to the solution, followed by stirring. The organic layer was separated, dried and concentrated. To the residue were added 518 mg of 2-aminomethylpyridine and 10 ml of chloroform, and the resulting mixture was refluxed for 15 hours and concentrated. The residue was purified by silica gel (100 g) chromatography [eluent: n-hexane-ethyl acetate (1:3)] to give 1.45 g of the objective compound.

TLC[silica gel; n-hexane-ethyl acetate (1:2)] Rf = 0.25

IR(KBr) $cm^{-1}$: 3300, 2905, 2840, 1680, 1530

Example 50

2-[N-(3-Octadecylcarbamoyloxypropoxycarbonyl)aminomethyl]-1-ethylpyridinium iodide

In 5 ml of ethyl iodide was dissolved the pyridine derivative as obtained in Example 49, and the reaction

solution was heated at 90°C overnight in a sealed vessel and concentrated to dryness under reduced pressure. The residue was purified by silica gel (10 g) chromatography [eluent: chloroform-methanol (19:1)] to give 510 mg of the objective compound.

IR(KBr) $cm^{-1}$: 3320, 2925, 2850, 1690, 1635, 1535

For reference sake, structrual formulae of the compounds as synthesized in Examples are shown as follows:

Example 1

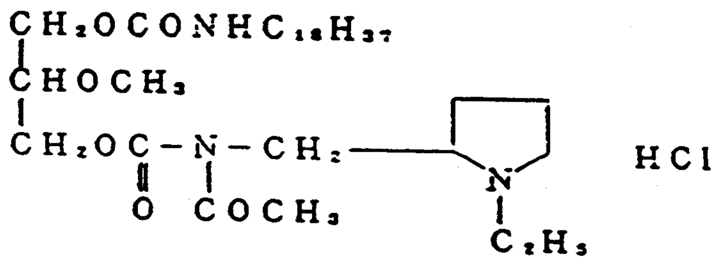

Example 2

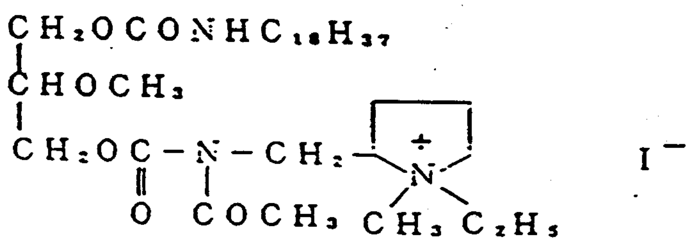

Example 3

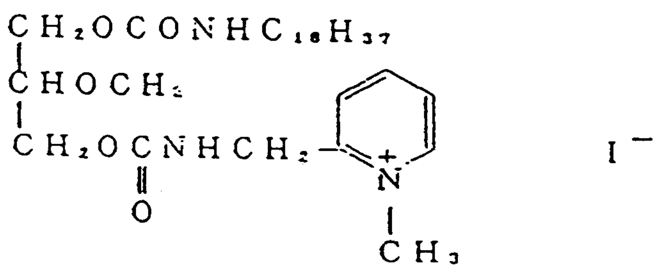

Example 4

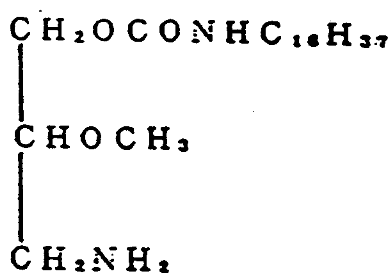

Example 5

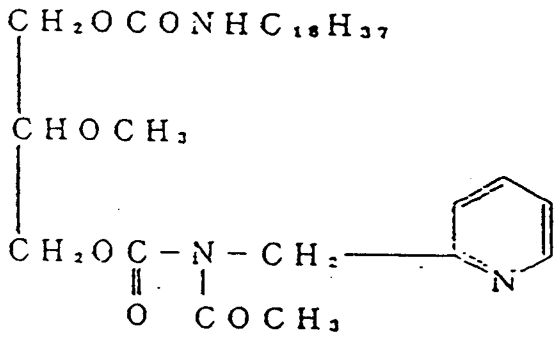

Example 6

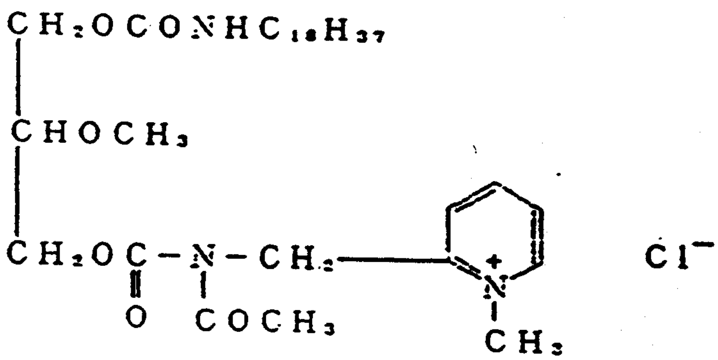

Example 7

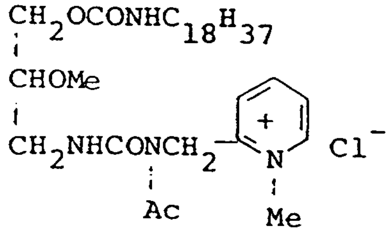

Example 8

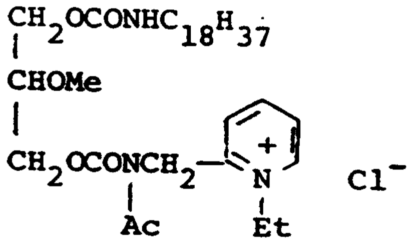

Example 9

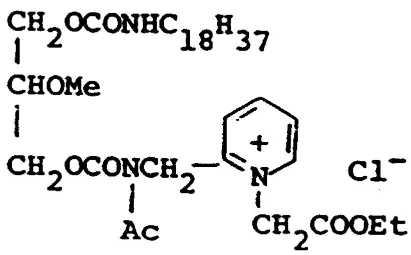

Example 10

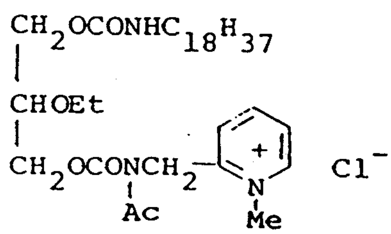

Example 11

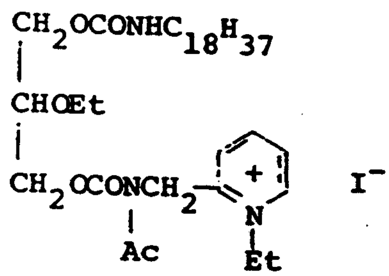

Example 12

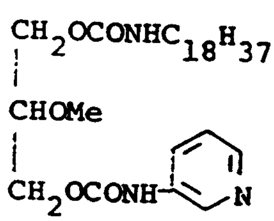

Example 13

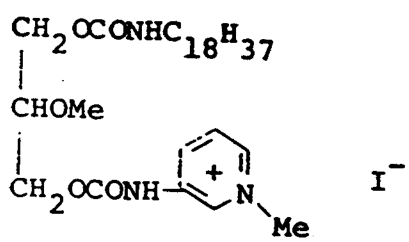

Example 14

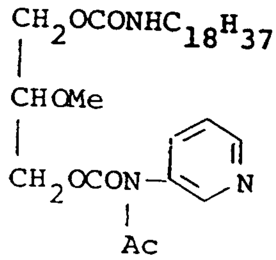

Example 15

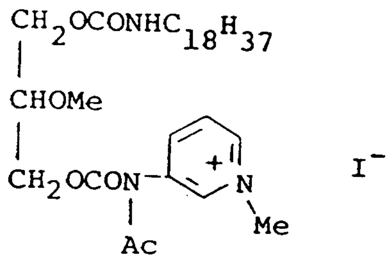

Example 16

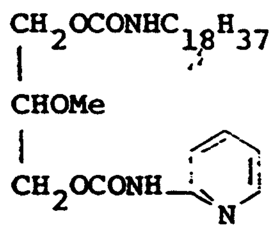

Example 17

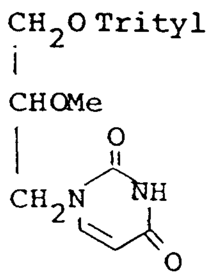

Example 18

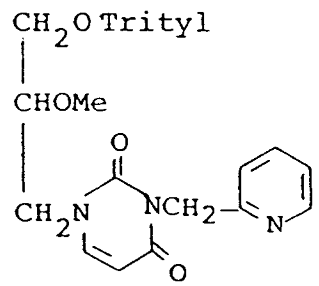

Example 19

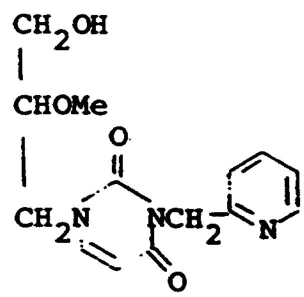

Example 20

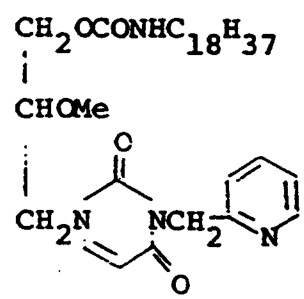

Example 21

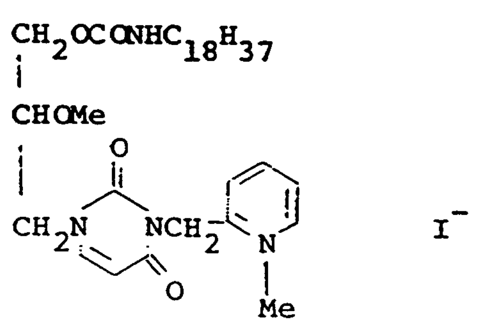

Example 22

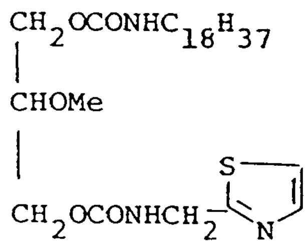

Example 23

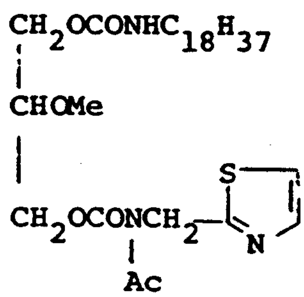

Example 24

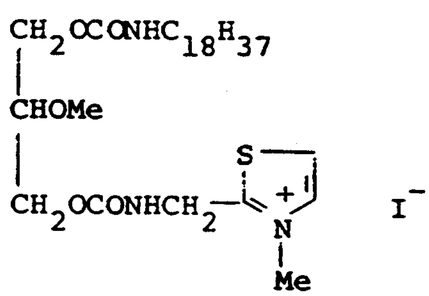

Example 25

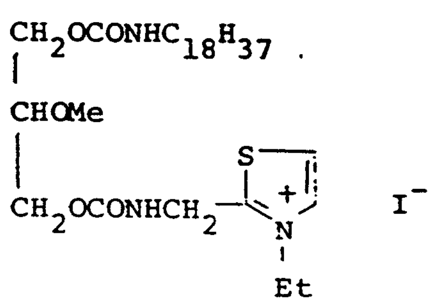

Example 26

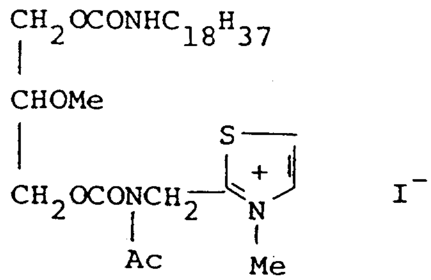

Example 27

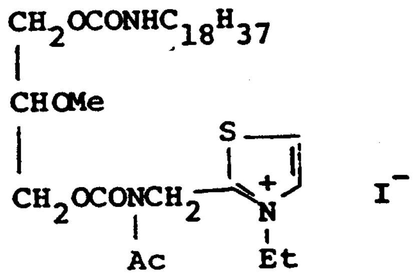

Example 28

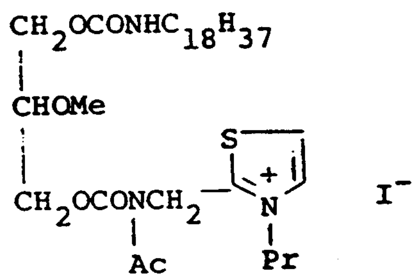

Example 29

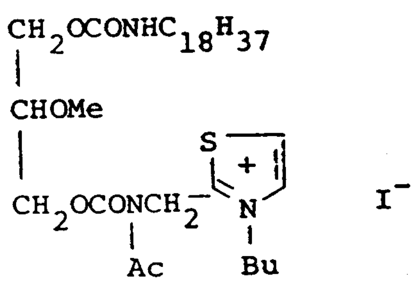

Example 30

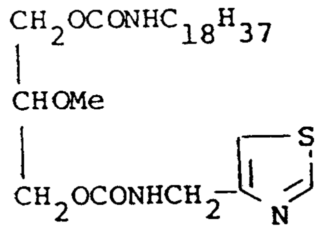

Example 31

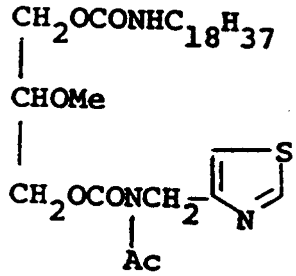

Example 32

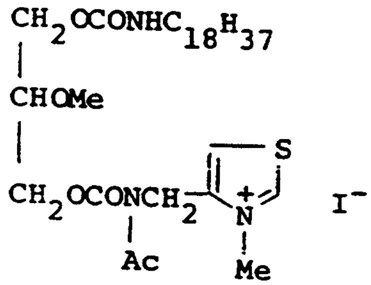

Example 33

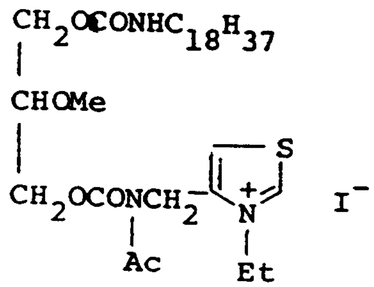

Example 34

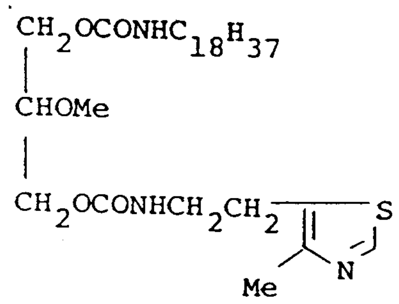

Example 35

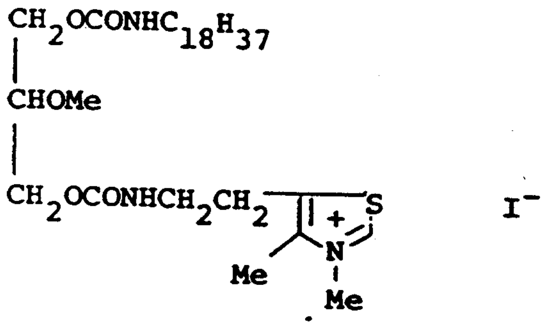

Example 36

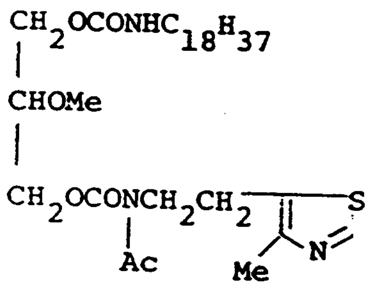

Example 37

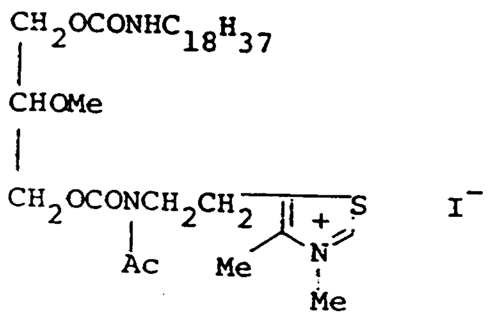

Example 38

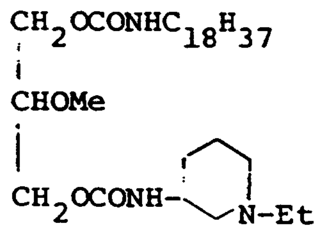

Example 39

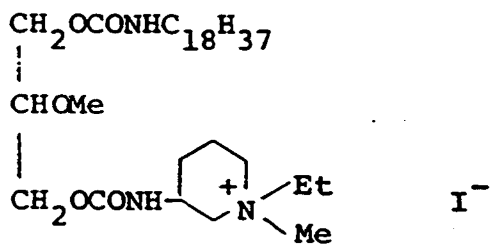

Example 40

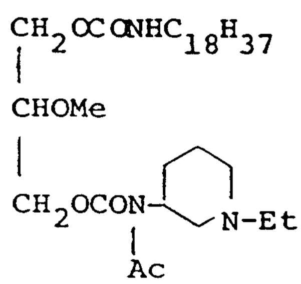

Example 41

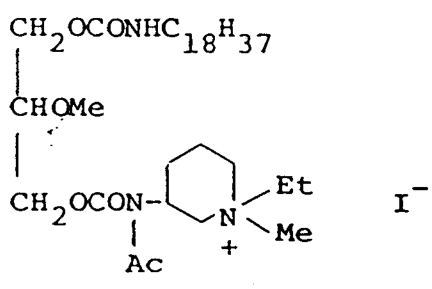

Example 42

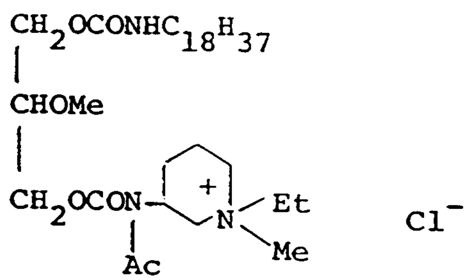

Example 43

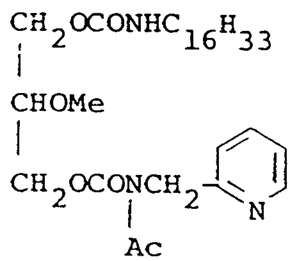

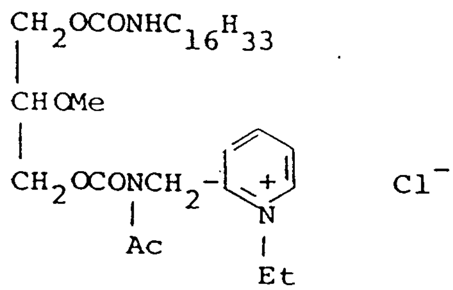

Example 44

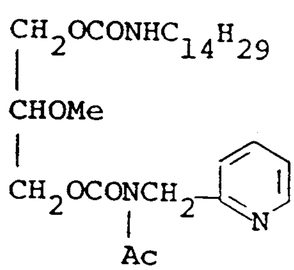

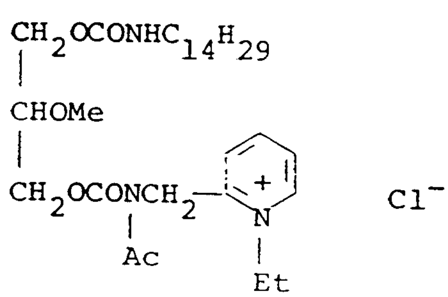

Example 45

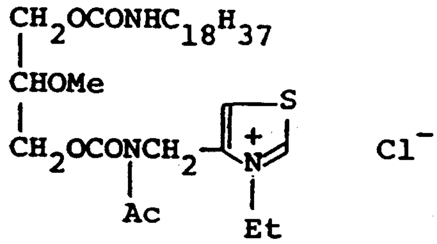

Example 46

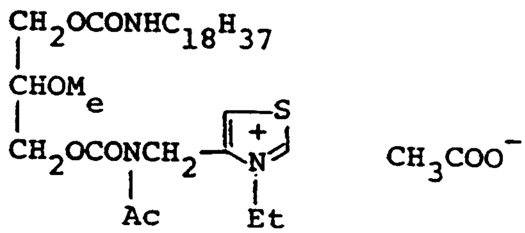

Example 47

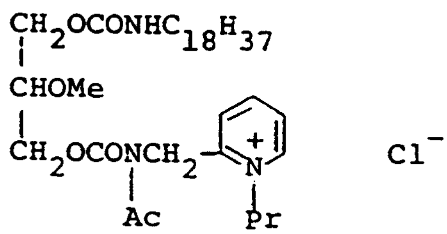

Example 48

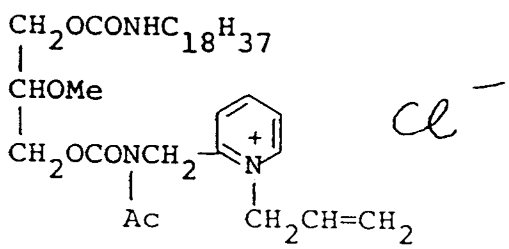

Example 49

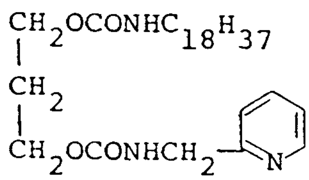

Example 50

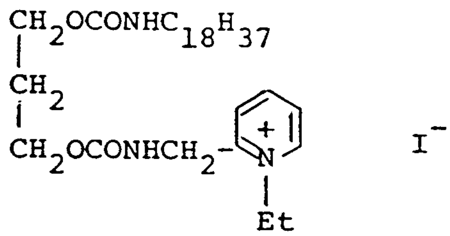

In the above-mentioned formulae, Ac is acetyl; Bu is butyl; Et is ethyl; Me is methyl; Ph is phenyl; Pr is propyl; Trityl is trityl; and Ts is tosyl.

Experimental Example 1

Inhibitory activity on platelet aggregation

[Method]

With use of an injection syringe containing 3.15% citric acid (at a ratio of 1 part against 9 parts of blood) as an anticoagulant, blood was collected by cardiac puncture from a male rabbit. The blood was then centrifuged at room temperature at 800 r.p.m. for 10 minutes to prepare platelet rich plasma (PRP). The remaining blood was further centrifuged at 3000 r.p.m. for 10 minutes to separate platelet poor plasma (PPP) as a supernatant liquid. The PRP was diluted with the PPP to adjust the number of platelets to about 5 x $10^5$ per µl. This PRP (250 µl) was stirred at 37°C for 2 minutes, and a test drug was added. After the mixture was stirred for 2 minutes, 1 x $10^{-8}$M of PAF was added to the mixture. Platelet aggregation was determined with an aggregometer (manufactured by Rika Denki K.K.). The inhibitory activity on platelet aggregation of the test drug was determined from the inhibition rate in relation to the maximum transmission (maximum aggregation ratio) of control PRP by PAF.

The test compounds were investigated for the inhibitory activity against platelet aggregation by PAF, The results are shown in Table 1.

Table 1

| Inhibitory activity against rabbit platelet aggregation by PAF | | | |
|---|---|---|---|
| Test compound (Example No.) | Test drug concentration and rate of inhibition (%) | | |
| | 3 x $10^{-7}$M | 3 x $10^{-6}$M | 3 x $10^{-5}$M |
| 2 | 41 | 100 | 100 |
| 6 | 100 | 100 | 100 |

Experiment Example 2

Preventive activity against PAF-induced death in mice

[Method]

The test drug was investigated for the preventive activity against shock death to be brought about within 30 min. after 50 µg kg for PAF in the form of 0.1 ml/kg physiological saline solution was given intravenously to a male Jcl = ICR mouse. The test drug was administered intravenously 5 min. before the injection of PAF.

The test compounds were investigated for the preventive activity against PAF-induced death in mice. The results are shown in Table 2.

Table 2

| Preventive activity against PAF-induced death in mice | | |
|---|---|---|
| Test compound Example No. | Dose mg/kg i.v. | Survival rate (%) |
| (Control) | - | 24 |
| 2 | 1.0 | 100 |
| 2 | 0.3 | 77 |

Experiment Example 3

Restoring activity against PAF-induced hypotension

[Method]

Male S.D. rats, aged 16 weeks, were employed.

Under anesthesia with pentobarbital, cannulas were inserted into the femoral artery and vein of rats for measurement of blood pressure and for administration of a drug, respectively. Three (3) minutes after intravenous administration of 1 $\mu$g/kg (0.25% BAS physiological saline solution; volume of 0.2 ml/kg) of PAF, a test drug (physiological saline solution; volume of 0.5 ml/kg) was administered intravenously, and blood pressure was measured for subsequent 1 hour.

The ratio of hypertension by the test drug to hypotension by PAF was taken as a restoration rate.

Restoration rate (%) = [(Blood pressure just before i.v. administration of a test drug)-(Blood pressure 1 min. after i.v. administration of a test drug)/(Blood pressure just before i.v. administration of PAF) - (Blood pressure just before i.v. administration of a test drug)] x 100

The results are shown in Table 3.

Table 3

| Restoration from PAF-induced hypotension | | |
|---|---|---|
| Test drug (Example No.) | Dose mg/kg i.v. | Restoration rate (%) |
| 6 | 0.1 | 94 |

Experiment Example 4

In accordance with the same procedure as described in Experiment Example 1, the test compounds were investigated for the inhibitory activity against platelet aggregation by PAF. The results are shown in Table 4.

Table 4

| Inhibitory activity against rabbit platelet aggregation by PAF | | | | |
|---|---|---|---|---|
| Test Compound (Example No.) | Test drug concentration and rate of inhibition (%) | | | |
| | $3x10^{-8}$ M | $3x10^{-7}$ M | $3x10^{-6}$ M | $3x10^{-5}$ M |
| 8 | | 100 | 100 | 100 |
| 10 | | 100 | 100 | 100 |
| 11 | | 100 | 100 | |
| 24 | 18 | 100 | 100 | 100 |
| 26 | 25 | 100 | 100 | 100 |
| 27 | | 100 | 100 | |
| 28 | 9 | 100 | 100 | |
| 29 | | 100 | 100 | |
| 32 | | 42 | 100 | |
| 33 | 78 | 100 | 100 | |
| 42 | | 6 | 100 | 100 |
| 43 | | 100 | 100 | 100 |
| 44 | | 100 | 100 | 100 |
| 47 | | 100 | 100 | 100 |
| 48 | | 100 | 100 | 100 |

Experiment Example 5

Inhibitory activity against PAF-induced hypotension in rats

[Method]

Male Spraque-Dawley rats, with a body weight of about 400 g, were used under pentobarbital sodium (50 mg/kg, intravenous administration) anesthesia. A cannula was inserted into the femoral artery and secured in position for measurement of blood pressure and another cannula into the femoral vein for administration of a solution of the drug. The blood pressure was measured via a pressure transducer and recorded on a polygraph. First, 0.3 µg/kg of PAF was administered intravenously and the hypotensive response was recorded.

[Results]

The inhibitory activity against PAF-induced hypotension was estimated from the rate of PAF-induced hypotensive response (Δ mmHg) after the drug administration to hypotensive response (Δ mmHg) by PAF before the drug administration. The results are shown in Table 5.

Table 5

| Restoration activity against PAF-induced hypotension | | | |
|---|---|---|---|
| Test compound (Example No.) | Dose mg/kg, i.v. | Restoration rate (%) | |
| | | After 5 min. | After 60 min. |
| 10 | 1 | 100 | 77 |
| 8 | 1 | 100 | 100 |
| 8 | 0.1 | 100 | 55 |
| 26 | 1 | 100 | 100 |
| 27 | 1 | 100 | 100 |
| 11 | 1 | 100 | 100 |
| 33 | 1 | 100 | 100 |
| 33 | 0.1 | 100 | 39 |

Experiment Example 6

Anti-endotoxin shock activity in rats

[Method]

Male Spraque-Dawley rats weighing about 400 g were used under pentobarbital sodium (50 mg/kg, i.v.) anesthesia. A cannula was inserted into the femoral artery and secured in position for measurement of blood pressure and another cannula into femoral vein for administration of a solution of the drug. The blood pressure was measured via a pressure transducer and recorded on a polygraph. First, a suspension of endotoxin (15 mg/kg) [Lipopolysaccharide W.E. coli 0111: B4, Wako Pure Chemical] in physiological saline was administered intravenously and the maximum hypotensive response (Δ mmHg) occurring 3 to 5 minutes after administration was recorded. Simultaneously, the compound of Example 8 was dissolved in physiological saline and administered intravenously in a dose of 1 to 100 μg/kg. The restoration rate against endotoxin-induced hypotension one minute after the administration was calculated. The presence of preventive activity against endotoxin shock was judged from the strength of the restoration rate.

[Results]

The results are shown in Table 6.

Table 6

| Inhibition against endotoxin-induced hypotension in rats | | | |
|---|---|---|---|
| Compound (Example No.) | Dose mg/kg, i.v. | Number of rats | Restoration rate (%) against endotoxin-induced hypotension |
| 8 | 0.01 | 2 | 100<br>59 |
| | 0.1 | 3 | 97 ± 1* |

* : Mean ± standard error

Preparation Example 1

In 1.0 l of distilled water is dissolved 10 g of 2-O-acetyl-3-O-[N-acetyl-N-(2'-trimethylammonioethyl)]-carbamoyl-1-O-octadecylglycerol-iodide, and after sterile filtration, the solution is distributed and filled in 1 ml portions into 1000 vials and lyophilized, followed by tightly closing.

On the other hand, 21 of distilled water for injection containing 100 g of mannitol is distributed and filled

in 2 ml portions into 1000 ampoules for injection, followed by sealing for tightly closing to prepare 1000 injectable solutions.

On the occasion of use, the powder contained in the former one vial is dissolved in the mannitol solution for injection, and is to be put in use.

## Claims

**Claims for the following Contracting States : BE, CH, DE, FR, GB, IT, LI, LU, NL, SE**

**1.** A compound of the formula:

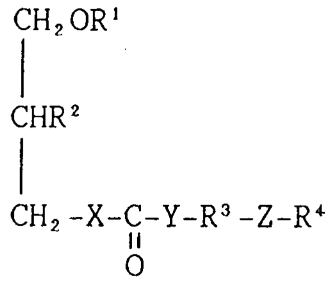

(I)

[wherein $R^1$ is $C_{10-30}$ alkyl or a group of the formula:

$R^5NHCO-$

(in which $R^5$ is $C_{10-30}$ alkyl); $R^2$ is hydrogen, hydroxy, $C_{1-5}$ alkoxy, phenyl-$C_{1-5}$ alkoxy, $C_{1-5}$ alkanoyloxy, benzyloxy, phenoxycarbonyloxy, $C_{1-5}$ alkoxycarbonyloxy, a group of the formula:

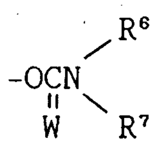

(in which W is oxygen or sulfur, and $R^6$ and $R^7$ are independently hydrogen or $C_{1-5}$ alkyl or both, taken together with the adjacent nitrogen atom, form a 3- to 7-membered hetero ring), amino, $C_{1-5}$ alkanoylamino, benzoylamino, 3- to 7- membered monocyclic amino or $C_{8-9}$ fused cyclic amino; said 3- to 7- membered monocyclic amino or $C_{8-9}$ fused cyclic amino group being unsubstituted or substituted by one or two oxo groups; $R^3$ is a chemical binding or $C_{1-8}$ alkylene unsubstituted or substituted by $C_{1-4}$ alkoxycarbonyl or carboxylato; $R^4$ is hydrogen, $C_{1-6}$ alkyl, $C_{2-6}$ alkenyl or phenyl-$C_{1-6}$ alkyl; X is O or a group of the formula:

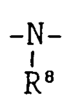

(in which $R^8$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl); Y is O or a group of the formula:

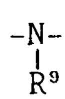

(in which $R^9$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl

or 3- to 7-membered cyclic aminocarbonyl), and $R^8$ and $R^9$, or $R^4$ and $R^9$ may form $C_{1-4}$ alkenylene or alkylene, each of said groups being unsubstituted or substituted by one or two oxo groups; Z is a nitrogen-containing heterocyclic ring which may be substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl, $R^3$ being bound to a position other than the nitrogen atom in said nitrogen-containing heterocyclic ring; provided that X is O, then Y is a group of the formula:

$$\underset{\displaystyle R^{9}}{\underset{|}{-N-}}$$

in which $R^9$ is as defined above] or a pharmaceutically acceptable salt thereof.

**2.** A compound according to Claim 1, wherein $R^1$ is $C_{14-20}$ alkyl or $C_{14-20}$ alkyl-NHCO-.

**3.** A compound according to Claim 1, wherein $R^1$ is $C_{14-18}$ alkyl or $C_{14-18}$ alkyl-NHCO-.

**4.** A compound according to Claim 1, wherein $R^1$ is $C_{15-18}$ alkyl or $C_{15-18}$ alkyl-NHCO-.

**5.** A compound according to claim 1, wherein $R^1$ is $C_{16-18}$ alkyl or $C_{16-18}$ alkyl-NHCO-.

**6.** A compound according to Claim 1, wherein $R^1$ is octadecyl or octadecylcarbamoyl.

**7.** A compound according to Claim 1, wherein $R^1$ is octadecylcarbamoyl.

**8.** A compound according to Claim 1, wherein $R^2$ is $C_{1-5}$ alkoxy, phenyl-$C_{1-5}$ alkoxy, $C_{1-5}$ alkanoyloxy, $C_{1-5}$ alkoxycarbonyloxy, mono- or di-$C_{1-5}$ alkylcarbamoyloxy, $C_{1-5}$ alkanoylamino, 3- to 7-membered cyclic amino or phthalimido.

**9.** A compound according to Claim 1, wherein $R^2$ is $C_{1-5}$ alkoxy.

**10.** A compound according to Claim 1, wherein $R^2$ is methoxy.

**11.** A compound according to Claim 1, wherein $R^3$ is $C_{1-8}$ alkylene.

**12.** A compound according to Claim 1, wherein $R^3$ is methylene, ethylene or trimethylene.

**13.** A compound according to Claim 1, wherein $R^3$ is methylene or ethylene.

**14.** A compound according to Claim 1, wherein $R^4$ is hydrogen or $C_{1-6}$ alkyl.

**15.** A compound according to Claim 1, wherein $R^4$ is hydrogen, methyl or ethyl.

**16.** A compound according to Claim 1, wherein X is O, imino or $C_{1-5}$ alkanoylimino.

**17.** A compound according to Claim 1, wherein X is O.

**18.** A compound according to Claim 1, wherein Y is O or a group of the formula:

$$\underset{\displaystyle R^{9}}{\underset{|}{-N-}}$$

in which $R^9$ is hydrogen, $C_{1-5}$ alkyl, $C_{1-5}$ alkanoyl, $C_{1-5}$ alkoxycarbonyl, carbamoyl, mono- di-$C_{1-5}$ alkylcarbamoyl or 3- to 7-membered cyclic aminocarbonyl.

**19.** A compound according to Claim 1, wherein Y is $C_{1-5}$ alkanoylimino.

**20.** A compound according to Claim 1, wherein Y is acetylimino.

**21.** A compound according to Claim 1, wherein

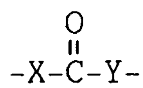

is a group of the formula:

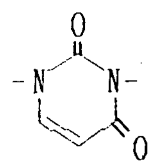

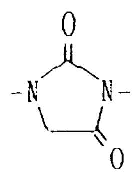

**22.** A compound according to Claim 1, wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted azetidinyl, pyrrolidinyl, piperidinyl, perhydroazepinyl, pyrrolinyl, pyrazolinyl, pyrrolyl, pyridyl, oxazolyl, thiazolyl, pyridazinyl, pyrimidyl, pyrazinyl, imidazolyl, morpholinyl, thiomorpholinyl, piperazinyl, pyrazolidinyl, indolyl, isoindolyl, 1H-indazolyl, purinyl, quinolinyl, isoquinolinyl, perhydroindolyl or perhydroisoquinolinyl group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**23.** A compound according to Claim 1, wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted morpholinyl, pyrrolidinyl, piperidinyl, imidazolyl, pyridyl or thiazolyl group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**24.** A compound according to Claim 1, wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted pyridyl or thiazolyl group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxyl, carboxylato or $C_{1-4}$ alkoxycarbonyl.

**25.** A compound according to Claim 1, wherein Z-$R^4$ is pyridyl or thiazolyl.

**26.** A compound according to Claim 1, wherein Z-$R^4$ is 2-pyridyl, thiazol-2-yl or thiazol-4-yl.

**27.** A compound according to Claim 1, wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted imidazolio, pyridinio, oxazolio, thiazolio, pyridazinio, pyrimidinio, pyrazinio, quinolinio, isoquinolinio, morpholinio, piperidinio, piperazinio or pyrrolidinio group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**28.** A compund according to Claim 1, wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted morpholinio, pyrrolidinio, piperidinio, imidazolio, pyridinio or thiazolio group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**29.** A compound according to Claim 1, wherein Z-$R^4$ is an optionally $C_{1-6}$ alkyl, $C_{1-4}$ alkoxycarbonyl-$C_{1-6}$ alkyl

alkyl, carboxylato-$C_{1-6}$ alkyl or phenyl-$C_{1-6}$ alkyl substituted pyridinio or thiazolio group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**30.** A compound according to Claim 1, wherein Z-$R^4$ is 1-($C_{1-6}$ alkyl)pyridinio-2-yl.

**31.** A compound according to Claim 1, wherein Z-$R^4$ is 1-ethylpyridinio-2-yl.

**32.** A compound according to Claim 1, wherein Z-$R^4$ is 3-($C_{1-6}$ alkyl)thiazolio-2-yl.

**33.** A compound according to Claim 1, wherein Z-$R^4$ is 3-ethylthiazolio-2-yl.

**34.** A compound according to Claim 1, wherein Z-$R^4$ is 3-($C_{1-6}$ alkyl)thiazolio-4-yl.

**35.** A compound according to Claim 1, wherein Z-$R^4$ is 3-ethylthiazolio-4-yl.

**36.** A compound according to Claim 1, which is 3-O-[N-acetyl-N-(N-methylpyridinio-2-yl)methyl]carbamoyl-2-O-methyl-1-octadecylcarbamoylglycerol chloride.

**37.** A compound according to Claim 1, which is 1-ethyl-2-[N-acetyl-N-(3-octadecylcarbamoyloxy-2-methoxypropyloxy)carbonylamino]methylpyridinium halide.

**38.** A compound according to Claim 1, which is 2-[N-acetyl-N-(3-octadecylcarbamoyloxy-2-methoxypropoxycarbonyl)amino] methyl-3-methylthiazolium halide.

**39.** A compound according to Claim 1, which is 2-[N-acetyl-N-(3-octadecylcarbamoyloxy-2-methoxypropoxycarbonyl)amino]methyl-3-ethylthiazolium halide.

**40.** A compound according to Claim 1, which is 2-[N-(3-octadecylcarbamoyloxy-2-ethoxypropyloxycarbonyl)amino]methyl-N-ethylpyridinium halide.

**41.** A compound according to Claim 1, which is 4-[N-acetyl-N-(3-octadecylcarbamoyloxy-2-methoxypropoxycarbonyl)amino]methyl-3-ethylthiazolium halide.

**42.** A compound according to Claim 37, wherein halide is chloride.

**43.** A compound according to Claim 38, wherein halide is iodide.

**44.** A compound according to claim 39, wherein halide is iodide.

**45.** A compound according to Claim 40, wherein halide is iodide.

**46.** A compound according to Claim 41, wherein halide is iodide.

**47.** A compound according to Claim 1, wherein $R^3$ is $C_{1-8}$ alkylene and Z is a nitrogen-containing heterocyclic ring which may be substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**48.** A pharmaceutical composition which comprises, as an active ingredient, an effective amount of a compound as claimed in any one of Claims 1 to 47 in asociation with a pharmaceutically acceptable carrier, excipient or diluent therefor.

**49.** A compound as claimed in any one of Claims 1 to 47 or a pharmaceutical composition as claimed in Claim 48 for use in the production of a preventive or therapeutic agent for a variety of circulatory diseases and allergic disorders or of an antineoplastic agent.

**50.** A process for producing a compound of the formula:

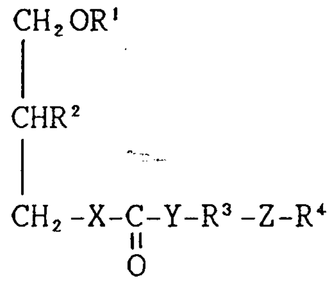

(I)

[wherein $R^1$ is $C_{10-30}$ alkyl or a group of the formula:

$R^5NHCO$-

(in which $R^5$ is $C_{10-30}$ alkyl); $R^2$ is hydrogen, hydroxy, $C_{1-5}$ alkoxy, phenyl-$C_{1-5}$ alkoxy, $C_{1-5}$ alkanoyloxy, benzyloxy, phenoxycarbonyloxy, $C_{1-5}$ alkoxycarbonyloxy, a group of the formula:

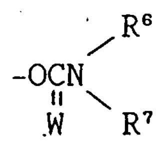

(in which W is oxygen or sulfur, and $R^6$ and $R^7$ are independently hydrogen or $C_{1-5}$ alkyl or both, taken together with the adjacent nitrogen atom, form a 3- to 7-membered hetero ring), amino, $C_{1-5}$ alkanoylamino, benzoylamino, 3- to 7- membered monocyclic amino or $C_{8-9}$ fused cyclic amino; said 3- to 7- membered monocyclic amino or $C_{8-9}$ fused cyclic amino group being unsubstituted or substituted by one or two oxo groups; $R^3$ is a chemical binding or $C_{1-8}$ alkylene unsubstituted or substituted by $C_{1-4}$ alkoxycarbonyl or carboxylato; $R^4$ is hydrogen, $C_{1-6}$ alkyl, $C_{2-6}$ alkenyl or phenyl-$C_{1-6}$ alkyl; X is O or a group of the formula:

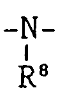

(in which $R^8$ is hydrogen, optionally carboxy or $C_{1-5}$ alkoxycarbonyl substituted $C_{1-5}$ alkyl; $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl); Y is O or a group of the formula:

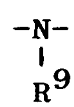

(in which $R^9$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl), and $R^8$ and $R^9$ or $R^4$ and $R^9$ may form $C_{1-4}$ alkenylene or alkylene unsubstituted or substituted by oxo; Z is a nitrogen-containing heterocyclic ring which may be substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl, $R^3$ being bound to a position other than the nitrogen atom in said nitrogen-containing heterocyclic ring; provided that X is O, then Y is a group of the formula:

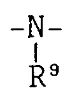

in which $R^9$ is as defined above] or a pharmaceutically acceptable salt thereof, which comprises

a) reacting a compound of the formula:

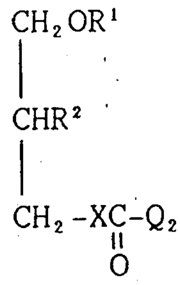

[wherein $Q_2$ is a group which activates a carbonyl group]
with a compound of the formula:

$HY-R^3-Z-R^4$

[wherein all symbols are as defined above], or

b) reacting a compound of the formula:

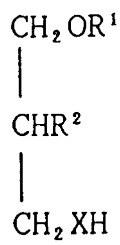

[wherein all symbols are as defined above]
with a compound of the formula:

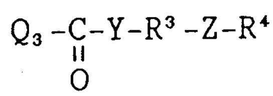

[wherein $Q_3$ is a group which activates a carbonyl group and the other symbols are as defined above], or

c) reacting a compound of the formula:

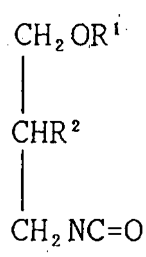

[wherein all symbols are as defined above]
with a compound of the formula:

$HY-R^3-Z-R^4$

[wherein all symbols are as defined above] , to provide a compound of the formula (I) wherein X is an imino group, or

d) reacting a compound of the formula:

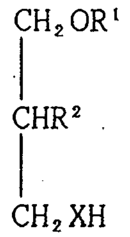

[wherein all symbols are as defined above]
with a compound of the formula:

$O=C=N\text{-}R^3\text{-}Z\text{-}R^4$

[wherein all symbols are as defined above], to provide a compound of the formula (I) wherein Y is an imino group,
e) subjecting a compound of the formula (I) wherein $R^2$ is benzyloxy, to catalytic reduction to provide a compound of the formula (I) wherein $R^2$ is hydroxy, or
f) subjecting a compound of the formula (I) wherein $R^2$ is hydroxy, to an acylation or carbamoylation reaction, to provide a compound of tee formula (I) wherein $R^2$ is acyloxy or a group represented by the formula:

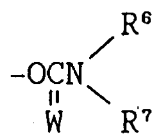

(in which all symbols are as defined above], or
g) reacting a compound of the formula (I) wherein X and/or Y is an unsubstituted imino group with acid anhydride, acid halide, alkyl halide or alkyl isocyanate, to provide a compound of the formula (I) wherein X is a group of the formula:

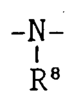

(in which $R^8$ is optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl)
and/or Y is a group of the formula:

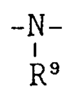

(in which $R^9$ is optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl), or
h) reacting a compound of the formula (I) wherein a nitrogen atom in the group represented by Z is primary, secondary or tertiary amino with alkyl halide, to provide a compound of the formula (I) wherein a nitrogen atom in the group represented by Z is secondary, tertiary or quaternary amino, or
i) reacting a compound of the formula (I) wherein $R^1$ is hydrogen with alkyl isocyanate to provide a compound of the formula (I) wherein $R^1$ is a group of the formula:

$R^5NHCO$-

(in which $R^5$ is $C_{10-30}$ alkyl), and if desired,
j) converting thus obtained compound of the formula (I) into a salt thereof.

**Claims for the following Contracting State : AT**

**1.** A process for producing a compound of the formula:

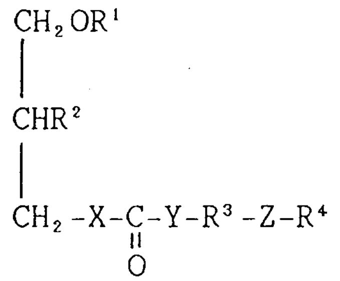

(I)

[wherein $R^1$ is $C_{10-30}$ alkyl or a group of the formula:

$R^5NHCO$-

(in which $R^5$ is $C_{10-30}$ alkyl); $R^2$ is hydrogen, hydroxy, $C_{1-5}$ alkoxy, phenyl-$C_{1-5}$ alkoxy, $C_{1-5}$ alkanoyloxy, benzyloxy, phenoxycarbonyloxy, $C_{1-5}$ alkoxycarbonyloxy, a group of the formula:

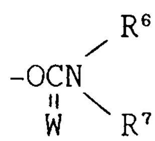

(in which W is oxygen or sulfur, and $R^6$ and $R^7$ are independently hydrogen or $C_{1-5}$ alkyl or both, taken together with the adjacent nitrogen atom, form a 3- to 7-membered hetero ring), amino, $C_{1-5}$ alkanoylamino, benzoylamino, 3- to 7- membered monocyclic amino or $C_{8-9}$ fused cyclic amino; said 3- to 7- membered monocyclic amino or $C_{8-9}$ fused cyclic amino group being unsubstituted or substituted by one or two oxo groups; $R^3$ is a chemical binding or $C_{1-8}$ alkylene unsubstituted or substituted by $C_{1-4}$ alkoxycarbonyl or carboxylato; $R^4$ is hydrogen, $C_{1-6}$ alkyl, $C_{2-6}$ alkenyl or phenyl-$C_{1-6}$ alkyl; X is O or a group of the formula:

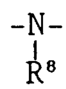

(in which $R^8$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl); Y is O or a group of the formula:

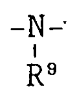

(in which $R^9$ is hydrogen, optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl), and $R^8$ and $R^9$ or $R^4$ and $R^9$ may form $C_{1-4}$ alkenylene or

alkylene unsubstituted or substituted by oxo; Z is a nitrogen-containing heterocyclic ring which may be substituted by optionally hydroxy or amino substituted $C_{1-4}$ alkyl, hydroxy,, amino, imino, mono- or di-$C_{1-4}$ alkylamino, carbamoyl, ureido, carboxy, carboxylato or $C_{1-4}$ alkoxycarbonyl, $R^3$ being bound to a position other than the nitrogen atom in said nitrogen-containing heterocyclic ring; provided that X is O, then Y is a group of the formula:

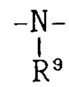

in which $R^9$ is as defined above] or a pharmaceutically acceptable salt thereof, which comprises

a) reacting a compound of the formula:

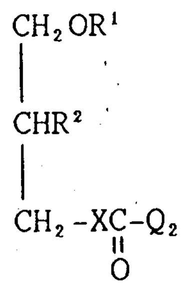

[wherein $Q_2$ is a group which may activate a carbonyl group]
with a compound of the formula:

$HY\text{-}R^3\text{-}Z\text{-}R^4$

[wherein all symbols are as defined above], or

b) reacting a compound of the formula:

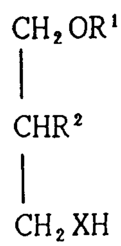

[wherein all symbols are as defined above]
with a compound of the formula:

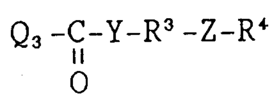

[wherein $Q_3$ is a group which activates a carbonyl group and the other symbols are as defined above], or

c) reacting a compound of the formula:

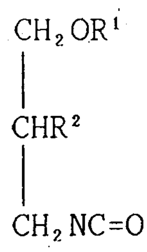

[wherein all symbols are as defined above]
with a compound of the formula:

HY-$R^3$-Z-$R^4$

[wherein all symbols are as defined above], to provide a compound of the formula (I) wherein X is an imino group, or
d) reacting a compound of the formula:

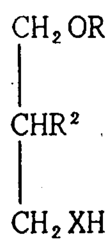

[wherein all symbols are as defined above]
with a compound of the formula:

O = C = N-$R^3$-Z-$R^4$

[wherein all symbols are as defined above], to provide a compound of the formula (I) wherein Y is an imino group,
e) subjecting a compound of the formula (I) wherein $R^2$ is benzyloxy, to catalytic reduction to provide a compound of the formula (I) wherein $R^2$ is hydroxy, or
f) subjecting a compound of the formula (I) wherein $R^2$ is hydroxy, to an acylation or carbamoylation reaction, to provide a compound of the formula (I) wherein $R^2$ is acyloxy or a group represented by the formula:

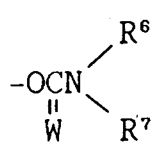

(in which all symbols are as defined above], or
g) reacting a compound of the formula (I) wherein X and/or Y is an unsubstituted imino group with acid anhydride, acid halide, alkyl halide or alkyl isocyanate, to provide a compound of the formula (I) wherein X is a group of the formula:

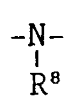

(in which $R^8$ is optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3-

to 7-membered cyclic aminocarbonyl) and/or Y is a group of the formula:

$$-\underset{R^9}{\underset{|}{N}}-$$

(in which $R^9$ is optionally carboxy or $C_{1-5}$ alkoxycarbonyl substituted $C_{1-5}$ alkyl, $C_{1-5}$ alkanoyl, benzoyl, phenoxycarbonyl, $C_{1-5}$ alkoxycarbonyl, carbamoyl, mono- or di-$C_{1-5}$ alkylcarbamoyl or 3- to 7-membered cyclic aminocarbonyl), or

h) reacting a compound of the formula (I) wherein a nitrogen atom in the group represented by Z is primary, secondary or tertiary amino with alkyl halide, to provide a compound of the formula (I) wherein a nitrogen atom in the group represented by Z is secondary, tertiary or quaternary amino, or

i) reacting a compound of the formula (I) wherein $R^1$ is hydrogen with alkyl isocyanate to provide a compound of the formula (I) wherein $R^1$ is a group of the formula:

$R^5NHCO$-

(in which $R^5$ is $C_{10-30}$ alkyl), and if desired,

j) converting thus obtained compound of the formula (I) into a salt thereof.

**2.** A process according to Claim 1, wherein the product is a compound of the formula (I) wherein $R^1$ is $C_{14-20}$ alkyl or $C_{14-20}$ alkyl-NHCO-.

**3.** A process according to Claim 1, wherein the product is a compound of the formula (I) wherein $R^1$ is $C_{14-18}$ alkyl or $C_{14-18}$ alkyl-NHCO-.

**4.** A process according to Claim 1, wherein the product is a compound of the formula (I) wherein $R^1$ is $C_{15-18}$ alkyl or $C_{15-18}$ alkyl-NHCO-.

**5.** A process according to Claim 1, wherein the product is a compound of the formula (I) wherein $R^1$ is $C_{16-18}$ alkyl or $C_{16-18}$ alkyl-NHCO-.

**6.** A process according to Claim 1, wherein the product is a compound of the formula (I) wherein $R^1$ is octadecyl or octadecylcarbamoyl.

**7.** A process according to Claim 1, wherein the product is a compound of the formula (I) wherein $R^1$ is octadecylcarbamoyl.

**8.** A process according to Claim 1, wherein the product is a compound of the formula (I) wherein $R^2$ is $C_{1-5}$ alkoxy, phenyl-$C_{1-5}$ alkoxy, $C_{1-5}$ alkanoyloxy, $C_{1-5}$ alkoxycarbonyloxy, mono- or di-$C_{1-5}$ alkylcarbamoyloxy, $C_{1-5}$ alkanoylamino, 3- to 7-membered cyclic amino or phthalimido.

**9.** A process according to Claim 1, wherein the product is a compound of the formula (I) wherein $R^2$ is $C_{1-5}$ alkoxy.

**10.** A process according to Claim 1, wherein the product is a compound of the formula (I) wherein $R^2$ is methoxy.

**11.** A process according to Claim 1, wherein the product is a compound of the formula (I) wherein $R^3$ is $C_{1-8}$ alkylene.

**12.** A process according to Claim 1, wherein the product is a compound of the formula (I) wherein $R^3$ is methylene, ethylene or trimethylene.

**13.** A process according to Claim 1, wherein the product is a compound of the formula (I) wherein $R^3$ is methylene or ethylene.

**14.** A process according to Claim 1, wherein the product is a compound of the formula (I) wherein $R^4$ is hydrogen or $C_{1-6}$ alkyl.

**15.** A process according to Claim 1, wherein the product is a compound of the formula (I) wherein $R^4$ is hydrogen, methyl or ethyl.

**16.** A process according to Claim 1, wherein the product is a compound of the formula (I) wherein X is O, imino or $C_{1-5}$ alkanoylimino.

**17.** A process according to Claim 1, wherein the product is a compound of the formula (I) wherein X is O.

**18.** A process according to Claim 1, wherein the product is a compound of the formula (I) wherein Y is O or a group of the formula:

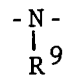

in which $R^9$ is hydrogen, $C_{1-5}$ alkyl, $C_{1-5}$ alkanoyl, $C_{1-5}$ alkoxycarbonyl, carbamoyl, mono- or di-$C_{1-5}$ alkylcarbamoyl or 3 - to 7-membered cyclic aminocarbonyl.

**19.** A process according to Claim 1, wherein the product is a compound of the formula (I) wherein Y is $C_{1-5}$ alkanoylimino.

**20.** A process according to Claim 1, wherein the product is a compound of the formula (I) wherein Y is acetylimino.

**21.** A process according to Claim 1, wherein the product is a compound of the formula (I) wherein

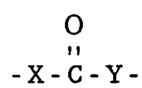

is a group of

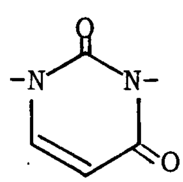

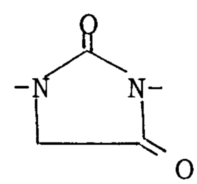

**22.** A process according to Claim 1, wherein the product is a compound of the formula (I) wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted azetidinyl, pyrrolidinyl, piperidinyl, perhydrozepinyl, pyrrolinyl, pyrazolinyl, pyrrolyl, pyridyl, oxazolyl, thiazolyl, pyridazinyl, pyrimidyl, pyradinyl, imidazolyl, morpholinyl, thiomorpholinyl, piperazinyl, pyrazolidinyl, indolyl, isoindolyl, 1H-indazolyl, purinyl, quinolinyl, isoquinolinyl, perhydroindolyl or perhydroisoquinolinyl group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**23.** A process according to Claim 1, wherein the product is a compound of the formula (I) wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted morpholinyl, pyrrolidinyl, piperidinyl, imidazolyl, pyridyl or thiazolyl group being unsubstituted or substituted by optionally hydroxy or amino substituted $C_{1-4}$ alkyl, hydroxy, amino, imino,

mono- or di-$C_{1-4}$ alkylamino, carbamoyl, ureido, carboxy, carboxylato or $C_{1-4}$ alkoxycarbonyl.

**24.** A process according to Claim 1, wherein the product is a compound of the formula (I) wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted pyridyl or thiazolyl group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxyl, carboxylato or $C_{1-4}$ alkoxycarbonyl.

**25.** A process according to Claim 1, wherein the product is a compound of the formula (I) wherein Z-$R^4$ is pyridyl or thiazolyl.

**26.** A process according to Claim 1, wherein the product is a compound of the formula (I) wherein Z-$R^4$ is 2-pyridyl, thiazol-2-yl or thiazol-4-yl.

**27.** A process according to Claim 1, wherein the product is a compound of the formula (I) wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted imidazolio, pyridinio, oxazolio, thiazolio, pyridazinio, pyrimidinio, pyrazinio, quinolinio, isoquinolinio, morpholinio, piperidinio, piperazinio or pyrrolidinio group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**28.** A process according to Claim 1, wherein the product is a compound of the formula (I) wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted morpholinio, pyrrolidinio, piperidinio, imidazolio, pyridinio or thiazolio group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**29.** A process according to Claim 1, wherein the product is a compound of the formula (I) wherein Z-$R^4$ is an optionally $C_{1-6}$ alkyl, $C_{1-4}$ alkoxycarbonyl-$C_{1-6}$ alkyl, carboxylato-$C_{1-6}$ alkyl or phenyl-$C_{1-6}$ alkyl substituted pyridinio or thiazolio group being unsubstituted or substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**30.** A process according to Claim 1, wherein the product is a compound of the formula (I) wherein Z-$R^4$ is 1-($C_{1-6}$ alkyl)pyridinio-2-yl.

**31.** A process according to Claim 1, wherein the product is a compound of the formula (I) wherein Z-$R^4$ is 1-ethylpyridinio-2-yl.

**32.** A process according to Claim 1, wherein the product is a compound of the formula (I) wherein Z-$R^4$ is 3-($C_{1-6}$ alkyl)thiazolio-2-yl.

**33.** A process according to Claim 1, wherein the product is a compound of the formula (I) wherein Z-$R^4$ is 3-ethylthiazolio-2-yl.

**34.** A process according to Claim 1, wherein the product is a compound of the formula (I) wherein Z-$R^4$ is 3-($C_{1-6}$ alkyl)thiazolio-4-yl.

**35.** A process according to Claim 1, wherein the product is a compound of the formula (I) wherein Z-$R^4$ is 3-ethylthiazolio-4-yl.

**36.** A process according to Claim 1, wherein the product is 3-O-[N-acetyl-N-(N-methylpyridinio-2-yl)-methyl]carbamoyl-2-O-methyl-1-octadecylcarbamoylglycerol chloride.

**37.** A process according to Claim 1, wherein the product is 1-ethyl-2-[N-acetyl-N-(3-octadecylcarbamoyloxy-2-methoxypropyloxy)carbonyl-amino]methylpyridinium halide.

**38.** A process according to Claim 1, wherein the product is 2-[N-acetyl-N-(3-octadecylcarbamoyloxy-2-methoxypropoxycarbonyl)amino]methyl-3-methylthiazolium halide.

**39.** A process according to Claim 1, wherein the product is 2-[N-acetyl-N-(3-octadecylcarbamoyloxy-2-methoxypropoxycarbonyl)amino]methyl-3-ethylthiazolium halide.

**40.** A process according to Claim 1, wherein the product is 2-[N-(3-octadecylcarbamoyloxy-2-ethoxypropyloxycarbonyl)amino]methyl-N-ethylpyridinium halide.

**41.** A process according to Claim 1, wherein the product is 4-[N-acetyl-N-(3-octadecylcarbamoyloxy-2-methoxypropoxycarbonyl)amino]methyl-3-ethylthiazolium halide.

**42.** A process according to Claim 37, wherein halide is chloride.

**43.** A process according to Claim 38, wherein halide is iodide.

**44.** A process according to Claim 39, wherein halide is iodide.

**45.** A process according to Claim 40, wherein halide is iodide.

**46.** A process according to Claim 41, wherein halide is iodide.

**47.** A process according to Claim 1, wherein $R^3$ is $C_{1-8}$ alkylene and Z is a nitrogen-containing heterocyclic ring which may be substituted by optionally hydroxy or amino substituted $C_{1-4}$ alkyl, hydroxy, amino, imino, mono- or di-$C_{1-4}$ alkylamino, carbamoyl, ureido, carboxy, carboxylato or $C_{1-4}$ alkoxycarbonyl.

**Patentansprüche**
**Patentansprüche für folgende Vertragsstaaten : BE, CH, DE, FR, GB, IT, LI, LU, NL, SE**

**1.** Verbindung der Formel

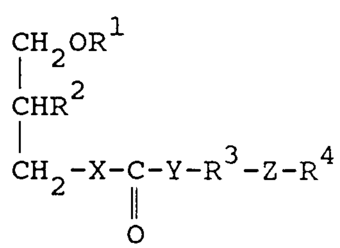

(I) ,

[worin

$R^1$ $C_{10-30}$-Alkyl oder eine Gruppe der Formel $R^5NHCO$- ist, (worin $R^5$ $C_{10-30}$-Alkyl ist);

$R^2$ Wasserstoff, Hydroxy, $C_{1-5}$-Alkoxy, Phenyl-$C_{1-5}$-alkoxy, $C_{1-5}$-Alkanoyloxy, Benzyloxy, Phenoxycarbonyloxy, $C_{1-5}$-Alkoxycarbonyloxy, eine Gruppe der Formel

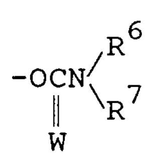

(worin W Sauerstoff oder Schwefel ist und $R^6$ und $R^7$ unabhängig Wasserstoff oder $C_{1-5}$-Alkyl sind oder beide, mit dem benachbarten Stickstoff-Atom zusammengenommen, einen 3- bis 7-gliedrigen Hetero-Ring bilden), Amino, $C_{1-5}$-Alkanoylamino, Benzoylamino, 3- bis 7-gliedriges monocyclisches Amino oder $C_{8-9}$-anelliertes cyclisches Amino ist, wobei die genannten 3- bis 7-gliedrigen monocyclischen Amino-Gruppen oder $C_{8-9}$-anellierten cyclischen Amino-Gruppen unsubstituiert oder mit einer oder zwei Oxo-Gruppen substituiert sind;

$R^3$ eine chemische Bindung oder ein $C_{1-8}$-Alkylen ist, das unsubstituiert oder mit $C_{1-4}$-

Alkoxycarbonyl oder Carboxylato substituiert ist;

$R^4$ Wasserstoff, $C_{1-6}$-Alkyl, $C_{2-6}$-Alkenyl oder Phenyl-$C_{1-6}$-alkyl ist;

X O oder eine Gruppe der Formel

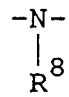

ist (worin $R^8$ Wasserstoff, gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist);

Y O oder eine Gruppe der Formel

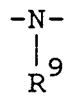

ist (worin $R^9$ Wasserstoff, gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist) und $R^8$ und $R^9$ oder $R^4$ und $R^9$ $C_{1-4}$-Alkenylen oder Alkylen bilden können, wobei jede der genannten Gruppen unsubstituiert oder mit einer oder zwei Oxo-Gruppen substituiert ist;

Z ein Stickstoff enthaltender heterocyclischer Ring ist, der durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert sein kann, wobei $R^3$ an eine Position gebunden ist, die nicht diejenige des Stickstoff-Atoms in dem genannten Stickstoff enthaltenden heterocyclischen Ring ist;

mit der Maßgabe, daß dann, wenn X O ist, Y eine Gruppe der Formel

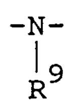

ist, worin $R^9$ die oben angegebenen Bedeutungen hat] oder ein pharmazeutisch annehmbares Salz derselben.

**2.** Verbindung nach Anspruch 1, worin $R^1$ $C_{14-20}$-Alkyl oder $C_{14-20}$-Alkyl-NHCO- ist.

**3.** Verbindung nach Anspruch 1, worin $R^1$ $C_{14-18}$-Alkyl oder $C_{14-18}$-Alkyl-NHCO- ist.

**4.** Verbindung nach Anspruch 1, worin $R^1$ $C_{15-18}$-Alkyl oder $C_{15-18}$-Alkyl-NHCO- ist.

**5.** Verbindung nach Anspruch 1, worin $R^1$ $C_{16-18}$-Alkyl oder $C_{16-18}$-Alkyl-NHCO- ist.

**6.** Verbindung nach Anspruch 1, worin $R^1$ Octadecyl oder Octadecylcarbamoyl ist.

**7.** Verbindung nach Anspruch 1, worin $R^1$ Octadecylcarbamoyl ist.

**8.** Verbindung nach Anspruch 1, worin $R^2$ $C_{1-5}$-Alkoxy, Phenyl-$C_{1-5}$-alkoxy, $C_{1-5}$-Alkanoyloxy, $C_{1-5}$-Alkoxycarbonyloxy, Mono- oder Di-$C_{1-5}$-alkylcarbamoyloxy, $C_{1-5}$-Alkanoylamino, 3- bis 7-gliedriges cyclisches Amino oder Phthalimido ist.

**9.** Verbindung nach Anspruch 1, worin $R^2$ $C_{1-5}$-Alkoxy ist.

**10.** Verbindung nach Anspruch 1, worin $R^2$ Methoxy ist.

**11.** Verbindung nach Anspruch 1, worin $R^3$ $C_{1-8}$-Alkylen ist.

**12.** Verbindung nach Anspruch 1, worin $R^3$ Methylen, Ethylen oder Trimethylen ist.

**13.** Verbindung nach Anspruch 1, worin $R^3$ Methylen oder Ethylen ist.

**14.** Verbindung nach Anspruch 1, worin $R^4$ Wasserstoff oder $C_{1-6}$-Alkyl ist.

**15.** Verbindung nach Anspruch 1, worin $R^4$ Wasserstoff, Methyl oder Ethyl ist.

**16.** Verbindung nach Anspruch 1, worin X O, Imino oder $C_{1-5}$-Alkanoylimino ist.

**17.** Verbindung nach Anspruch 1, worin X O ist.

**18.** Verbindung nach Anspruch 1, worin Y O oder eine Gruppe der Formel

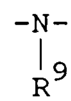

ist, worin $R^9$ Wasserstoff, $C_{1-5}$-Alkyl, $C_{1-5}$-Alkanoyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist.

**19.** Verbindung nach Anspruch 1, worin Y $C_{1-5}$-Alkanoylimino ist.

**20.** Verbindung nach Anspruch 1, worin Y Acetylimino ist.

**21.** Verbindung nach Anspruch 1, worin

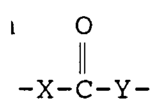

eine Gruppe der Formel

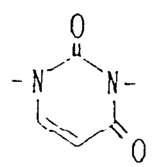

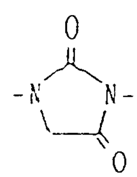

ist.

**22.** Verbindung nach Anspruch 1, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Azetidinyl-, Pyrrolidinyl-, Piperidinyl-, Perhydroazepinyl-, Pyrrolinyl-, Pyrazolinyl-, Pyrrolyl-, Pyridyl-, Oxazolyl-, Thiazolyl-, Pyridazinyl-, Pyrimidyl-, Pyrazinyl-, Imidazolyl-, Morpholinyl-, Thiomorpholinyl-, Piperazinyl-, Pyrazolidinyl-, Indolyl-, Isoindolyl-, 1H-Indazolyl-, Purinyl-, Chinolinyl-, Isochinolinyl-, Perhydroindolyl- oder Perhydroisochinolyl-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**23.** Verbindung nach Anspruch 1, worin Z-$R^4$ eine gegebenenfalls durch $C_{1-6}$-Alkyl, $C_{1-4}$-Alkoxycarbonyl-

$C_{1-6}$-alkyl, Carboxylato-$C_{1-6}$-alkyl oder Phenyl-$C_{1-6}$-alkyl substituierte Morpholinyl-, Pyrrolidinyl-, Piperidinyl-, Imidazolyl-, Pyridyl- oder Thiazolyl-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**24.** Verbindung nach Anspruch 1, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Pyridyl- oder Thiazolyl-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**25.** Verbindung nach Anspruch 1, worin Z-$R^4$ Pyridyl oder Thiazolyl ist.

**26.** Verbindung nach Anspruch 1, worin Z-$R^4$ 2-Pyridyl, Thiazol-2-yl oder Thiazol-4-yl ist.

**27.** Verbindung nach Anspruch 1, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Imidazolio-, Pyridinio-, Oxazolio-, Thiazolio-, Pyridazinio-, Pyrimidinio-, Pyrazinio-, Chinolinio-, Isochinolinio-, Morpholinio-, Piperidinio-, Piperazinio- oder Pyrrolidinio-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**28.** Verbindung nach Anspruch 1, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Morpholinio-, Pyrrolidinio-, Piperidinio-, Imidazolio-, Pyridinio- oder Thiazolio-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**29.** Verbindung nach Anspruch 1, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Pyridinio- oder Thiazolio-Gruppe ist, die unsubstituiert oder durch gegebenenfalls Hydroxy- oder Amino-substituiertes $C_{1-4}$-Alkyl, Hydroxy, Amino, Imino, Monooder Di-$C_{1-4}$-alkylamino, Carbamoyl, Ureido, Carboxy, Carboxylato oder $C_{1-4}$-Alkoxycarbonyl substituiert ist.

**30.** Verbindung nach Anspruch 1, worin Z-$R^4$ 1-($C_{1-6}$-Alkyl)-pyridino-2-yl ist.

**31.** Verbindung nach Anspruch 1, worin Z-$R^4$ 1-Ethylpyridino-2-yl ist.

**32.** Verbindung nach Anspruch 1, worin Z-$R^4$ 3-($C_{1-6}$-Alkyl)-thiazolio-2-yl ist.

**33.** Verbindung nach Anspruch 1, worin Z-$R^4$ 3-Ethyl-thiazolio-2-yl ist.

**34.** Verbindung nach Anspruch 1, worin Z-$R^4$ 3-($C_{1-6}$-Alkyl)thiazolio-4-yl ist.

**35.** Verbindung nach Anspruch 1, worin Z-$R^4$ 3-Ethylthiazolio-4-yl ist.

**36.** Verbindung nach Anspruch 1, die 3-O-[N-Acetyl-N-(N-methylpyridinio-2-yl)methyl]carbamoyl-2-O-methyl-1-octadecylcarbamoylglycerin-chlorid ist.

**37.** Verbindung nach Anspruch 1, die 1-Ethyl-2-[N-acetyl-N-(3-octadecylcarbamoyloxy-2-methoxypropyloxy)carbonylamino]methylpyridiniumhalogenid ist.

**38.** Verbindung nach Anspruch 1, die 2-[N-Acetyl-N-(3-octadecylcarbamoyloxy-2-methoxypropyloxy)-carbonyl)amino]methyl-3-methylthiazoliumhalogenid ist.

**39.** Verbindung nach Anspruch 1, die 2-[N-Acetyl-N-(3-octadecylcarbamoyloxy-2-methoxypropyloxy)-carbonyl)amino]methyl-3-ethylthiazoliumhalogenid ist.

**40.** Verbindung nach Anspruch 1, die 2-[N-(3-octadecylcarbamoyloxy-2-ethoxypropyloxycarbonyl)amino]-methyl-N-pyridiniumhalogenid ist.

**41.** Verbindung nach Anspruch 1, die 4-[N-Acetyl-N-(3-octadecylcarbamoyloxy-2-methoxypropyloxycarbonyl)amino]methyl-3-ethylthiazoliumhalogenid ist.

**42.** Verbindung nach Anspruch 37, worin das Halogenid Chlorid ist.

**43.** Verbindung nach Anspruch 38, worin das Halogenid Iodid ist.

**44.** Verbindung nach Anspruch 39, worin das Halogenid Iodid ist.

**45.** Verbindung nach Anspruch 40, worin das Halogenid Iodid ist.

**46.** Verbindung nach Anspruch 41, worin das Halogenid Iodid ist.

**47.** Verbindung nach Anspruch 1, worin $R^3$ $C_{1-8}$-Alkylen ist und Z ein Stickstoff enthaltender heterocyclischer Ring ist, der durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert sein kann.

**48.** Pharmazeutische Zusammensetzung, umfassend als Wirkstoff eine wirksame Menge einer Verbindung nach irgendeinem der Ansprüche 1 bis 47 in Verbindung mit einem pharmazeutisch annehmbaren Träger, Streckmittel oder Verdünnungsmittel für diese.

**49.** Verbindung nach irgendeinem der Ansprüche 1 bis 47 oder pharmazeutische Zusammensetzung nach Anspruch 48 zur Verwendung bei der Herstellung eines präventiven oder therapeutischen Mittels für eine Vielfalt von Kreislauf-Erkrankungen und allergischen Störungen oder eines antineoplastischen Mittels.

**50.** Verfahren zur Herstellung einer Verbindung der Formel

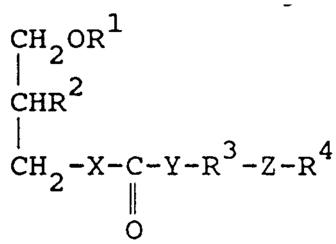

(I) ,

[worin

$R^1$ $C_{10-30}$-Alkyl oder eine Gruppe der Formel $R^5NHCO$- ist, (worin $R^5$ $C_{10-30}$-Alkyl ist);

$R^2$ Wasserstoff, Hydroxy, $C_{1-5}$-Alkoxy, Phenyl-$C_{1-5}$-alkoxy, $C_{1-5}$-Alkanoyloxy, Benzyloxy, Phenoxycarbonyloxy, $C_{1-5}$-Alkoxycarbonyloxy, eine Gruppe der Formel

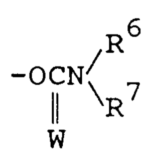

(worin W Sauerstoff oder Schwefel ist und $R^6$ und $R^7$ unabhängig Wasserstoff oder $C_{1-5}$-Alkyl sind oder beide, mit dem benachbarten Stickstoff-Atom zusammengenommen, einen 3- bis 7-gliedrigen Hetero-Ring bilden), Amino, $C_{1-5}$-Alkanoylamino, Benzoylamino, 3- bis 7-gliedriges monocyclisches Amino oder $C_{8-9}$-anelliertes cyclisches Amino ist, wobei die genannten 3- bis 7-gliedrigen monocyclischen Amino-Gruppen oder $C_{8-9}$-anellierten cyclischen Amino-Gruppen unsubstituiert oder mit einer oder zwei Oxo-Gruppen substituiert sind;

R3 eine chemische Bindung oder ein $C_{1-8}$-Alkylen ist, das unsubstituiert oder mit $C_{1-4}$-Alkoxycarbonyl oder Carboxylato substituiert ist;

$R^4$ Wasserstoff, $C_{1-6}$-Alkyl, $C_{2-6}$-Alkenyl oder Phenyl-$C_{1-6}$-alkyl ist;

X O oder eine Gruppe der Formel

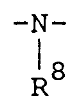

ist (worin $R^8$ Wasserstoff, gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist);

Y O oder eine Gruppe der Formel

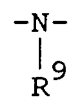

ist (worin $R^9$ Wasserstoff, gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist) und $R^8$ und $R^9$ oder $R^4$ und $R^9$ $C_{1-4}$-Alkenylen oder Alkylen bilden können, wobei jede der genannten Gruppen unsubstituiert oder mit einer oder zwei Oxo-Gruppen substituiert ist;

Z ein Stickstoff enthaltender heterocyclischer Ring ist, der durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert sein kann, wobei $R^3$ an eine Position gebunden ist, die nicht diejenige des Stickstoff-Atoms in dem genannten Stickstoff enthaltenden heterocyclischen Ring ist;

mit der Maßgabe, daß dann, wenn X O ist, Y eine Gruppe der Formel

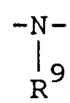

ist, worin $R^9$ die oben angegebenen Bedeutungen hat] oder eines pharmazeutisch annehmbaren Salzes derselben, umfassend

a) die Umsetzung einer Verbindung der Formel

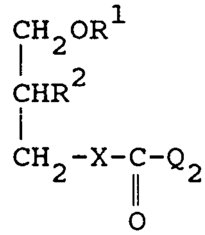

[worin $Q_2$ eine Gruppe ist, die eine Carbonyl-Gruppe aktiviert] mit einer Verbindung der Formel

$HY-R^3-Z-R^4$

[worin alle Symbole die oben angegebenen Bedeutungen haben], oder

b) die Umsetzung einer Verbindung der Formel

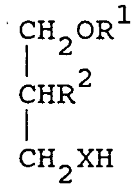

[worin alle Symbole die oben angegebenen Bedeutungen haben] mit einer Verbindung der Formel

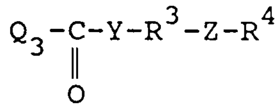

[worin $Q_3$ eine Gruppe ist, die eine Carbonyl-Gruppe aktiviert und die anderen Symbole die oben angegebenen Bedeutungen haben], oder
c) die Umsetzung einer Verbindung der Formel

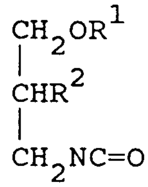

[worin alle Symbole die oben angegebenen Bedeutungen haben] mit einer Verbindung der Formel

$HY\text{-}R^3\text{-}Z\text{-}R^4$,

[worin alle Symbole die oben angegebenen Bedeutungen haben], zur Bereitstellung einer Verbindung der Formel (I), worin X eine Imino-Gruppe ist, oder
d) die Umsetzung einer Verbindung der Formel

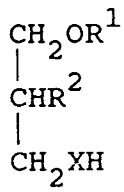

[worin alle Symbole die oben angegebenen Bedeutungen haben] mit einer Verbindung der Formel

$O = C = N\text{-}R^3\text{-}Z\text{-}R^4$

[worin alle Symbole die oben angegebenen Bedeutungen haben] zur Bereitstellung einer Verbindung der Formel (I), worin Y eine Imino-Gruppe ist,
e) die Durchführung einer katalytischen Reduktion mit einer Verbindung der Formel (I), in der $R^2$ Benzyloxy ist, um eine Verbindung der Formel (I) bereitzustellen, in der $R^2$ Hydroxy ist, oder
f) die Durchführung einer Reaktion der Acylierung oder Carbamoylierung mit einer Verbindung der Formel (I), in der $R^2$ Hydroxy ist, um eine Verbindung der Formel (I) bereitzustellen, in der $R^2$ Acyloxy oder eine Gruppe der Formel

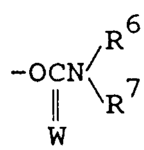

ist [worin alle Symbole die oben angegebenen Bedeutungen haben], oder
g) die Umsetzung einer Verbindung der Formel (I), in der X und/oder Y eine unsubstituierte Imino-Gruppe ist, mit einem Säureanhydrid, Säurehalogenid, Alkylhalogenid oder Alkylisocyanat zur Bereitstellung einer Verbindung der Formel (I), worin X eine Gruppe der Formel

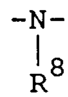

ist (worin $R^8$ gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist) und/oder Y eine Gruppe der Formel

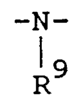

ist (worin $R^9$ gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist), oder
h) die Umsetzung einer Verbindung der Formel (I), worin ein Stickstoff-Atom in der durch Z dargestellten Gruppe primäres, sekundäres oder tertiäres Amino ist, mit einem Alkylhalogenid zur Bereitstellung einer Verbindung der Formel (I), worin ein Stickstoff-Atom in der durch Z dargestellten Gruppe sekundäres, tertiäres oder quaternäres Amino ist, oder
i) die Umsetzung einer Verbindung der Formel (I), worin $R^1$ Wasserstoff ist, mit Alkylisocyanat zur Bereitstellung einer Verbindung der Formel (I), worin $R^1$ eine Gruppe der Formel

$R^5NHCO-$

ist (worin $R^5$ $C_{10-30}$-Alkyl ist)
und gewünschtenfalls
j) die Umwandlung der so erhaltenen Verbindung der Formel (I) in ein Salz derselben.

**Patentansprüche für folgenden Vertragsstaat : AT**

**1.** Verfahren zur Herstellung einer Verbindung der Formel

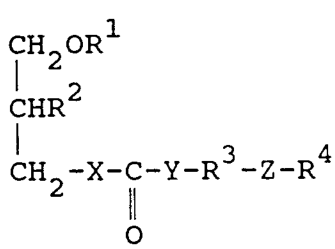

(I) ,

[worin
$R^1$ $C_{10-30}$-Alkyl oder eine Gruppe der Formel $R^5NHCO-$ ist, (worin $R^5$ $C_{10-30}$-Alkyl ist);
$R^2$ Wasserstoff, Hydroxy, $C_{1-5}$-Alkoxy, Phenyl-$C_{1-5}$-alkoxy, $C_{1-5}$-Alkanoyloxy, Benzyloxy, Phenoxycarbonyloxy, $C_{1-5}$-Alkoxycarbonyloxy, eine Gruppe der Formel

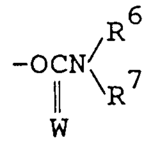

(worin W Sauerstoff oder Schwefel ist und $R^6$ und $R^7$ unabhängig Wasserstoff oder $C_{1-5}$-Alkyl sind oder beide, mit dem benachbarten Stickstoff-Atom zusammengenommen, einen 3- bis 7-gliedrigen Hetero-Ring bilden), Amino, $C_{1-5}$-Alkanoylamino, Benzoylamino, 3- bis 7-gliedriges monocyclisches Amino oder $C_{8-9}$-anelliertes cyclisches Amino ist, wobei die genannten 3- bis 7-gliedrigen monocyclischen Amino-Gruppen oder $C_{8-9}$-anellierten cyclischen Amino-Gruppen unsubstituiert oder mit einer oder zwei Oxo-Gruppen substituiert sind;

$R^3$ eine chemische Bindung oder ein $C_{1-8}$-Alkylen ist, das unsubstituiert oder mit $C_{1-4}$-Alkoxycarbonyl oder Carboxylato substituiert ist;

$R^4$ Wasserstoff, $C_{1-6}$-Alkyl, $C_{2-6}$-Alkenyl oder Phenyl-$C_{1-6}$-alkyl ist;

X O oder eine Gruppe der Formel

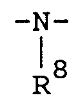

ist (worin $R^8$ Wasserstoff, gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist);

Y O oder eine Gruppe der Formel

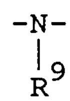

ist (worin $R^9$ Wasserstoff, gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist) und $R^8$ und $R^9$ oder $R^4$ und $R^9$ $C_{1-4}$-Alkenylen oder Alkylen bilden können, wobei jede der genannten Gruppen unsubstituiert oder mit einer oder zwei Oxo-Gruppen substituiert ist;

Z ein Stickstoff enthaltender heterocyclischer Ring ist, der durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert sein kann, wobei $R^3$ an eine Position gebunden ist, die nicht diejenige des Stickstoff-Atoms in dem genannten Stickstoff enthaltenden heterocyclischen Ring ist;

mit der Maßgabe, daß dann, wenn X O ist, Y eine Gruppe der Formel

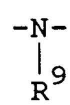

ist, worin $R^9$ die oben angegebenen Bedeutungen hat] oder eines pharmazeutisch annehmbaren Salzes derselben, umfassend

a) die Umsetzung einer Verbindung der Formel

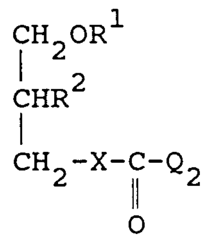

[worin $Q_2$ eine Gruppe ist, die eine Carbonyl-Gruppe aktiviert] mit einer Verbindung der Formel

$HY-R^3-Z-R^4$

[worin alle Symbole die oben angegebenen Bedeutungen haben], oder

b) die Umsetzung einer Verbindung der Formel

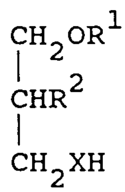

[worin alle Symbole die oben angegebenen Bedeutungen haben] mit einer Verbindung der Formel

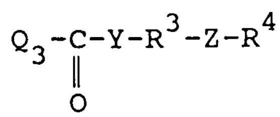

[worin $Q_3$ eine Gruppe ist, die eine Carbonyl-Gruppe aktiviert und die anderen Symbole die oben angegebenen Bedeutungen haben], oder

c) die Umsetzung einer Verbindung der Formel

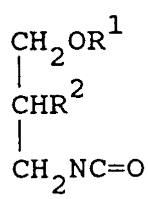

[worin alle Symbole die oben angegebenen Bedeutungen haben] mit einer Verbindung der Formel

$HY-R^3-Z-R^4$

[worin alle Symbole die oben angegebenen Bedeutungen haben], zur Bereitstellung einer Verbindung der Formel (I), worin X eine Imino-Gruppe ist, oder

d) die Umsetzung einer Verbindung der Formel

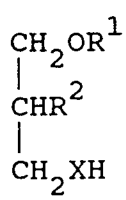

[worin alle Symbole die oben angegebenen Bedeutungen haben] mit einer Verbindung der Formel

$O=C=N\text{-}R^3\text{-}Z\text{-}R^4$

[worin alle Symbole die oben angegebenen Bedeutungen haben] zur Bereitstellung einer Verbindung der Formel (I), worin Y eine Imino-Gruppe ist,
e) die Durchführung einer katalytischen Reduktion mit einer Verbindung der Formel (I), in der $R^2$ Benzyloxy ist, um eine Verbindung der Formel (I) bereitzustellen, in der $R^2$ Hydroxy ist, oder
f) die Durchführung einer Reaktion der Acylierung oder Carbamoylierung mit einer Verbindung der Formel (I), in der $R^2$ Hydroxy ist, um eine Verbindung der Formel (I) bereitzustellen, in der $R^2$ Acyloxy oder eine Gruppe der Formel

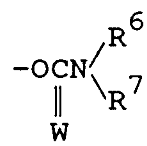

ist [worin alle Symbole die oben angegebenen Bedeutungen haben], oder
g) die Umsetzung einer Verbindung der Formel (I), in der X und/oder Y eine unsubstituierte Imino-Gruppe ist, mit einem Säureanhydrid, Säurehalogenid, Alkylhalogenid oder Alkylisocyanat zur Bereitstellung einer Verbindung der Formel (I), worin X eine Gruppe der Formel

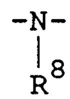

ist (worin $R^8$ gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist) und/oder Y eine Gruppe der Formel

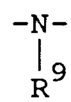

ist (worin $R^9$ gegebenenfalls Carboxy- oder $C_{1-5}$-Alkoxycarbonyl-substituiertes $C_{1-5}$-Alkyl, $C_{1-5}$-Alkanoyl, Benzoyl, Phenoxycarbonyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist), oder
h) die Umsetzung einer Verbindung der Formel (I), worin ein Stickstoff-Atom in der durch Z dargestellten Gruppe primäres, sekundäres oder tertiäres Amino ist, mit einem Alkylhalogenid zur Bereitstellung einer Verbindung der Formel (I), worin ein Stickstoff-Atom in der durch Z dargestellten Gruppe sekundäres, tertiäres oder quaternäres Amino ist, oder
i) die Umsetzung einer Verbindung der Formel (I), worin $R^1$ Wasserstoff ist, mit Alkylisocyanat zur Bereitstellung einer Verbindung der Formel (I), worin $R^1$ eine Gruppe der Formel

$R^5NHCO\text{-}$

ist (worin $R^5$ $C_{10-30}$-Alkyl ist)
und gewünschtenfalls
j) die Umwandlung der so erhaltenen Verbindung der Formel (I) in ein Salz derselben.

**2.** Verfahren nach Anspruch 1, wobei das Produkt eine Verbindung der Formel (I) ist, worin $R^1$ $C_{14-20}$-Alkyl oder $C_{14-20}$-Alkyl-NHCO- ist.

**3.** Verfahren nach Anspruch 1, wobei das Produkt eine Verbindung der Formel (I) ist, worin $R^1$ $C_{14-18}$-

Alkyl oder $C_{14-18}$-Alkyl-NHCO- ist.

4. Verfahren nach Anspruch 1, wobei das Produkt eine Verbindung der Formel (I) ist, worin $R^1$ $C_{15-18}$-Alkyl oder $C_{15-18}$-Alkyl-NHCO- ist.

5. Verfahren nach Anspruch 1, wobei das Produkt eine Verbindung der Formel (I) ist, worin $R^1$ $C_{16-18}$-Alkyl oder $C_{16-18}$-Alkyl-NHCO- ist.

6. Verfahren nach Anspruch 1, wobei das Produkt eine Verbindung der Formel (I) ist, worin $R^1$ Octadecyl oder Octadecylcarbamoyl ist.

7. Verfahren nach Anspruch 1, wobei das Produkt eine Verbindung der Formel (I) ist, worin $R^1$ Octadecylcarbamoyl ist.

8. Verfahren nach Anspruch 1, wobei das Produkt eine Verbindung der Formel (I) ist, worin $R^2$ $C_{1-5}$-Alkoxy, Phenyl-$C_{1-5}$-alkoxy, $C_{1-5}$-Alkanoyloxy, $C_{1-5}$-Alkoxycarbonyloxy, Mono- oder Di-$C_{1-5}$-alkylcarbamoyloxy, $C_{1-5}$-Alkanoylamino, 3- bis 7-gliedriges cyclisches Amino oder Phthalimido ist.

9. Verfahren nach Anspruch 1, wobei das Produkt eine Verbindung der Formel (I) ist, worin $R^2$ $C_{1-5}$-Alkoxy ist.

10. Verfahren nach Anspruch 1, wobei das Produkt eine Verbindung der Formel (I) ist, worin $R^2$ Methoxy ist.

11. Verfahren nach Anspruch 1, wobei das Produkt eine Verbindung der Formel (I) ist, worin $R^3$ $C_{1-8}$-Alkylen ist.

12. Verfahren nach Anspruch 1, wobei das Produkt eine Verbindung der Formel (I) ist, worin $R^3$ Methylen, Ethylen oder Trimethylen ist.

13. Verfahren nach Anspruch 1, wobei das Produkt eine Verbindung der Formel (I) ist, worin $R^3$ Methylen oder Ethylen ist.

14. Verfahren nach Anspruch 1, wobei das Produkt eine Verbindung der Formel (I) ist, worin $R^4$ Wasserstoff oder $C_{1-6}$-Alkyl ist.

15. Verfahren nach Anspruch 1, wobei das Produkt eine Verbindung der Formel (I) ist, worin $R^4$ Wasserstoff, Methyl oder Ethyl ist.

16. Verfahren nach Anspruch 1, wobei das Produkt eine Verbindung der Formel (I) ist, worin X O, Imino oder $C_{1-5}$-Alkanoylimino ist.

17. Verfahren nach Anspruch 1, wobei das Produkt eine Verbindung der Formel (I) ist, worin X O ist.

18. Verfahren nach Anspruch 1, wobei das Produkt eine Verbindung der Formel (I) ist, worin Y O oder eine Gruppe der Formel

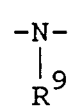

ist, wobei das Produkt eine Verbindung der Formel (I) ist, worin $R^9$ Wasserstoff, $C_{1-5}$-Alkyl, $C_{1-5}$-Alkanoyl, $C_{1-5}$-Alkoxycarbonyl, Carbamoyl, Mono- oder Di-$C_{1-5}$-alkylcarbamoyl oder 3- bis 7-gliedriges cyclisches Aminocarbonyl ist.

19. Verfahren nach Anspruch 1, wobei das Produkt eine Verbindung der Formel (I) ist, worin Y $C_{1-5}$-Alkanoylimino ist.

**20.** Verfahren nach Anspruch 1, wobei das Produkt eine Verbindung der Formel (I) ist, worin Y Acetylimino ist.

**21.** Verfahren nach Anspruch 1, wobei das Produkt eine Verbindung der Formel (I) ist, worin

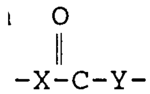

eine Gruppe der Formel

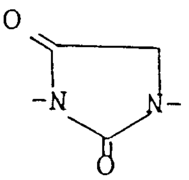

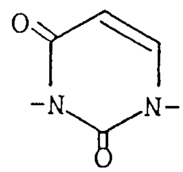

ist.

**22.** Verfahren nach Anspruch 1, wobei das Produkt eine Verbindung der Formel (I) ist, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Azetidinyl-, Pyrrolidinyl-, Piperidinyl-, Perhydroazepinyl-, Pyrrolinyl-, Pyrazolinyl-, Pyrrolyl-, Pyridyl-, Oxazolyl-, Thiazolyl-, Pyridazinyl-, Pyrimidyl-, Pyrazinyl-, Imidazolyl-, Morpholinyl-, Thiomorpholinyl-, Piperazinyl-, Pyrazolidinyl-, Indolyl-, Isoindolyl-, 1H-Indazolyl-, Purinyl-, Chinolinyl-, Isochinolinyl-, Perhydroindolyl- oder PerhydroisochinolylGruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**23.** Verfahren nach Anspruch 1, wobei das Produkt eine Verbindung der Formel (I) ist, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Morpholinyl-, Pyrrolidinyl-, Piperidinyl-, Imidazolyl-, Pyridyl- oder Thiazolyl-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**24.** Verfahren nach Anspruch 1, wobei das Produkt eine Verbindung der Formel (I) ist, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Pyridyl- oder Thiazolyl-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**25.** Verfahren nach Anspruch 1, wobei das Produkt eine Verbindung der Formel (I) ist, worin Z-$R^4$ Pyridyl oder Thiazolyl ist.

**26.** Verfahren nach Anspruch 1, wobei das Produkt eine Verbindung der Formel (I) ist, worin Z-$R^4$ 2-Pyridyl, Thiazol-2-yl oder Thiazol-4-yl ist.

**27.** Verfahren nach Anspruch 1, wobei das Produkt eine Verbindung der Formel (I) ist, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Imidazolio-, Pyridinio-, Oxazolio-, Thiazolio-, Pyridazinio-, Pyrimidinio-, Pyrazinio-, Chinolinio-, Isochinolinio-, Morpholinio-, Piperidinio-, Piperazinio- oder Pyrrolidinio-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**28.** Verfahren nach Anspruch 1, wobei das Produkt eine Verbindung der Formel (I) ist, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Morpholinio-, Pyrrolidinio-, Piperidinio-, Imidazolio-, Pyridinio- oder Thiazolio-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**29.** Verfahren nach Anspruch 1, wobei das Produkt eine Verbindung der Formel (I) ist, worin Z-$R^4$ eine gegebenenfalls durch $C_{1-6}$-Alkyl, $C_{1-4}$-Alkoxycarbonyl-$C_{1-6}$-alkyl, Carboxylato-$C_{1-6}$-alkyl oder Phenyl-$C_{1-6}$-alkyl substituierte Pyridinio- oder Thiazolio-Gruppe ist, die unsubstituiert oder durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert ist.

**30.** Verfahren nach Anspruch 1, wobei das Produkt eine Verbindung der Formel (I) ist, worin Z-$R^4$ 1-($C_{1-6}$-Alkyl)pyridino-2-yl ist.

**31.** Verfahren nach Anspruch 1, wobei das Produkt eine Verbindung der Formel (I) ist, worin Z-$R^4$ 1-Ethylpyridino-2-yl ist.

**32.** Verfahren nach Anspruch 1, wobei das Produkt eine Verbindung der Formel (I) ist, worin Z-$R^4$ 3-($C_{1-6}$-Alkyl)thiazolio-2-yl ist.

**33.** Verfahren nach Anspruch 1, wobei das Produkt eine Verbindung der Formel (I) ist, worin Z-$R^4$ 3-Ethylthiazolio-2-yl ist.

**34.** Verfahren nach Anspruch 1, wobei das Produkt eine Verbindung der Formel (I) ist, worin Z-$R^4$ 3-($C_{1-6}$-Alkyl)thiazolio-4-yl ist.

**35.** Verfahren nach Anspruch 1, wobei das Produkt eine Verbindung der Formel (I) ist, worin Z-$R^4$ 3-Ethylthiazolio-4-yl ist.

**36.** Verfahren nach Anspruch 1 worin das Produkt 3-O-[N-Acetyl-N-(N-methylpyridinio-2-yl)methyl]-carbamoyl-2-O-methyl-1-octadecylcarbamoylglycerin-chlorid ist.

**37.** Verfahren nach Anspruch 1, worin das Produkt 1-Ethyl-2-[N-acetyl-N-(3-octadecylcarbamoyloxy-2-methoxypropyloxy)carbonylamino]methylpyridiniumhalogenid ist.

**38.** Verfahren nach Anspruch 1, worin das Produkt 2-[N-Acetyl-N-(3-octadecylcarbamoyloxy-2-methoxypropyloxy)carbonyl)amino]methyl-3-methylthiazoliumhalogenid ist.

**39.** Verfahren nach Anspruch 1, worin das Produkt 2-[N-Acetyl-N-(3-octadecylcarbamoyloxy-2-methoxypropyloxy)carbonyl)amino]methyl-3-ethylthiazoliumhalogenid ist.

**40.** Verfahren nach Anspruch 1, worin das Produkt 2-[N-(3-octadecylcarbamoyloxy-2-ethoxypropyloxycarbonyl)amino]methyl-N-pyridiniumhalogenid ist.

**41.** Verfahren nach Anspruch 1, worin das Produkt 4-[N-Acetyl-N-(3-octadecylcarbamoyloxy-2-methoxypropyloxycarbonyl)amino]methyl-3-ethylthiazoliumhalogenid ist.

**42.** Verfahren nach Anspruch 37, worin das Halogenid Chlorid ist.

**43.** Verfahren nach Anspruch 38, worin das Halogenid Iodid ist.

**44.** Verfahren nach Anspruch 39, worin das Halogenid Iodid ist.

**45.** Verfahren nach Anspruch 40, worin das Halogenid Iodid ist.

**46.** Verfahren nach Anspruch 41, worin das Halogenid Iodid ist.

**47.** Verfahren nach Anspruch 1, wobei das Produkt eine Verbindung der Formel (I) ist, worin $R^3$ $C_{1-8}$-Alkylen ist und Z ein Stickstoff enthaltender heterocyclischer Ring ist, der durch gegebenenfalls Hydroxy- oder Amino-substituiertes $C_{1-4}$-Alkyl, Hydroxy, Amino, Imino, Mono- oder Di-$C_{1-4}$-alkylamino, Carbamoyl, Ureido, Carboxy, Carboxylato oder $C_{1-4}$-Alkoxycarbonyl substituiert sein kann.

## Revendications

### Revendications pour les Etats contractants suivants : BE, CH, DE, FR, GB, IT, LI, LU, NL, SE

**1.** Composé de formule:

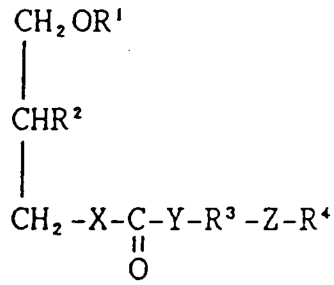

(I)

dans laquelle $R^1$ est un alcoyle en $C_{10-30}$ ou un groupe de formule:

$R^5NHCO-$

dans laquelle $R^5$ est un alcoyle en $C_{10-30}$; $R^2$ est l'hydrogène ou un groupe hydroxy, alcoxy en $C_{1-5}$, phénylalcoxy en $C_{1-5}$, alcanoyloxy en $C_{1-5}$, benzyloxy, phénoxycarbonyloxy, alcoxy en $C_{1-5}$-carbonyloxy, un groupe de formule:

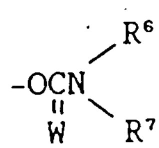

(dans laquelle W est l'oxygène ou le soufre et $R^6$ et $R^7$, indépendamment l'un de l'autre, représentent l'hydrogène ou un alcoyle en $C_{1-5}$ ou l'un et l'autre, pris ensemble avec l'atome d'azote adjacent, forment un noyau hétérocyclique à 3-7 chaînons), un groupe amino, alcanoylamino en $C_{1-5}$, benzoylamino, amino monocyclique à 3-7 chaînons ou amino cyclique condensé en $C_{8-9}$; ledit groupe amino monocyclique à 3-7 chaînons ou amino cyclique condensé en $C_{8-9}$ étant non substitué ou substitué par un ou deux groupes oxo; $R^3$ est une liaison chimique ou un alcoylène en $C_{1-8}$ non substitué ou substitué par un alcoxy en $C_{1-4}$-carbonyle ou un carboxylate; $R^4$ est l'hydrogène ou un groupe alcoyle en $C_{1-6}$, alcényle en $C_{2-6}$ ou phényl-alcoyle en $C_{1-6}$; X est O ou un groupe de formule:

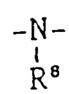

dans laquelle $R^8$ est l'hydrogène ou un groupe alcoyle en $C_{1-5}$ éventuellement substitué par un carboxyle ou un alcoxy en $C_{1-5}$-carbonyle, alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons; Y est O ou un groupe de formule:

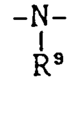

dans laquelle $R^9$ est l'hydrogène ou un groupe alcoyle en $C_{1-5}$, éventuellement substitué par un carboxyle ou un alcoxy en $C_{1-5}$-carbonyle, alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, et $R^8$ et $R^9$ ou $R^4$ et $R^9$ peuvent former un alcénylène ou alcoylène en $C_{1-4}$, chacun desdits groupes étant non substitué ou substitué par un ou deux groupes oxo; Z est un noyau hétérocyclique contenant de l'azote qui peut être substitué par un alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle, $R^3$ étant lié à une position autre que l'atome d'azote dans ledit noyau hétérocyclique contenant de l'azote; avec la condition que Y est un groupe de formule:

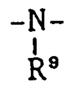

lorsque X est O, formule dans laquelle $R^9$ est tel que défini ci-dessus, ou un sel pharmaceutiquement acceptable de ce composé.

2. Composé selon la revendication 1, dans lequel $R^1$ est un alcoyle en $C_{14-20}$ ou un alcoyle en $C_{14-20}$-NHCO-.

3. Composé selon la revendication 1, dans lequel $R^1$ est un alcoyle en $C_{14-18}$ ou un alcoyle en $C_{14-18}$-NHCO-.

4. Composé selon la revendication 1, dans lequel $R^1$ est un alcoyle en $C_{15-18}$ ou un alcoyle en $C_{15-18}$-NHCO-.

5. Composé selon la revendication 1, dans lequel $R^1$ est un alcoyle en $C_{16-18}$ ou un alcoyle en $C_{16-18}$-NHCO-.

6. Composé selon la revendication 1, dans lequel $R^1$ est l'octadécyle ou l'octacécylcarbamoyle.

7. Composé selon la revendication 1, dans lequel $R^1$ est l'octadécylcarbamoyle.

8. Composé selon la revendication 1, dans lequel $R^2$ est un groupe alcoxy en $C_{1-5}$, phényl-alcoxy en $C_{1-5}$, alcanoyloxy en $C_{1-5}$, alcoxy en $C_{1-5}$-carbonyloxy, mono- ou di-(alcoyle en $C_{1-5}$)carbamoyloxy, alcanoylamino en $C_{1-5}$, aminocyclique à 3-7 chaînons ou phtalimido.

9. Composé selon la revendication 1, dans lequel $R^2$ est un alcoxy en $C_{1-5}$.

10. Composé selon la revendication 1, dans lequel $R^2$ est le méthoxy.

11. Composé selon la revendication 1, dans lequel $R^3$ est un alcoylène en $C_{1-8}$.

12. Composé selon la revendication 1, dans lequel $R^3$ est le méthylène, l'éthylène ou le triméthylène.

13. Composé selon la revendication 1, dans lequel $R^3$ est le méthylène ou l'éthylène.

14. Composé selon la revendication 1, dans lequel $R^4$ et l'hydrogène ou un alcoyle en $C_{1-6}$.

15. Composé selon la revendication 1, dans lequel $R^4$ est l'hydrogène, le méthyle ou l'éthyle.

16. Composé selon la revendication 1, dans lequel X est O ou un imino ou alcanoylimino en $C_{1-5}$.

17. Composé selon la revendication 1, dans lequel X est O.

18. Composé selon la revendication 1, dans lequel Y est O ou un groupe de formule:

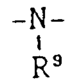

dans laquelle $R^9$ est l'hydrogène ou un groupe alcoyle en $C_{1-5}$, alcanoyle en $C_{1-5}$, alcoxy en $C_{1-5}$-carbonyle, carbamoyle, mono- ou di-(alcoyle en $C_{1-5}$)carbamoyle ou aminocarbonyle cyclique à 3-7 chaînons.

**19.** Composé selon la revendication 1, dans lequel Y est un alcanoylimino en $C_{1-5}$.

**20.** Composé selon la revendication 1, dans lequel Y est l'acétylimino.

**21.** Composé selon la revendication 1, dans lequel

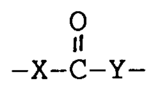

est un groupe de formule:

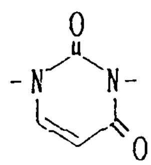

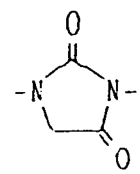

**22.** Composé selon la revendication 1, dans lequel Z-$R^4$ est un groupe azétidinyle, pyrrolidinyle, pipéridinyle, perhydroazépinyle, pyrrolinyle, pyrazolinyle, pyrrolyle, pyridyle, oxazolyle, thiazolyle, pyridazinyle, pyrimidyle, pyrazinyle, imidazolyle, morpholinyle, thiomorpholinyle, pipérazinyle, pyrazolidinyle, indolyle, isoindolyle, 1H-indazolyle, purinyle, quinoléinyle, isoquinoléinyle, perhydroindolyle ou perhydroisoquinoléinyle, éventuellement substitué par un groupe alcoyle en $C_{1-6}$, alcoxy en $C_{1-4}$-carbonyle-alcoyle en $C_{1-6}$, carboxylate-alcoyle en $C_{1-6}$ ou phénylalcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)-amino, carbamoyle, uréido, carboxylique, carboxylate ou alcoxy en $C_{1-4}$-carbonyle.

**23.** Composé selon la revendication 1, dans lequel Z-$R^4$ est un groupe morpholinyle, pyrrolidinyle, pipéridinyle, imidazolyle, pyridyle ou thiazolyle éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**24.** Composé selon la revendication 1, dans lequel Z-$R^4$ est un groupe pyridyle ou thiazolyle éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**25.** Composé selon la revendication 1, dans lequel Z-$R^4$ est un groupe pyridyle ou thiazolyle.

**26.** Composé selon la revendication 1, dans lequel Z-$R^4$ est un groupe 2-pyridyle, thiazol-2-yle ou thiazol-4-yle.

**27.** Composé selon la revendication 1, dans lequel Z-$R^4$ est un groupe imidazolio, pyridinio, oxazolio, thiazolio, pyridazinio, pyrimidinio, pyrazinio, quinoléinio, isoquinoléinio, morpholinio, pipéridinio, pipérazinio ou pyrrolidinio éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$) amino, carbamoyle, uréido, carboxylique, carboxylate ou alcoxy en $C_{1-4}$-carbonyle.

**28.** Composé selon la revendication 1, dans lequel Z-$R^4$ est un groupe morpholinio, pyrrolidinio, pipéridinio, imidazolio, pyridinio ou thiazolio éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**29.** Composé selon la revendication 1, dans lequel Z-$R^4$ est un groupe pyridinio ou thiazolio éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**30.** Composé selon la revendication 1, dans lequel Z-$R^4$ est le groupe 1-(alcoyle en $C_{1-6}$)pyridinio-2-yle.

**31.** Composé selon la revendication 1, dans lequel Z-$R^4$ est le groupe 1-éthylpyridinio-2-yle.

**32.** Composé selon la revendication 1, dans lequel Z-$R^4$ est un groupe 3-(alcoyle en $C_{1-6}$)thiazolio-2-yle.

**33.** Composé selon la revendication 1, dans lequel Z-$R^4$ est le groupe 3-éthylthiazolio-2-yle.

**34.** Composé selon la revendication 1, dans lequel Z-$R^4$ est un groupe 3-(alcoyle en $C_{1-6}$)thiazolio-4-yle.

**35.** Composé selon la revendication 1, dans lequel Z-$R^4$ est le groupe 3-éthylthiazolio-4-yle.

**36.** Composé selon la revendication 1, qui est le chlorure de 3-O-[N-acétyl-N-(N-méthylpyridinio-2-yl)-méthyl]carbamoyl-2-O-méthyl-1-octadécylcarbamoylglycérol.

**37.** Composé selon la revendication 1, qui est un halogénure de 1-éthyl-2-[N-acétyl-N-(3-octadécylcarbamoyloxy-2-méthoxypropyloxy)carbonylamino]méthylpyridinium.

**38.** Composé selon la revendication 1, qui est un halogénure de 2-[N-acétyl-N-(3-octadécylcarbamoyloxy-2-méthoxypropoxycarbonyl)amino]méthyl-3-méthylthiazolium.

**39.** Composé selon la revendication 1, qui est un halogénure de 2-[N-acétyl-N-(3-octadécylcarbamoyloxy-2-méthoxypropoxycarbonyl)amino]méthyl-3-éthylthiazolium.

**40.** Composé selon la revendication 1, qui est un halogénure de 2-[N-(3-octadécylcarbamoyloxy-2-éthoxy-propyloxycarbonyl)amino]méthyl-N-éthylpyridinium.

**41.** Composé selon la revendication 1, qui est un halogénure de 4-[N-acétyl-N-(3-octadécylcarbamoyloxy-2-méthoxypropoxycarbonyl)amino]méthyl-3-éthylthiazolium.

**42.** Composé selon la revendication 37, dans lequel l'halogénure est le chlorure.

**43.** Composé selon la revendication 38, dans lequel l'halogénure est l'iodure.

**44.** Composé selon la revendication 39, dans lequel l'halogénure est l'iodure.

**45.** Composé selon la revendication 40, dans lequel l'halogénure est l'iodure.

**46.** Composé selon la revendication 41, dans lequel l'halogénure est l'iodure.

**47.** Composé selon la revendication 1, dans lequel $R^3$ est un alcoylène en $C_{1-8}$ et Z est un noyau hétérocyclique contenant de l'azote qui peut être substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**48.** Composition pharmaceutique qui comprend, comme substance active, une quantité efficace d'un composé tel que revendiqué dans l'une quelconque des revendications 1 à 47, en association avec une matière de support, un excipient ou diluant pharmaceutiquement acceptables.

**49.** Composé selon l'une quelconque des revendications 1 à 47 ou composition pharmaceutique selon la revendication 48, pour son utilisation à la préparation d'un agent prophylactique ou curatif de différentes maladies de la circulation ou troubles allergiques ou d'un agent antinéoplastique.

**50.** Procédé de préparation d'un composé de formule:

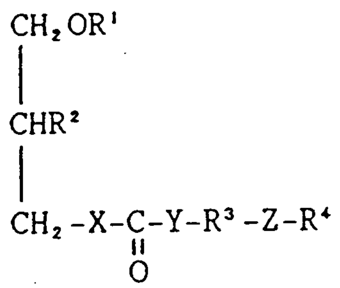

(I)

dans laquelle $R^1$ est un alcoyle en $C_{10-30}$ ou un groupe de formule:

$R^5NHCO-$

dans laquelle $R^5$ est un alcoyle en $C_{10-30}$; $R^2$ est l'hydrogène ou un groupe hydroxy, alcoxy en $C_{1-5}$, phénylalcoxy en $C_{1-5}$, alcanoyloxy en $C_{1-5}$, benzyloxy, phénoxycarbonyloxy, alcoxy en $C_{1-5}$-carbonyloxy, un groupe de formule:

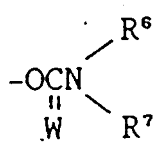

(dans laquelle W est l'oxygène ou le soufre et $R^6$ et $R^7$, indépendamment l'un de l'autre, représentent l'hydrogène ou un alcoyle en $C_{1-5}$ ou l'un et l'autre pris ensemble avec l'atome d'azote adjacent, forment un noyau hétérocyclique à 3-7 chaînons), un groupe amino, alcanoylamino en $C_{1-5}$, benzoylamino, amino monocyclique à 3-7 chaînons ou amino cyclique condensé en $C_{8-9}$; ledit groupe amino monocyclique à 3-7 chaînons ou amino cyclique condensé en $C_{8-9}$ étant non substitué ou substitué par un ou deux groupes oxo; $R^3$ est une liaison chimique ou un alcoylène en $C_{1-8}$ non substitué ou substitué par un alcoxy en $C_{1-4}$-carbonyle ou un carboxylate; $R^4$ est l'hydrogène ou un groupe alcoyle en $C_{1-6}$, alcényle en $C_{2-6}$ ou phényl-alcoyle en $C_{1-6}$; X est O ou un groupe de formule:

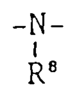

dans laquelle $R^8$ est l'hydrogène ou un groupe alcoyle en $C_{1-5}$ éventuellement substitue par un carboxyle ou un alcoxy en $C_{1-5}$-carbonyle, alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons; Y est O ou un groupe de formule:

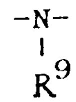

dans laquelle $R^9$ est l'hydrogène ou un groupe alcoyle en $C_{1-5}$ éventuellement substitué par un carboxyle ou un alcoxy en $C_{1-5}$-carbonyle, alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, et $R^8$ et $R^9$ ou $R^4$ et $R^9$ peuvent former un alcénylène ou alcoylène en $C_{1-4}$, chacun desdits groupes étant non substitué ou substitué par un ou deux groupes oxo; Z est un noyau hétérocyclique contenant de l'azote qui peut être substitué par un alcoyle en $C_{1-4}$ éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle, $R^3$ étant lié à une position autre que l'atome d'azote dans ledit noyau hétérocyclique contenant de l'azote; avec la condition que Y est un groupe de formule:

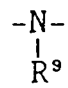

lorsque X est O, formule dans laquelle $R^9$ est tel que défini ci-dessus, ou d'un sel pharmaceutiquement acceptable de celui-ci, selon lequel

a) on fait réagir un composé de formule:

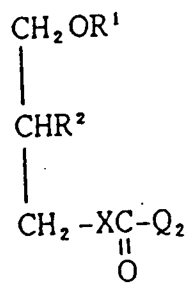

dans laquelle $Q_2$ est un groupe qui active un groupe carbonyle, avec un composé de formule:

$HY\text{-}R^3\text{-}Z\text{-}R^4$

dans laquelle tous les symboles sont tels que défini ci-dessus, ou

b) on fait réagir un composé de formule:

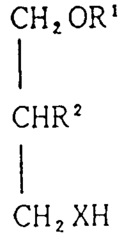

dans laquelle tous les symboles sont tels que défini ci-dessus, avec un composé de formule:

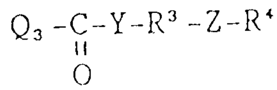

dans laquelle $Q_3$ est un groupe qui active le groupe carbonyle et les autres symboles sont tels que défini ci-dessus, ou

c) on fait réagir un composé de formule:

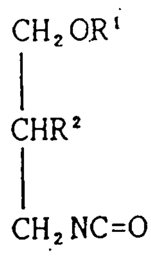

dans laquelle tous les symboles sont tels que défini ci-dessus, avec un composé de formule:

$HY-R^3-Z-R^4$

dans laquelle tous les symboles sont tels que défini ci-dessus, pour former un composé de formule (I), dans laquelle X est un groupe imino, ou

d) on fait réagir un composé de formule:

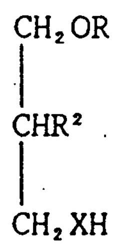

dans laquelle tous les symboles sont tels que défini ci-dessus, avec un composé de formule:

$O=C=N-R^3-Z-R^4$

dans laquelle tous les symboles sont tels que défini ci-dessus, pour former un composé de formule (I), dans laquelle Y est un groupe imino,

e) on soumet un composé de formule (I), dans laquelle $R^2$ est un groupe benzyloxy, à une réduction catalytique pour former un composé de formule (I), dans laquelle $R^2$ est l'hydroxy, ou

f) on soumet un composé de formule (I), dans laquelle $R^2$ est l'hydroxy, à une réaction d'acylation ou de carbamoylation, pour former un composé de formule (I), dans laquelle $R^2$ est un acyloxy ou un groupe représenté par la formule:

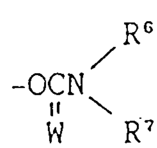

dans laquelle tous les symboles sont tels que défini ci-dessus, ou

g) on fait réagir un composé de formule (I), dans laquelle X et/ou Y est un groupe imino non substitué, avec un anhydride d'acide, un halogénure d'acide, un halogénure d'alcoyle ou un isocyanate d'alcoyle, pour former un composé de formule (I), dans laquelle X est un groupe de

formule:

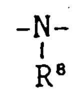

dans laquelle $R^8$ est un groupe alcoyle en $C_{1-5}$, éventuellement substitué par un carboxyle ou un alcoxy en $C_{1-5}$-carbonyle, ou un groupe alcanoyle en $C_{1-5}$, benzyole, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, et/ou Y est un groupe de formule:

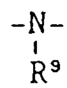

dans laquelle $R^9$ est un groupe alcoyle en $C_{1-5}$, éventuellement substitué par un carboxyle ou un alcoxy en $C_{1-5}$-carbonyle, ou un groupe alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, ou

h) on fait réagir un composé de formule (I), dans laquelle l'atome d'azote dans le groupe représenté par Z est un groupe amino primaire, secondaire ou tertiaire, avec un halogénure d'alcoyle, pour former un composé de formule (I), dans laquelle l'atome d'azote dans le groupe représenté par Z est un groupe amino secondaire, tertiaire ou quaternaire, ou

i) on fait réagir un composé de formule (I), dans laquelle $R^1$ est l'hydrogène, avec un isocyanate d'alcoyle, pour former un composé de formule (I), dans laquelle $R^1$ est un groupe de formule:

$R^5NHCO-$

dans laquelle $R^5$ est un alcoyle en $C_{10-30}$, et si désiré,

j) on transforme le composé ainsi obtenu, de formule (I), en un sel de celui-ci.

**Revendications pour l'Etat contractant suivant : AT**

**1.** Procédé de préparation d'un composé de formule:

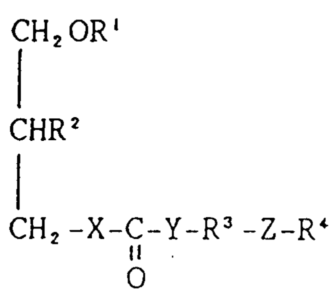

(I)

dans laquelle $R^1$ est un alcoyle en $C_{10-30}$ ou un groupe de formule:

$R^5NHCO-$

dans laquelle $R^5$ est un alcoyle en $C_{10-30}$; $R^2$ est l'hydrogène ou un groupe hydroxy, alcoxy en $C_{1-5}$, phénylalcoxy en $C_{1-5}$, alcanoyloxy en $C_{1-5}$, benzyloxy, phénoxycarbonyloxy, alcoxy en $C_{1-5}$-carbonyloxy, un groupe de formule:

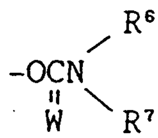

(dans laquelle W est l'oxygène ou le soufre et $R^6$ et $R^7$, indépendamment l'un de l'autre, représentent l'hydrogène ou un alcoyle en $C_{1-5}$ ou l'un et l'autre pris ensemble avec l'atome d'azote adjacent, forment un noyau hétérocyclique à 3-7 chaînons), un groupe amino, alcanoylamino en $C_{1-5}$, benzoylamino, amino monocyclique à 3-7 chaînons ou amino cyclique condensé en $C_{8-9}$; ledit groupe amino monocyclique à 3-7 chaînons ou amino cyclique condensé en $C_{8-9}$ étant non substitué ou substitué par un ou deux groupes oxo; $R^3$ est une liaison chimique ou un alcoylène en $C_{1-8}$ non substitué ou substitué par un alcoxy en $C_{1-4}$-carbonyle ou un carboxylate; $R^4$ est l'hydrogène ou un groupe alcoyle en $C_{1-6}$, alcényle en $C_{2-6}$ ou phényl-alcoyle en $C_{1-6}$; X est O ou un groupe de formule:

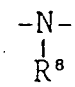

dans laquelle $R^8$ est l'hydrogène ou un groupe alcoyle en $C_{1-5}$ éventuellement substitué par un carboxyle ou un alcoxy en $C_{1-5}$-carbonyle, alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons; Y est O ou un groupe de formule:

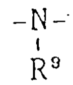

dans laquelle $R^9$ est l'hydrogène ou un groupe alcoyle en $C_{1-5}$ éventuellement substitué par un carboxyle ou un alcoxy en $C_{1-5}$-carbonyle, alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, et $R^8$ et $R^9$ ou $R^4$ et $R^9$ peuvent former un alcénylène ou alcoylène en $C_{1-4}$, chacun desdits groupes étant non substitué ou substitué par un ou deux groupes oxo; Z est un noyau hétérocyclique contenant de l'azote qui peut être substitué par un alcoyle en $C_{1-4}$ éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle, $R^3$ étant lié à une position autre que l'atome d'azote dans ledit noyau hétérocyclique contenant de l'azote; avec la condition que Y est un groupe de formule:

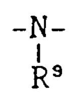

lorsque X est O, formule dans laquelle $R^9$ est tel que défini ci-dessus, ou d'un sel pharmaceutiquement acceptable de celui-ci, selon lequel

a) on fait réagir un composé de formule:

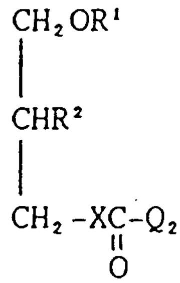

dans laquelle $Q_2$ est un groupe qui active un groupe carbonyle, avec un composé de formule:

$HY-R^3-Z-R^4$

dans laquelle tous les symboles sont tels que défini ci-dessus, ou
b) on fait réagir un composé de formule:

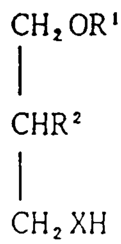

dans laquelle tous les symboles sont tels que défini ci-dessus, avec un composé de formule:

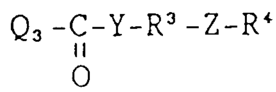

dans laquelle $Q_3$ est un groupe qui active le groupe carbonyle et les autres symboles sont tels que défini ci-dessus, ou
c) on fait réagir un composé de formule:

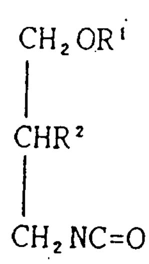

dans laquelle tous les symboles sont tels que défini ci-dessus, avec un composé de formule:

$HY-R^3-Z-R^4$

dans laquelle tous les symboles sont tels que défini ci-dessus, pour former un composé de formule (I), dans laquelle X est un groupe imino, ou
d) on fait réagir un composé de formule:

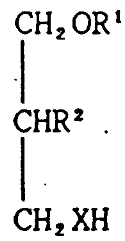

dans laquelle tous les symboles sont tels que défini ci-dessus, avec un composé de formule:

$O=C=N\text{-}R^3\text{-}Z\text{-}R^4$

dans laquelle tous les symboles sont tels que défini ci-dessus, pour former un composé de formule (I), dans laquelle Y est un groupe imino,
e) on soumet un composé de formule (I), dans laquelle $R^2$ est un groupe benzyloxy, à une réduction catalytique pour former un composé de formule (I), dans laquelle $R^2$ est l'hydroxy, ou
f) on soumet un composé de formule (I), dans laquelle $R^2$ est l'hydroxy, à une réaction d'acylation ou de carbamoylation, pour former un composé de formule (I), dans laquelle $R^2$ est un acyloxy ou un groupe représenté par la formule:

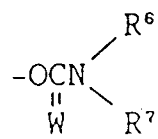

dans laquelle tous les symboles sont tels que défini ci-dessus, ou
g) on fait réagir un composé de formule (I), dans laquelle X et/ou Y est un groupe imino non substitué, avec un anhydride d'acide, un halogénure d'acide, un halogénure d'alcoyle ou un isocyanate d'alcoyle, pour former un composé de formule (I), dans laquelle X est un groupe de formule:

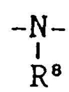

dans laquelle $R^8$ est un groupe alcoyle en $C_{1-5}$, éventuellement substitué par un carboxyle ou un alcoxy en $C_{1-5}$-carbonyle, ou un groupe alcanoyle en $C_{1-5}$, benzyole, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, et/ou Y est un groupe de formule:

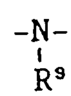

dans laquelle $R^9$ est un groupe alcoyle en $C_{1-5}$, éventuellement substitué par un carboxyle ou un alcoxy en $C_{1-5}$-carbonyle, ou un groupe alcanoyle en $C_{1-5}$, benzoyle, phénoxycarbonyle, alcoxy en $C_{1-5}$-carbonyle, carbamoyle, mono- ou di-(alcoyle en $C_{1-5}$)carbamoyle ou aminocarbonyle cyclique à 3-7 chaînons, ou
h) on fiat réagir un composé de formule (I), dans laquelle l'atome d'azote dans le groupe représenté par Z est un groupe amino primaire, secondaire ou tertiaire, avec un halogénure d'alcoyle, pour former un composé de formule (I), dans laquelle l'atome d'azote dans le groupe représenté par Z est un groupe amino secondaire, tertiaire ou quaternaire, ou
i) on fait réagir un composé de formule (I), dans laquelle $R^1$ est l'hydrogène, avec un isocyanate d'alcoyle, pour former un composé de formule (I), dans laquelle $R^1$ est un groupe de formule:

$R^5NHCO-$

dans laquelle $R^5$ est un alcoyle en $C_{10-30}$, et si désiré,
j) on transforme le composé ainsi obtenu, de formule (I), en un sel de celui-ci.

**2.** Procédé selon la revendication 1, dans lequel le produit est un composé de formule (I), dans laquelle $R^1$ est un groupe alcoyle en $C_{14-20}$ ou alcoyle en $C_{14-20}$-NHCO-.

**3.** Procédé selon la revendication 1, dans lequel le produit est un composé de formule (I), dans laquelle $R^1$ est un groupe alcoyle en $C_{14-18}$ ou alcoyle en $C_{14-18}$-NHCO-.

**4.** Procédé selon la revendication 1, dans lequel le produit est un composé de formule (I), dans laquelle $R^1$ est un alcoyle en $C_{15-18}$ ou un alcoyle en $C_{15-18}$-NHCO-.

**5.** Procédé selon la revendication 1, dans lequel le produit est un composé de formule (I), dans laquelle $R^1$ est un alcoyle en $C_{16-18}$ ou un alcoyle en $C_{16-18}$-NHCO-.

**6.** Procédé selon la revendication 1, dans lequel le produit est un composé de formule (I), dans laquelle $R^1$ est l'octadécyle ou l'octacécylcarbamoyle.

**7.** Procédé selon la revendication 1, dans lequel le produit est un composé de formule (I), dans laquelle $R^1$ est l'octadécylcarbamoyle.

**8.** Procédé selon la revendication 1, dans lequel le produit est un composé de formule (I), dans laquelle $R^2$ est un groupe alcoxy en $C_{1-5}$, phényl-alcoxy en $C_{1-5}$, alcanoyloxy en $C_{1-5}$, alcoxy en $C_{1-5}$-carbonyloxy, mono- ou di-(alcoyle en $C_{1-5}$)carbamoyloxy, alcanoylamino en $C_{1-5}$, aminocyclique à 3-7 chaînons ou phtalimido.

**9.** Procédé selon la revendication 1, dans lequel le produit est un composé de formule (I), dans laquelle $R^2$ est un alcoxy en $C_{1-5}$.

**10.** Procédé selon la revendication 1, dans lequel le produit est un composé de formule (I), dans laquelle $R^2$ est le méthoxy.

**11.** Procédé selon la revendication 1, dans lequel le produit est un composé de formule (I), dans laquelle $R^3$ est un alcoylène en $C_{1-8}$.

**12.** Procédé selon la revendication 1, dans lequel le duit est un composé de formule (I), dans laquelle $R^3$ est le méthylène, l'éthylène ou le triméthylène.

**13.** Procédé selon la revendication 1, dans lequel le produit est un composé de formule (I), dans laquelle $R^3$ est le méthylène ou l'éthylène.

**14.** Procédé selon la revendication 1, dans lequel le produit est un composé de formule (I), dans laquelle $R^4$ est l'hydrogène ou un alcoyle en $C_{1-6}$.

**15.** Procédé selon la revendication 1, dans lequel le produit est un composé de formule (I), dans laquelle $R^4$ est l'hydrogène, le méthyle ou l'éthyle.

**16.** Procédé selon la revendication 1, dans lequel le produit est un composé de formule (I), dans laquelle X est O ou un imino ou alcanoylimino en $C_{1-5}$.

**17.** Procédé selon la revendication 1, dans lequel le produit est un composé de formule (I), dans laquelle X est O.

**18.** Procédé selon la revendication 1, dans lequel le produit est un composé de formule (I), dans laquelle Y est O ou un groupe de formule:

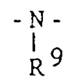

dans laquelle $R^9$ est l'hydrogène ou un groupe alcoyle en $C_{1-5}$, alcanoyle en $C_{1-5}$, alcoxy en $C_{1-5}$-carbonyle, carbamoyle, mono- ou di-(alcoyle en $C_{1-5}$)carbamoyle ou aminocarbonyle cyclique à 3-7 chaînons.

**19.** Procédé selon la revendication 1, dans lequel le produit est un composé de formule (I), dans laquelle Y est un alcanoylimino en $C_{1-5}$.

**20.** Procédé selon la revendication 1, dans lequel le produit est un composé de formule (I), dans laquelle Y est l'acétylimino.

**21.** Procédé selon la revendication 1, dans lequel le produit est un composé de formule (I), dans laquelle

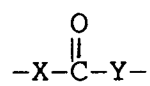

est un groupe de formule:

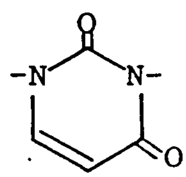

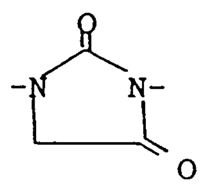

**22.** Procédé selon la revendication 1, dans lequel le produit est un composé de formule (I), dans laquelle Z-$R^4$ est un groupe azétidinyle, pyrrolidinyle, pipéridinyle, perhydroazépinyle, pyrrolinyle, pyrazolinyle, pyrrolyle, pyridyle, oxazolyle, thiazolyle, pyridazinyle, pyrimidyle, pyrazinyle, imidazolyle, morpholinyle, thiomorpholinyle, pipérazinyle, pyrazolidinyle, indolyle, isoindolyle, 1H-indazolyle, purinyle, quinoléinyle, isoquinoléinyle, perhydroindolyle ou perhydroisoquinoléinyle, éventuellement substitué par un groupe alcoyle en $C_{1-6}$, alcoxy en $C_{1-4}$-carbonyle-alcoyle en $C_{1-6}$, carboxylate-alcoyle en $C_{1-6}$ ou phénylalcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**23.** Procédé selon la revendication 1, dans lequel le produit est un composé de formule (I), dans laquelle Z-$R^4$ est un groupe morpholinyle, pyrrolidinyle, pipéridinyle, imidazolyle, pyridyle ou thiazolyle éventuellement substitué par un groupe alcoyle en $C_{1-6}$, alcoxy en $C_{1-4}$-carbonyl-alcoyle en $C_{1-6}$, carboxylatealcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**24.** Procédé selon la revendication 1, dans lequel le produit est un composé de formule (I), dans laquelle Z-$R^4$ est un groupe pyridyle ou thiazolyle éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**25.** Procédé selon la revendication 1, dans lequel le produit est un composé de formule (I), dans laquelle Z-$R^4$ est un groupe pyridyle ou thiazolyle.

**26.** Procédé selon la revendication 1, dans lequel le produit est un composé de formule (I), dans laquelle Z-$R^4$ est un groupe 2-pyridyle, thiazol-2-yle ou thiazol-4-yle.

**27.** Procédé selon la revendication 1, dans lequel le produit est un composé de formule (I), dans laquelle Z-$R^4$ est un groupe imidazolio, pyridinio, oxazolio, thiazolio, pyridazinio, pyrimidinio, pyrazinio, quinoléinio, isoquinoléinio, morpholinio, pipéridinio, pipérazinio ou pyrrolidinio éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$) amino, carbamoyle, uréido, carboxylique, carboxylate ou alcoxy en $C_{1-4}$-carbonyle.

**28.** Procédé selon la revendication 1, dans lequel le produit est un composé de formule (I), dans laquelle Z-$R^4$ est un groupe morpholinio, pyrrolidinio, pipéridinio, imidazolio, pyridinio ou thiazolio éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**29.** Procédé selon la revendication 1, dans lequel le produit est un composé de formule (I), dans laquelle Z-$R^4$ est un groupe pyridinio ou thiazolio éventuellement substitué par un groupe alcoyle en $C_{1-6}$, alcoxy en $C_{1-4}$-carbonyl-alcoyle en $C_{1-6}$, carboxylate-alcoyle en $C_{1-6}$ ou phényl-alcoyle en $C_{1-6}$, ledit groupe étant non substitué ou substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.

**30.** Procédé selon la revendication 1, dans lequel le produit est un composé de formule (I), dans laquelle Z-$R^4$ est le groupe 1-(alcoyle en $C_{1-6}$)pyridinio-2-yle.

**31.** Procédé selon la revendication 1, dans lequel le produit est un composé de formule (I), dans laquelle Z-$R^4$ est le groupe 1-éthylpyridinio-2-yle.

**32.** Procédé selon la revendication 1, dans lequel le produit est un composé de formule (I), dans laquelle Z-$R^4$ est un groupe 3-(alcoyle en $C_{1-6}$)thiazolio-2-yle.

**33.** Procédé selon la revendication 1, dans lequel le produit est un composé de formule (I), dans laquelle Z-$R^4$ est le groupe 3-éthylthiazolio-2-yle.

**34.** Procédé selon la revendication 1, dans lequel le produit est un composé de formule (I), dans laquelle Z-$R^4$ est un groupe 3-(alcoyle en $C_{1-6}$)thiazolio-4-yle.

**35.** Procédé selon la revendication 1, dans lequel le produit est un composé de formule (I), dans laquelle Z-$R^4$ est le groupe 3-éthylthiazolio-4-yle.

**36.** Procédé selon la revendication 1, dans lequel le produit est un chlorure de 3-O-[N-acétyl-N-(N-méthylpyridinio-2-yl)mé-thyl]carbamoyl-2-O-méthyl-1-octadécylcarbamoylglycérol.

**37.** Procédé selon la revendication 1, dans lequel le produit est un halogénure de 1-éthyl-2-[N-acétyl-N-(3-octadécylcarba-moyloxy-2-méthoxypropyloxy)carbonylamino]méthylpyridinium.

**38.** Procédé selon la revendication 1, dans lequel le produit est un halogénure de 2-[N-acétyl-N-(3-octadécylcarbamoyloxy-2-méthoxypropoxycarbonyl)amino]méthyl-3-méthylthiazolium.

**39.** Procédé selon la revendication 1, dans lequel le produit est un halogénure de 2-[N-acétyl-N-(3-octadécylcarbamoyloxy-2-méthoxypropoxycarbonyl)amino]méthyl-3-éthylthiazolium.

**40.** Procédé selon la revendication 1, dans lequel le produit est un halogénure de 2-[N-(3-octadécylcarbamoyloxy-2-éthoxypro-pyloxycarbonyl)amino]méthyl-N-éthylpyridinium.

**41.** Procédé selon la revendication 1, dans lequel le produit est un halogénure de 4-[N-acétyl-N-(3-octadécylcarbamoyloxy-2-méthoxypropoxycarbonyl)amino]méthyl-3-éthylthiazolium.

**42.** Procédé selon la revendication 37, dans lequel l'halogénure est le chlorure.

**43.** Procédé selon la revendication 38, dans lequel l'halogénure est l'iodure.

**44.** Procédé selon la revendication 39, dans lequel l'halogénure est l'iodure.

**45.** Procédé selon la revendication 40, dans lequel l'halogénure est l'iodure.

**46.** Procédé selon la revendication 41, dans lequel l'halogénure est l'iodure.

**47.** Procédé selon la revendication 1, dans lequel $R^3$ est un alcoylène en $C_{1-8}$ et Z est un noyau hétérocyclique contenant de l'azote qui peut être substitué par un groupe alcoyle en $C_{1-4}$, éventuellement substitué par un hydroxy ou un amino, ou par un groupe hydroxy, amino, imino, mono- ou di-(alcoyle en $C_{1-4}$)amino, carbamoyle, uréido, carboxylique, carboxylate ou alcoxy en $C_{1-4}$-carbonyle.